# EUROPEAN PATENT APPLICATION

(11) **EP 0 943 606 A1**
(43) Date of publication of application: **22.09.1999**
(21) Application number: 97912435.1
(22) Date of filing: 12.11.1997
(51) Int. Cl.: C07C 311/19, C07C 311/29, C07D 333/24, A61K 31/195, A61K 31/215, A61K 31/38

(54) **BENZENESULFONAMIDE DERIVATIVES AND DRUGS CONTAINING THE SAME**

(30) Priority: 13.11.1996 JP 31710996; 16.09.1997 JP 26923497
(71) Applicant: HOKURIKU SEIYAKU CO., LTD., Katsuyama-shi, Fukui 911 (JP)
(72) Inventor: YASUDA, Shingo, Katsuyama-shi,Fukui 911 (JP); OGAWA, Nobuo, Katsuyama-shi,Fukui 911 (JP); SAKURAI, Shunichiro, Katsuyama-shi,Fukui 911 (JP)
(74) Representative: Godemeyer, Thomas, Dr.
(86) International application number: JP9704125
(87) International publication number: WO9821177

(57) **Abstract**

Novel benzenesulfonamide derivatives represented by the following general formula (I) are provided: 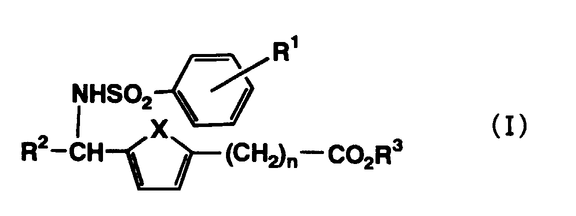 wherein R¹ represents hydrogen atom, a halogen atom, a lower alkyl group, a lower alkoxyl group, or nitro group; R² represents a C₄-C₈ alkyl group which is substituted with one or more fluorine atoms; R³ represents hydrogen atom or a lower alkyl group; X represents sulfur atom or a group represented by CH=CH; and n represents an integer of from 2 to 4. Medicaments comprising said compound as an active ingredient can be used for preventive and/or therapeutic treatment of thrombosis, asthma, or allergic diseases.

## Description

### Technical Field

The present invention relates to novel benzenesulfonamide derivatives which have both of thromboxane A₂ and leukotriene D₄ antagonistic activities, and are useful as active ingredients of medicaments such as platelet aggregation inhibitors, antithrombotic agents, antiasthmatic agents, and antiallergic agents.

### Background Art

Thromboxane A₂ and leukotrienes are known as chemical mediators which cause ischemic heart diseases, cerebrovascular disorders, thrombosis, asthma, allergic inflammations and the like. As antagonists against these chemical mediators, numbers of thromboxane A₂ antagonists and leukotriene antagonists have been found so far and investigated in their efficacy on these diseases. However, thromboxane A₂ antagonists or leukotriene antagonists alone fail to achieve sufficient efficacy.

### Disclosure of the Invention

The inventors of the present invention conducted various studies based on a hypothesis that a medicament having both of thromboxane A₂ and leukotriene D₄ antagonistic activities may achieve a more excellent therapeutic effect than those having one of the antagonism. As a result, they found benzenesulfonamide derivatives having both of thromboxane A₂ and leukotriene D₄ antagonistic activities (Japanese Patent Unexamined Publication (KOKAI) No. (Hei) 7-53505/1995). However, it was revealed that these compounds were rather insufficient in anti-leukotriene D₄ activity when administered orally and metabolically unstable. The inventors conducted further intensive studies and they found novel benzenesulfondamide derivatives according to the present invention which can solve the foregoing problems. The present invention was achieved on the basis of these findings.

The present invention thus provide novel benzenesulfonamide derivatives represented by the following general formula (I) or salts thereof: 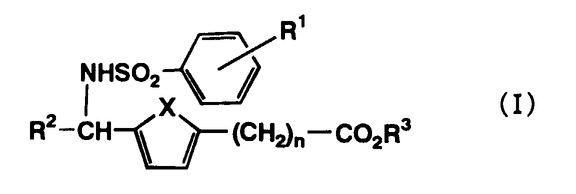 wherein R¹ represents hydrogen atom, a halogen atom, a lower alkyl group, a lower alkoxyl group, or nitro group; R² represents a C₄-C₈ alkyl group which is substituted with one or more fluorine atoms; R³ represents hydrogen atom or a lower alkyl group; X represents sulfur atom or a group represented by CH=CH; and n represents an integer of from 2 to 4.

According to preferred embodiments of the present invention, there are provided the compounds wherein X is sulfur atom in the aforementioned general formula (I); and the compounds wherein X is a group represented by CH=CH in the aforementioned general formula (I). According to further preferred embodiments, there are provided the compounds wherein R¹ is a halogen atom which substitutes in the para-position, R² is a C₅-C₇ straight or branched chain alkyl group which is substituted with three or more fluorine atoms, and n is 3 or 4 in the aforementioned general formula (I).

According to another aspect of the present invention, there is provided medicaments which comprises as an active ingredient a substance selected from the group consisting of the compound represented by the aforementioned general formula (I) and the physiologically acceptable salt thereof, and a hydrate thereof and a solvate thereof. The medicaments can be used for preventive and/or therapeutic treatment of thrombosis, asthma, or allergic diseases. As preferred embodiments of the invention, there are provided the medicaments in the form of pharmaceutical compositions such as, for example, platelet aggregation inhibitors, antithrombotic agents, antiasthmatic agents, or antiallergic agents.

According to further aspects of the present invention there are provided a use of the substance selected from the group consisting of the compound represented by the aforementioned general formula (I) and the physiologically acceptable salt thereof, and the hydrate thereof and the solvate thereof for a manufacture of the aforementioned medicament; and a method for the therapeutic and/or preventive treatment of thrombosis, asthma, or allergic diseases which comprises the step of administering an effective amount of the substance selected from the group consisting of the compound represented by the aforementioned general formula (I) and the physiologically acceptable salt thereof, and the hydrate thereof and the solvate thereof to a mammal including a human.

### Best Mode for Carrying Out the Invention

In the aforementioned general formula (I) of the present invention, as the lower alkyl group represented by R¹ and R³, for example, a straight or branched chain alkyl group having 1 to 4 carbon atoms can be used. More specifically, for example, methyl group, ethyl group, n-propyl group, isopropyl group, n-butyl group, isobutyl group, sec-butyl group, tert-butyl group can be used. As the lower alkoxyl group represented by R¹, for example, a straight or branched chain alkoxyl group having 1 to 4 carbon atoms can be used. More specifically, for example, methoxy group, ethoxy group, n-propoxy group, isopropoxy group, n-butoxy group, isobutoxy group, sec-butoxy group, tert-butoxy group and other can be used. The halogen atom may be any one of fluorine atom, chlorine atom, bromine atom, and iodine atom.

As the C₄-C₈ alkyl group substituted with one or more fluorine atoms which is represented by R², a straight or branched chain alkyl group can be used. The examples include 4-fluorobutyl group, 5-fluoropentyl group, 6-fluorohexyl group, 4,4-difluorobutyl group, 5,5-difluoropentyl group, 6,6-difluorohexyl group, 4,4,4-trifluorobutyl group, 5,5,5-trifluoropentyl group, 6,6,6-trifluorohexyl group, 7,7,7-trifluoroheptyl group, 4,4,4-trifluoro-3-methylbutyl group, 5,5,5-trifluoro-4-methylpentyl group, 6,6,6-trifluoro-5-methylhexyl group, 3,3,4,4,4-pentafluorobutyl group, 4,4,5,5,5-pentafluoropentyl group, 5,5,6,6,6-pentafluorohexyl group, 6,6,7,7,7-pentafluoroheptyl group, 3,3,4,4,5,5,5-heptafluoropentyl group, 4,4,5,5,6,6,6-heptafluorohexyl group, 5,5,6,6,7,7,7-heptafluoroheptyl group, 3,3,4,4,5,5,6,6,6-nonafluorohexyl group, 4,4,5,5,6,6,7,7,7-nonafluoroheptyl group, 3,3,4,4,5,5,6,6,7,7,7-undecafluoroheptyl group, 3,3,4,4,5,5,6,6,7,7,8,8,8-tridecafluorooctyl group, 4,4,4-trifluoro-3-trifluoromethylbutyl group, 5,5,5-trifluoro-4-trifluoromethylpentyl group, 6,6,6-trifluoro-5-trifluoromethylhexyl group, 3,4,4,4-tetrafluoro-3-trifluoromethylbutyl group, 4,5,5,5-tetrafluoro-4-trifluoromethylpentyl group, 5,6,6,6-tetrafluoro-5-trifluoromethylhexyl group, 3,3,4,5,5,5-hexafluoro-4-trifluoromethylpentyl group, 4,4,5,6,6,6-hexafluoro-5-trifluoromethylhexyl group, 3,4,4,5,5,5-hexafluoro-3-trifluoromethylpentyl group, 3,3,4,4,5,6,6,6-octafluoro-5-trifluoromethylhexyl group, 5,5,5-trifluoro-4,4-bis(trifluoromethyl)pentyl group, 6,6,6-trifluoro-5,5-bis(trifluoromethyl)hexyl group, and 4,4,5,5,6,6,6-heptafluoro-3,3-bis(trifluoromethyl)hexyl group.

The compounds of the present invention represented by the aforementioned general formula (I) have one or more asymmetric carbon atoms, and their optical isomers and diastereoisomers may exist. Any one of pure isomers, racemates, and any mixture of the isomers and the like fall within the scope of the present invention.

Among the compounds of the present invention represented by the aforementioned general formula (I), those wherein R³ is hydrogen atom may sometimes exist as salts. The aforementioned compounds in the free form can be converted into the salts, preferably physiologically acceptable salts, if required. In addition, the resulting salts can be further converted into free acids.

The physiologically acceptable salts of the compounds of the present invention include, for example, inorganic alkali salts such as sodium, potassium, calcium, and ammonium salts, and salts with organic bases such as trimethylamine, triethylamine, triethanolamine, pyrrolidine, piperidine, piperazine, N-methylmorpholine, (S)-(-)-1-phenethylamine, (R)-(+)-1-phenethylamine, (R)-(-)-1-cyclohexylethylamine, (S)-(+)-1-cyclohexylethylamine, (R)-(-)-2-amino-1-butanol, (S)-(+)-2-amino-1-butanol, L(+)-lysine, and L-(+)-arginine.

The compounds of the present invention represented by the aforementioned general formula (I) and physiologically acceptable salts thereof may exist in any crystal forms depending on manufacturing conditions, or may exist as any hydrate. Moreover, they may sometimes exist as solvates which contain one or more organic solvents such as acetone, ethanol, and tetrahydrofuran. It should be understood that substances in any one of these forms fall within the scope of the present invention.

Examples of the preferred compounds of the present invention include the following compounds. However, the present invention is not limited to these examples.
(1) methyl 4-[5-[1-(4-chlorophenylsulfonylamino)-6-fluorohexyl]-2-thienyl]butyrate
(2) 4-[5-[1-(4-chlorophenylsulfonylamino)-6-fluorohexyl]-2-thienyl]butyric acid
(3) methyl 4-[5-[6-fluoro-1-(4-fluorophenylsulfonylamino)hexyl]-2-thienyl]butyrate
(4) 4-[5-[6-fluoro-1-(4-fluorophenylsulfonylamino)hexyl]-2-thienyl]butyric acid
(5) methyl 4-[5-[6-fluoro-1-(4-nitrophenylsulfonylamino)hexyl]-2-thienyl]butyrate
(6) 4-[5-[6-fluoro-1-(4-nitrophenylsulfonylamino)hexyl]-2-thienyl]butyric acid
(7) methyl 4-[5-[1-(4-chlorophenylsulfonylamino)-5,5,5-trifluoropentyl]-2-thienyl]butyrate
(8) 4-[5-[1-(4-chlorophenylsulfonylamino)-5,5,5-trifluoropentyl]-2-thienyl]butyric acid
(9) methyl 4-[5-[1-(4-chlorophenylsulfonylamino)-6,6,6-trifluorohexyl]-2-thienyl]butyrate
(10) 4-[5-[1-(4-chlorophenylsulfonylamino)-6,6,6-trifluorohexyl]-2-thienyl]butyric acid
(11) methyl 4-[5-[6,6,6-trifluoro-1-(4-fluorophenylsulfonylamino)hexyl]-2-thienyl]butyrate
(12) 4-[5-[6,6,6-trifluoro-1-(4-fluorophenylsulfonylamino)hexyl]-2-thienyl]butyric acid
(13) methyl 4-[5-[6,6,6-trifluoro-1-(4-nitrophenylsulfonylamino)hexyl]-2-thienyl]butyrate
(14) 4-[5-[6,6,6-trifluoro-1-(4-nitrophenylsulfonylamino)hexyl]-2-thienyl]butyric acid
(15) methyl 4-[5-[1-(4-chlorophenylsulfonylamino)-7,7,7-trifluoroheptyl]-2-thienyl]butyrate
(16) 4-[5-[1-(4-chlorophenylsulfonylamino)-7,7,7-trifluoroheptyl]-2-thienyl]butyric acid
(17) methyl 4-[5-[7,7,7-trifluoro-1-(4-fluorophenylsulfonylamino)heptyl]-2-thienyl]butyrate
(18) 4-[5-[7,7,7-trifluoro-1-(4-fluorophenylsulfonylamino)heptyl]-2-thienyl]butyric acid
(19) methyl 4-[5-[1-(4-chlorophenylsulfonylamino)-6,6,6-trifluoro-5-methylhexyl]-2-thienyl]butyrate
(20) 4-[5-[1-(4-chlorophenylsulfonylamino)-6,6,6-trifluoro-5-methylhexyl]-2-thienyl]butyric acid
(21) methyl 4-[5-[6,6,6-trifluoro-1-(4-fluorophenylsulfonylamino)-5-methylhexyl]-2-thienyl]butyrate
(22) 4-[5-[6,6,6-trifluoro-1-(4-fluorophenylsulfonylamino)-5-methylhexyl]-2-thienyl]butyric acid
(23) methyl 4-[5-[1-(4-chlorophenylsulfonylamino)-5,5,6,6,6-pentafluorohexyl]-2-thienyl]butyrate
(24) 4-[5-[1-(4-chlorophenylsulfonylamino)-5,5,6,6,6-pentafluorohexyl]-2-thienyl]butyric acid
(25) methyl 4-[5-[5,5,6,6,6-pentafluoro-1-(4-fluorophenylsulfonylamino)hexyl]-2-thienyl]butyrate
(26) 4-[5-[5,5,6,6,6-pentafluoro-1-(4-fluorophenylsulfonylamino)hexyl]-2-thienyl]butyric acid
(27) (+)-4-[5-[5,5,6,6,6-pentafluoro-1-(4-fluorophenylsulfonylamino)hexyl]-2-thienyl]butyric acid
(28) (-)-4-[5-[5,5,6,6,6-pentafluoro-1-(4-fluorophenylsulfonylamino)hexyl]-2-thienyl]butyric acid
(29) methyl 4-[5-[5,5,6,6,6-pentafluoro-1-(4-nitrophenylsulfonylamino)hexyl]-2-thienyl]butyrate
(30) 4-[5-[5,5,6,6,6-pentafluoro-1-(4-nitrophenylsulfonylamino)hexyl]-2-thienyl]butyric acid
(31) methyl 5-[5-[1-(4-chlorophenylsulfonylamino)-5,5,6,6,6-pentafluorohexyl]-2-thienyl]valerate
(32) 5-[5-[1-(4-chlorophenylsulfonylamino)-5,5,6,6,6-pentafluorohexyl]-2-thienyl]valeric acid
(33) methyl 5-[5-[5,5,6,6,6-pentafluoro-1-(4-fluorophenylsulfonylamino)hexyl]-2-thienyl]valerate
(34) 5-[5-[5,5,6,6,6-pentafluoro-1-(4-fluorophenylsulfonylamino)hexyl]-2-thienyl]valeric acid
(35) methyl 4-[5-[1-(4-chlorophenylsulfonylamino)-6,6,7,7,7-pentafluoroheptyl]-2-thienyl]butyrate
(36) 4-[5-[1-(4-chlorophenylsulfonylamino)-6,6,7,7,7-pentafluoroheptyl]-2-thienyl]butyric acid
(37) methyl 4-[5-[6,6,7,7,7-pentafluoro-1-(4-fluorophenylsulfonylamino)heptyl]-2-thienyl]butyrate
(38) 4-[5-[6,6,7,7,7-pentafluoro-1-(4-fluorophenylsulfonylamino)heptyl]-2-thienyl]butyric acid
(39) methyl 4-[5-[1-(4-chlorophenylsulfonylamino)-4,4,5,5,6,6,6-heptafluorohexyl]-2-thienyl]butyrate
(40) 4-[5-[1-(4-chlorophenylsulfonylamino)-4,4,5,5,6,6,6-heptafluorohexyl]-2-thienyl]butyric acid
(41) methyl 4-[5-[4,4,5,5,6,6,6-heptafluoro-1-(4-fluorophenylsulfonylamino)hexyl]-2-thienyl]butyrate
(42) 4-[5-[4,4,5,5,6,6,6-heptafluoro-1-(4-fluorophenylsulfonylamino)hexyl]-2-thienyl]butyric acid
(43) methyl 4-[5-[1-(4-chlorophenylsulfonylamino)-5,5,6,6,7,7,7-pentafluoroheptyl]-2-thienyl]butyrate
(44) 4-[5-[1-(4-chlorophenylsulfonylamino)-5,5,6,6,7,7,7-pentafluoroheptyl]-2-thienyl]butyric acid
(45) methyl 4-[5-[5,5,6,6,7,7,7-pentafluoro-1-(4-fluorophenylsulfonylamino)heptyl]-2-thienyl]butyrate
(46) 4-[5-[5,5,6,6,7,7,7-pentafluoro-1-(4-fluorophenylsulfonylamino)heptyl]-2-thienyl]butyric acid
(47) methyl 4-[5-[1-(4-chlorophenylsulfonylamino)-4,4,5,5,6,6,7,7,7-nonafluoroheptyl]-2-thienyl]butyrate
(48) 4-[5-[1-(4-chlorophenylsulfonylamino)-4,4,5,5,6,6,7,7,7-nonafluoroheptyl]-2-thienyl]butyric acid
(49) methyl 4-[5-[4,4,5,5,6,6,7,7,7-nonafluoro-1-(4-fluorophenylsulfonylamino)heptyl]-2-thienyl]butyrate
(50) 4-[5-[4,4,5,5,6,6,7,7,7-nonafluoro-1-(4-fluorophenylsulfonylamino)heptyl]-2-thienyl]butyric acid
(51) methyl 4-[5-[1-(4-chlorophenylsulfonylamino)-5,6,6,6-tetrafluoro-5-trifluoromethylhexyl]-2-thienyl]butyrate
(52) 4-[5-[1-(4-chlorophenylsulfonylamino)-5,6,6,6-tetrafluoro-5-trifluoromethylhexyl]-2-thienyl]butyric acid
(53) methyl 4-[5-[5,6,6,6-tetrafluoro- 1-(4-fluorophenylsulfonylamino)-5-trifluoromethylhexyl]-2-thienyl]butyrate
(54) 4-[5-[5,6,6,6-tetrafluoro-1-(4-fluorophenylsulfonylamino)-5-trifluoromethylhexyl]-2-thienyl]butyric acid
(55) methyl 4-[5-[1-(4-chlorophenylsulfonylamino)-4,4,5,5,6,7,7,7-octafluoro-6-trifluoromethylheptyl]-2-thienyl]butyrate
(56) 4-[5-[1-(4-chlorophenylsulfonylamino)-4,4,5,5,6,7,7,7-octafluoro-6-trifluoromethylheptyl]-2-thienyl]butyric acid
(57) methyl 4-[5-[4,4,5,5,6,7,7,7-octafluoro-1-(4-fluorophenylsulfonylamino)-6-trifluoromethylheptyl]-2-thienyl]butyrate
(58) 4-[5-[4,4,5,5,6,7,7,7-octafluoro-1-(4-fluorophenylsulfonylamino)-6-trifluoromethylheptyl]-2-thienyl]butyric acid
(59) methyl 4-[4-[1-(4-chlorophenylsulfonylamino)-6-fluorohexyl]phenyl]butyrate
(60) 4-[4-[1-(4-chlorophenylsulfonylamino)-6-fluorohexyl]phenyl]butyric acid
(61) methyl 4-[4-[6-fluoro-1-(4-fluorophenylsulfonylamino)hexyl]phenyl]butyrate
(62) 4-[4-[6-fluoro-1-(4-fluorophenylsulfonylamino)hexyl]phenyl]butyric acid
(63) methyl 4-[4-[6-fluoro-1-(4-nitrophenylsulfonylamino)hexyl]phenyl]butyrate
(64) 4-[4-[6-fluoro-1-(4-nitrophenylsulfonylamino)hexyl]phenyl]butyric acid
(65) methyl 4-[4-[1-(4-chlorophenylsulfonylamino)-6,6,6-trifluorohexyl]phenyl]butyrate
(66) 4-[4-[1-(4-chlorophenylsulfonylamino)-6,6,6-trifluorohexyl]phenyl]butyric acid
(67) methyl 4-[4-[6,6,6-trifluoro-1-(4-fluorophenylsulfonylamino)hexyl]phenyl]butyrate
(68) 4-[4-[6,6,6-trifluoro-1-(4-fluorophenylsulfonylamino)hexyl]phenyl]butyric acid
(69) methyl 4-[4-[6,6,6-trifluoro-1-(4-nitrophenylsulfonylamino)hexyl]phenyl]butyrate
(70) 4-[4-[6,6,6-trifluoro-1-(4-nitrophenylsulfonylamino)hexyl]phenyl]butyric acid
(71) methyl 4-[4-[1-(4-chlorophenylsulfonylamino)-7,7,7-trifluoroheptyl]phenyl]butyrate
(72) 4-[4-[1-(4-chlorophenylsulfonylamino)-7,7,7-trifluoroheptyl]phenyl]butyric acid
(73) methyl 4-[4-[7,7,7-trifluoro-1-(4-fluorophenylsulfonylamino)heptyl]phenyl]butyrate
(74) 4-[4-[7,7,7-trifluoro-1-(4-fluorophenylsulfonylamino)heptyl]phenyl]butyric acid
(75) methyl 4-[4-[1-(4-chlorophenylsulfonylamino)-6,6,6-trifluoro-5-methylhexyl]phenyl]butyrate
(76) 4-[4-[1-(4-chlorophenylsulfonylamino)-6,6,6-trifluoro-5-methylhexyl]phenyl]butyric acid
(77) methyl 4-[4-[6,6,6-trifluoro-1-(4-fluorophenylsulfonylamino)-5-methylhexyl]phenyl]butyrate
(78) 4-[4-[6,6,6-trifluoro-1-(4-fluorophenylsulfonylamino)-5-methylhexyl]phenyl]butyric acid
(79) methyl 4-[4-[1-(4-chlorophenylsulfonylamino)-5,5,6,6,6-pentafluorohexyl]phenyl]butyrate
(80) 4-[4-[1-(4-chlorophenylsulfonylamino)-5,5,6,6,6-pentafluorohexyl]phenyl]butyric acid
(81) methyl 4-[4-[5,5,6,6,6-pentafluoro-1-(4-fluorophenylsulfonylamino)hexyl]phenyl]butyrate
(82) ethyl 4-[4-[5,5,6,6,6-pentafluoro-1-(4-fluorophenylsulfonylamino)hexyl]phenyl]butyrate
(83) 4-[4-[5,5,6,6,6-pentafluoro-1-(4-fluorophenylsulfonylamino)hexyl]phenyl]butyric acid
(84) (+)-4-[4-[5,5,6,6,6-pentafluoro-1-(4-fluorophenylsulfonylamino)hexyl]phenyl]butyric acid
(85) (-)-4-[4-[5,5,6,6,6-pentafluoro-1-(4-fluorophenylsulfonylamino)hexyl]phenyl]butyric acid
(86) methyl 4-[4-[5,5,6,6,6-pentafluoro-1-(4-methoxyphenylsulfonylamino)hexyl]phenyl]butyrate
(87) 4-[4-[5,5,6,6,6-pentafluoro-1-(4-methoxyphenylsulfonylamino)hexyl]phenyl]butyric acid
(88) methyl 4-[4-[5,5,6,6,6-pentafluoro-1-(4-nitrophenylsulfonylamino)hexyl]phenyl]butyrate
(89) 4-[4-[5,5,6,6,6-pentafluoro-1-(4-nitrophenylsulfonylamino)hexyl]phenyl]butyric acid
(90) methyl 5-[4-[5,5,6,6,6-pentafluoro-1-(4-fluorophenylsulfonylamino)hexyl]phenyl]valerate
(91) 5-[4-[5,5,6,6,6-pentafluoro-1-(4-fluorophenylsulfonylamino)hexyl]phenyl]valeric acid
(92) methyl 4-[4-[1-(4-chlorophenylsulfonylamino)-6,6,7,7,7-pentafluoroheptyl]phenyl]butyrate
(93) 4-[4-[1-(4-chlorophenylsulfonylamino)-6,6,7,7,7-pentafluoroheptyl]phenyl]butyric acid
(94) methyl 4-[4-[6,6,7,7,7-pentafluoro-1-(4-fluorophenylsulfonylamino)heptyl]phenyl]butyrate
(95) 4-[4-[6,6,7,7,7-pentafluoro-1-(4-fluorophenylsulfonylamino)heptyl]phenyl]butyric acid
(96) methyl 4-[4-[1-(4-chlorophenylsulfonylamino)-4,4,5,5,6,6,6-heptafluorohexyl]phenyl]butyrate
(97) 4-[4-[1-(4-chlorophenylsulfonylamino)-4,4,5,5,6,6,6-heptafluorohexyl]phenyl]butyric acid
(98) methyl 4-[4-[4,4,5,5,6,6.6-heptafluoro-1-(4-fluorophenylsulfonylamino)hexyl]phenyl]butyrate
(99) 4-[4-[4,4,5,5,6,6,6-heptafluoro-1-(4-fluorophenylsulfonylamino)hexyl]phenyl]butyric acid
(100) (+)-4-[4-[4,4,5,5,6,6,6-heptafluoro-1-(4-fluorophenylsulfonylamino)hexyl]phenyl]butyric acid
(101) (-)-4-[4-[4,4,5,5,6,6,6-heptafluoro-1-(4-fluorophenylsulfonylamino)hexyl]phenyl]butyric acid
(102) methyl 4-[4-[1-(4-chlorophenylsulfonylamino)-5,5,6,6,7,7,7-heptafluoroheptyl]phenyl]butyrate
(103) 4-[4-[1-(4-chlorophenylsulfonylamino)-5,5,6,6,7,7,7-heptafluoroheptyl]phenyl]butyric acid
(104) methyl 4-[4-[5,5,6,6,7,7,7-heptafluoro-1-(4-fluorophenylsulfonylamino)heptyl]phenyl]butyrate
(105) 4-[4-[5,5,6,6,7,7,7-heptafluoro-1-(4-fluorophenylsulfonylamino)heptyl]phenyl]butyric acid
(106) methyl 4-[4-[1-(4-chlorophenylsulfonylamino)-4,4,5,5,6,6,7,7,7-nonafluoroheptyl]phenyl]butyrate
(107) 4-[4-[1-(4-chlorophenylsulfonylamino)-4,4,5,5,6,6,7,7,7-nonafluoroheptyl]phenyl]butyric acid
(108) methyl 4-[4-[4,4,5,5,6,6,7,7,7-nonafluoro-1-(4-fluorophenylsulfonylamino)heptyl]phenyl]butyrate
(109) 4-[4-[4,4,5,5,6,6,7,7,7-nonafluoro-1-(4-fluorophenylsulfonylamino)heptyl]phenyl]butyric acid
(110) methyl 4-[4-[1-(4-chlorophenylsulfonylamino)-5,6,6,6-tetrafluoro-5-trifluoromethylhexyl]phenyl]butyrate
(111) 4-[4-[1-(4-chlorophenylsulfonylamino)-5,6,6,6-tetrafluoro-5-trifluoromethylhexyl]phenyl]butyric acid
(112) methyl 4-[4-[5,6,6,6-tetrafluoro-1-(4-fluorophenylsulfonylamino)-5-trifluoromethylhexyl]phenyl]butyrate
(113) 4-[4-[5,6,6,6-tetrafluoro-1-(4-fluorophenylsulfonylamino)-5-trifluoromethylhexyl]phenyl]butyric acid
(114) methyl 4-[4-[1-(4-chlorophenylsulfonylamino)-4,4,5,5,6,7,7,7-octafluoro-6-trifluoromethylheptyl]phenyl]butyrate
(115) 4-[4-[1-(4-chlorophenylsulfonylamino)-4,4,5,5,6,7,7,7-octafluoro-6-trifluoromethylheptyl]phenyl]butyric acid
(116) methyl 4-[4-[4,4,5,5,6,7,7,7-octafluoro-1-(4-fluorophenylsulfonylamino)-6-trifluoromethylheptyl]phenyl]butyrate
(117) 4-[4-[4,4,5,5,6,7,7,7-octafluoro-1-(4-fluorophenylsulfonylamino)-6-trifluoromethylheptyl]phenyl]butyric acid

The compounds of the present invention represented by the aforementioned general formula (I) can be prepared by methods described below. Specified and detailed explanations of manufacturing processes of the aforementioned preferred compounds will also be given in Examples of the specification. Accordingly, it can be understood by a person skilled in the art that any compounds falling within the scope of the aforementioned general formula (I) can be easily prepared by referring to the following general explanations and specified explanations in Examples, and if necessary, by appropriately modifying or altering starting materials, reagents, and reaction conditions and the like. However, the methods for preparing the compounds of the present invention are not limited to those disclosed in the specification.

The compounds of the present invention represented by the general formula (I) can be prepared by reacting an amine derivative represented by the following general formula (II) wherein R², R³, X, and n have the same meanings as those defined above: 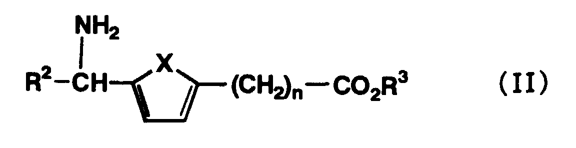 with a sulfonyl chloride derivative represented by the following general formula (III) wherein R¹ has the same meaning as that defined above: 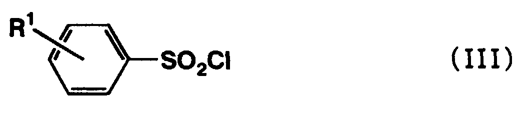 in a solvent and in the presence of a base, and then hydrolyzing the resulting ester, if necessary.

As the solvent used in the reaction of the compound of the general formula (II) with the compound of the general formula (III), any solvent may be used so far as it does not affect the reaction. For example, etheric solvents such as diethyl ether, tetrahydrofuran, and 1,4-dioxane; halogenated hydrocarbonic solvents such as chloroform, methylene chloride, and 1,2-dichloroethane; aromatic hydrocarbonic solvents such as benzene and toluene; and aprotic polar solvents such as acetonitrile, N,N-dimethylformamide, and dimethylsulfoxide may be used. The bases used in the reaction include, for example, organic bases such as triethylamine, diisopropylethylamine, 1,8-diazabicyclo[5.4.0]-7-undecene, pyridine, and 4-dimethylaminopyridine, and inorganic bases such as sodium carbonate, potassium carbonate, sodium hydrogen carbonate, anti potassium hydrogen carbonate. The reaction may be carried out at a temperature ranging from 0°C to the refluxing temperature of a solvent.

The hydrolysis of the ester can be carried out in water or a solvent containing water by using a base or an acid. The solvents containing water used in the hydrolysis include, for example, mixed solvents of water and organic solvents such as methanol, ethanol, n-propanol, isopropanol, n-butanol, acetone, tetrahydrofuran, and 1,4-dioxane. The bases used include, for example, sodium hydroxide, potassium hydroxide, lithium hydroxide, sodium carbonate, and potassium carbonate, and the acids include, for example, hydrochloric acid, hydrobromic acid, and sulfuric acid and the like. The hydrolysis is carried out at a temperature ranging from 0°C to the reflux temperature of a solvent.

Among the compounds of the present invention represented by the aforementioned general formula (I), those wherein R³ is hydrogen atom can be separated into (+)- or (-)-optical isomers by preparing a racemate, and subjecting salts formed with an optically active base to fractional recrystallization.

The optically active bases used in the above preparation include, for example, quinidine, hydroquinidine, quinine, brucine, cinchonidine, cinchonine, ephedrine, (S)-(-)-1-phenethylamine, (R)-(+)-1-phenethylamine, (R)-(-)-cyclohexylethylamine, (S)-(+)-1-cyclohexylethylamine, (R)-(-)-2-amino-1-butanol, (S)-(+)-2-amino-1-butanol, (1R,2S)-(-)-2-amino-1,2-diphenylethanol, (1S,2R)-(+)-2-amino-1,2-diphenylethanol, (-)-cis-2-benzylaminocyclohexanemethanol, (+)-cis-2-benzylaminocyclohexanemethanol, (R)-(+)-1-(p-tolyl)ethylamine, (S)-(-)-1-(p-tolyl)ethylamine, (S)-(+)-α-methyl-4-nitrobenzylamine, (R)-(-)-α-methyl-4-nitrobenzylamine, (S)-(-)-1-(1-naphthyl)ethylamine, (R)-(+)-1-(1-naphthyl)ethylamine, L-phenylalaninol, L-(+)-lysine, and L-(+)-arginine and the like.

The amine derivatives represented by the aforementioned general formula (II), used as the starting materials in the preparation, are also novel, and can be prepared, for example, according to the following method (in the formulas, R², R³, X and n have the same meanings as those described above). Specified anti detailed explanations of the preparations thereof are also given in Examples.

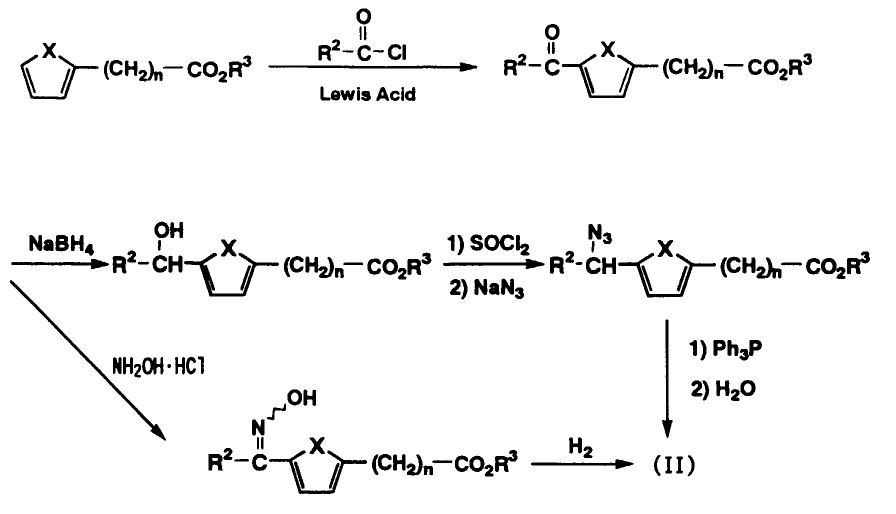

The novel benzenesulfonamide derivatives of the present invention represented by the aforementioned general formula (I) can be used for preventive and/or therapeutic treatment of thrombosis, asthma, or allergic diseases. As the medicament of the present invention, a substance selected from the group consisting of the compound in free form represented by the aforementioned general formula (I) and the physiologically acceptable salt thereof, and the hydrate thereof and the solvate thereof can be used. The substance, per se, may be administered as the medicament to mammals including humans. Generally, the medicament may preferably be administered as a pharmaceutical composition in the form of a platelet aggregation inhibitor, an antithrombotic agent, an antiasthmatic agent, or an antiallergic agent. The pharmaceutical composition may contain one or more of the aforementioned substances as the active ingredient.

The aforementioned pharmaceutical compositions may generally be administered as oral preparations in the forms of capsules, tablets, fine granules, granules, powders, syrups and the like, or as parenteral preparations in the forms of injections and the like. These preparations can be manufactured according to the conventional methods by using physiologically and pharmaceutically acceptable additives for pharmaceutical preparations together with the aforementioned substances as active ingredients.

For the manufacture of the oral preparations, pharmaceutical additives such as, for example, excipients such as lactose D-mannitol, corn starch, and crystalline cellulose, disintegrators such as carboxymethylcellulose and carboxymethylcellulose calcium; binders such as hydroxypropylcellulose, hydroxypropylmethylcellulose, and polyvinylpyrrolidone; lubricants such as magnesium stearate and talc; and coating agents such as hydroxypropylmehtylcellulose, sucrose, and titanium oxide may be used. For the manufacture of the injections, pharmaceutical additives such as, for example, solubilizers or solubilizing aids which may constitute aqueous injections or injections dissolved upon use such as distilled water for injection, physiological saline, and propylene glycol; pH modifier such as inorganic or organic acids or bases; isotonicities such as sodium chloride, glucose, and glycerin; stabilizer and the like may be used.

The forms of the medicaments of the present invention and manufacturing processes thereof, and pharmaceutical additives used for the manufacture of the medicament of the present invention are not particularly limited, and it should be understood that they can be appropriately chosen by a person skilled in the art.

A dose of the compounds of the present invention to a patient under therapeutic treatment can appropriately be chosen depending on the symptom, age, type of a disease and the like of the patient. For adults, the dose may generally be from 1 to 1,000 mg for oral administration and from 1 to 500 mg for parenteral administration. The aforementioned dose can be administered once a day or several times a day as divided portions.

### Examples

The present invention will be further explained with reference to examples. However, the present invention is not limited to these examples.

### Example 1: 5,6,6,6-Tetrafluoro-5-trifluoromethylhexanenitrile

To a solution of 10.0 g of 4,5,5,5-tetrafluoro-4-trifluoromethylpentanol and 6.72 ml of triethylamine in 30ml of dry diethyl ether, 3.73 ml of methanesulfonyl chloride was added dropwise under ice-cooling, and the mixture was stirred under ice-cooling for 1 hour. The precipitate was filtered off and the filtrate was concentrated under reduced pressure. To a solution of the resulting residue in 50 ml of dimethyl sulfoxide, 4.29g of sodium cyanide was added, and the mixture was stirred at 80°C for 3 hours. The reaction mixture was diluted with water and extracted with diethyl ether. The ether layer was washed with water, dried and then the solvent was carefully removed to obtain 8.29 g of brown oil.
IR spectrum ν (liq) cm⁻¹ : 2256
NMR spectrum δ (CDCl₃) ppm : 1.99(2H, qn, J=7.5Hz), 2.22-2.29(2H, m), 2.48(2H, t, J=7.5Hz)

The compounds of Example 2 and 3 were obtained in the same manner as described in Example 1.

### Example 2: 6,6,6-Trifluoro-5-methylhexanenitrile

Appearance pale red oil
IR spectrum ν (liq) cm⁻¹ : 2252
NMR spectrum δ (CDCl₃) ppm : 1.14(3H, d, J=6.5Hz), 1.46-1.54(1H, m), 1.67-1.85(3H, m), 2.12-2.27(1H, m), 2.33-2.43(2H, m)

### Example 3: 4,4,5,5,6,7,7,7-Octafluoro-6-trifluoromethylheptanenitrile

Appearance pale reddish brown oil
IR spectrum ν (liq) cm⁻¹ : 2260
NMR spectrum δ (CDCl₃) ppm : 2.48-2.58(2H, m), 2.69(2H, t, J=7.5Hz)

### Example 4: 5,6,6,6-Tetrafluoro-5-trifluoromethylhexanoic Acid

38 ml of 50%(v/v) sulfuric acid was added to 8.29 g of 5,6,6,6-tetrafluoro-5-trifluoromethylhexanenitrile, and the mixture was stirred at 120°C for 6 hours. The reaction mixture was diluted with water and extracted with diethyl ether. After the ether layer was back-extracted with dilute aqueous sodium hydroxide solution, the aqueous layer was acidified with dilute hydrochloric acid and extracted with diethyl ether. The ether layer was washed with water, dried, and then the solvent was removed. The resulting residue was distilled under reduced pressure to obtain 7.15 g of colorless oil, bp 101°C (21mmHg).
IR spectrum ν (liq) cm⁻¹ : 1722
NMR spectrum δ (CDCl₃) ppm : 1.94(2H, qn, J=7.5Hz), 2.13-2.20(2H, m), 2.47(2H, t, J=7.5Hz)

The compounds of Example 5 and 6 were obtained in the same manner as described in Example 4.

### Example 5: 6,6,6-Trifluoro-5-methylhexanoic Acid

Appearance pale red oil
IR spectrum ν (liq) cm⁻¹ : 1716
NMR spectrum δ (CDCl₃) ppm : 1.12(3H, d, J=7.5Hz), 1.34-1.41(1H, m), 1.61-1.82(3H, m), 2.08-2.22(1H, m), 2.34-2.44(2H, m)

### Example 6: 4,4,5,5,6,7,7,7-Octafluoro-6-trifluoromethylheptanoic Acid

Appearance colorless crystals
bp 109°C (21mmHg)
IR spectrum ν (liq) cm⁻¹ : 1726
NMR spectrum δ (CDCl₃) ppm : 2.44-2.55(2H, m), 2.70(2H, t, J=7.5Hz)

### Example 7: Methyl 4-[5-(5,5,6,6,6-Pentafluorohexanoyl)-2-thienyl]butyrate

To a solution of 7.15 g of methyl 4-(2-thienyl)butyrate in 20 ml of dry benzene, 20.2 g of anhydrous tin (IV) chloride was added dropwise under ice-cooling, and then a solution of 5,5,6,6,6-pentafluorohexanoyl chloride in 15 ml of dry benzene, which was prepared from 8.00 g of 5,5,6,6,6-pentafluorohexanoic acid and 3.40 ml of thionyl chloride in an usual manner, was added dropwise and then the mixture was stirred at room temperature for 30 minutes. The reaction mixture was poured into 200 ml of water and stirred with diethyl ether, and then the ether layer was separated. The ether layer was washed successively with water, aqueous potassium carbonate solution and water, dried, and then the solvent was removed. The resulting residue was purified by column chromatography on silica gel (methylene chloride) to obtain 9.98 g of pale yellowish brown oil.
IR spectrum ν (liq) cm⁻¹ : 1738, 1662
Mass spectrum m/z : 372 (M⁺)
NMR spectrum δ (CDCl₃) ppm : 2.00-2.08(4H, m), 2.10-2.20(2H, m), 2.38(2H, t, J=7.5Hz), 2.90(2H, t, J=7.5Hz), 2.97(2H, t, J=7Hz), 3.68(3H, s), 6.85(1H, d, J=4Hz), 7.55(1H, d, J=4Hz)

The compounds of Example 8 through 18 were obtained in the same manner as described in Example 7.

### Example 8: Methyl 5-[5-(5,5,6,6,6-Pentafluorohexanoyl)-2-thienyl]valerate

Appearance colorless needles (i-Pr₂O-n-Hexane)
mp 38.5∼39.5°C

| Analysis for C₁₆H₁₉F₅O₃S | | |
|---|---|---|
| Calculated % | C, 49.74; | H, 4.96 |
| Found % | C, 49.63; | H, 5.05 |

### Example 9: Methyl 4-[5-(5,5,5-Trifluorovaleryl)-2-thienyl]butyrate

Appearance colorless needles (i-Pr₂O-n-Hexane)
mp 52.5∼53°C

| Analysis for C₁₄H₁₇F₃O₃S | | |
|---|---|---|
| Calculated % | C, 52.17; | H, 5.32 |
| Found % | C, 52.04; | H, 5.31 |

### Example 10: Methyl 4-[5-(6,6,6-Trifluorohexanoyl)-2-thienyl]butyrate

Appearance pale yellow needles (i-Pr₂O)
mp 53.5∼54°C

| Analysis for C₁₅H₁₉F₃O₃S | | |
|---|---|---|
| Calculated % | C, 53.56; | H, 5.69 |
| Found % | C, 53.62; | H, 5.73 |

### Example 11: Methyl 4-[5-(7,7,7-Trifluoroheptanoyl)-2-thienyl]butyrate

Appearance pale yellowish brown oil
IR spectrum ν (liq) cm⁻¹ : 1740, 1658
Mass spectrum m/z : 350 (M⁺)
NMR spectrum δ (CDCl₃) ppm : 1.45(2H, qn, J=7.5Hz), 1.60(2H, qn, J=7.5Hz), 1.76(2H, qn, J=7.5Hz), 2.00-2.13(4H, m), 2.38(2H, t, J=7.5Hz), 2.86(2H, t, J=7.5Hz), 2.90(2H, t, J=7.5Hz), 3.68(3H, s), 6.84(1H, d, J=3.5Hz), 7.54(1H, d, J=3.5Hz)

### Example 12: Methyl 4-[5-(6,6,7,7,7-Pentafluoroheptanoyl)-2-thienyl]butyrate

Appearance colorless needles (i-Pr₂O)
mp 62∼62.5°C

| Analysis for C₁₆H₁₉F₅O₃S | | |
|---|---|---|
| Calculated % | C, 49.74; | H, 4.96 |
| Found % | C, 49.63; | H, 4.90 |

### Example 13: Methyl 4-[5-(4,4,5,5,6,6,6-Heptafluorohexanoyl)-2-thienyl]butyrate

Appearance colorless scales (i-Pr₂O)
mp 61∼61.5°C

| Analysis for C₁₅H₁₅F₇O₃S | | |
|---|---|---|
| Calculated % | C, 44.12; | H, 3.70 |
| Found % | C, 43.96; | H, 3.63 |

### Example 14: Methyl 4-[5-(5,5,6,6,7,7,7-Heptafluoroheptanoyl)-2-thienyl]butyrate

Appearance colorless scales (i-Pr₂O)
mp 45.5∼46°C

| Analysis for C₁₆H₁₇F₇O₃S | | |
|---|---|---|
| Calculated % | C, 45.50; | H, 4.06 |
| Found % | C, 45.53; | H, 4.18 |

### Example 15: Methyl 4-[5-(4,4,5,5,6,6,7,7,7-Nonafluoroheptanoyl)-2-thienyl]butyrate

Appearance colorless scales (i-Pr₂O)
mp 57∼57.5°C

| Analysis for C₁₆H₁₅F₉O₃S | | |
|---|---|---|
| Calculated % | C, 41.93: | H, 3.30 |
| Found % | C, 41.65; | H, 3.51 |

### Example 16: Methyl 4-[5-(6,6,6-Trifluoro-5-methylhexanoyl)-2-thienyl]butyrate

Appearance pale yellow oil
IR spectrum ν (liq) cm⁻¹ : 1740, 1662
Mass spectrum m/z : 350 (M⁺)
NMR spectrum δ (CDCl₃) ppm : 1.13(3H, d, J=7.5Hz), 1.35-1.43(1H, m), 1.68-1.77(2H, m), 1.85-1.93(1H, m), 2.03(2H, qn, J=7.5Hz), 2.13-2.22(1H, m), 2.38(2H, t, J=7.5Hz), 2.87(2H, t, J=7.5Hz), 2.90(2H, t, J=7.5Hz), 3.68(3H, s), 6.84(1H, d, J=4Hz), 7.54(1H, d, J=4Hz)

### Example 17: Methyl 4-[5-(5,6,6,6-Tetrafluoro-5-trifluoromethylhexanoyl)-2-thienyl]butyrate

Appearance colorless scales (i-Pr₂O)
mp 49∼49.5°C

| Analysis for C₁₆H₁₇F₇O₃S | | |
|---|---|---|
| Calculated % | C, 45.50; | H, 4.06 |
| Found % | C, 45.25; | H, 4.04 |

### Example 18: Methyl 4-[5-(4,4,5,5,6,7,7,7-Octafluoro-6-trifluoromethylheptanoyl)-2-thienyl]butyrate

Appearance pale pink feathers (i-Pr₂O-n-Hexane)
mp 67.5∼68.5°C

| Analysis for C₁₇H₁₅F₁₁O₃S | | |
|---|---|---|
| Calculated % | C, 40.17; | H, 2.97 |
| Found % | C, 40.06; | H, 2.77 |

### Example 19: 4-[5-(6-Fluorohexanoyl)-2-thienyl]butyric Acid

To a solution of 11.7 g of methyl 4-(2-thienyl)butyrate in 30 ml of benzene, a solution of 14.8 ml of anhydrous tin (IV) chloride in 20 ml of benzene was added under ice-cooling, and then a solution of 6-fluorohexanoyl chloride in 15 ml of benzene, which was prepared from 8.50g of 6-fluorohexanoic acid and 5.55 ml of thionyl chloride in an usual manner, was added dropwise. Then, the mixture was stirred at room temperature for 1.5 hours. The reaction mixture was poured into ice-water and extracted with diethyl ether. The ether layer was washed successively with water, aqueous potassium carbonate solution and water, dried, and then the solvent was removed. The resulting residue was purified by column chromatography on silica gel (ethyl acetate-n-hexane=1:4) to obtain a pale brown oil.

The resulting oil was added with 5.60 g of potassium fluoride and 30ml of ethylene glycol, and the mixture was stirred at 130°C for 31 hours. The reaction solution was diluted with water and extracted with diethyl ether. The ether layer was washed with water, dried, and then the solvent was removed.

The residue was added with 18.5 ml of 2N aqueous sodium hydroxide solution and 2 ml of tetrahydrofuran, and the mixture was stirred at room temperature for 2 hours. After the reaction mixture was washed with diethyl ether, the aqueous layer was acidified with dilute hydrochloric acid and extracted with diethyl ether. The ether layer was dried, and then the solvent was removed. The residue was purified by column chromatography on silica gel (methylene chloride-methanol=20:1) to obtain 6.50 g of pale yellow crystals. Recrystallization from diisopropyl ether gave colorless crystals, mp 49∼49.5°C.

| Analysis for C₁₄H₁₉FO₃S | | |
|---|---|---|
| Calculated % | C, 58.72; | H, 6.69 |
| Found % | C, 58.43; | H, 6.68 |

### Example 20: Methyl 4-[5-(6-Fluorohexanoyl)-2-thienyl]butyrate

To a solution of 6.54 g of 4-[5-(6-fluorohexanoyl)-2-thienyl]butyric acid in 50 ml of methanol, 0.13 ml of conc. sulfuric acid was added, and the mixture was refluxed for 2 hours. After the reaction solvent was removed under reduced pressure, water was added to the residue and the mixture was extracted with diethyl ether. The ether layer was washed successively with water, aqueous potassium carbonate solution and water, dried, and then the solvent was removed to obtain 6.55 g of pale yellow oil.
IR spectrum ν (liq) cm⁻¹ : 1738, 1660
Mass spectrum m/z : 300 (M⁺)
NMR spectrum δ (CDCl₃) ppm : 1.46-1.53(2H, m), 1.69-1.81(4H, m), 2.03(2H, qn, J=7.5Hz), 2.38(2H, t, J=7.5Hz), 2.86(2H, t, J=7.5Hz), 2.90(2H, t, J=7.5Hz), 3.68(3H, s), 4.45(2H, dt, J=47, 6Hz), 6.83(1H, d, J=3.5Hz), 7.54(1H, d, J=3.5Hz)

### Example 21: Methyl 4-[4-(5,5,6,6,6-Pentafluorohexanoyl)phenyl]butyrate

To a solution of 7.47 g of methyl 4-phenylbutyrate in 20 ml of carbon disulfide, 11.2 g of anhydrous aluminum chloride was added under ice-cooling, and then 5,5,6,6,6-pentafluorohexanoyl chloride, which was prepared from 8.64 g of 5,5,6,6,6-pentafluorohexanoic acid and 3.67 ml of thionyl chloride in an usual manner, was added dropwise, and stirring was continued at room temperature for 2.5 hours and then at 40°C for 1 hour. The reaction mixture was poured into ice-water and extracted with diethyl ether. The ether layer was washed successively with water, aqueous potassium carbonate solution and water, dried, and then the solvent was removed. The resulting residue was purified by column chromatography on silica gel (ethyl acetate-n-hexane=1:8) to obtain 8.78 g of colorless crystals. Recrystallization from diisopropyl ether gave colorless needles, mp 48∼48.5°C.

| Analysis for C₁₇H₁₉F₅O₃ | | |
|---|---|---|
| Calculated % | C, 55.74; | H, 5.23 |
| Found % | C, 55.65; | H, 5.17 |

The compounds of Example 22 through 31 were obtained in the same manner as described in Example 21.

### Example 22: Ethyl 4-[4-(5,5,6,6,6-Pentafluorohexanoyl)phenyl]butyrate

Appearance colorless crystals (i-Pr₂O-n-Hexane)
mp 34∼35°C

| Analysis for C₁₈H₂₁F₅O₃ | | |
|---|---|---|
| Calculated % | C, 56.84; | H, 5.57 |
| Found % | C, 56.76; | H, 5.52 |

### Example 23: Methyl 5-[4-(5,5,6,6,6-Pentafluorohexanoyl)phenyl]valerate

Appearance pale yellow oil
IR spectrum ν (liq) cm⁻¹ : 1738, 1688
Mass spectrum m/z : 380 (M⁺)
NMR spectrum δ (CDCl₃) ppm : 1.63-1.73(4H, m), 2.03-2.21(4H, m), 2.34(2H, t, J=7Hz), 2.69(2H, t, J=7Hz), 3.07(2H, t, J=6.5Hz), 3.67(3H, s), 7.27(2H, d, J=9Hz), 7.87(2H, d, J=9Hz)

### Example 24: Methyl 4-[4-(6,6,6-Trifluorohexanoyl)phenyl]butyrate

Appearance colorless crystals (i-Pr₂O)
mp 53.5∼54°C

| Analysis for C₁₇H₂₁F₃O₃ | | |
|---|---|---|
| Calculated % | C, 61.81; | H, 6.41 |
| Found % | C, 61.74; | H, 6.51 |

### Example 25: Methyl 4-[4-(6,6,7,7,7-Pentafluoroheptanoyl)phenyl]butyrate

Appearance colorless needles (i-Pr₂O)
mp 68∼69°C

| Analysis for C₁₈H₂₁F₅O₃ | | |
|---|---|---|
| Calculated % | C, 56.84; | H, 5.57 |
| Found % | C, 56.73; | H, 5.37 |

### Example 26: Methyl 4-[4-(4,4,5,5,6,6,6-Heptafluorohexanoyl)phenyl]butyrate

Appearance colorless needles (i-Pr₂O)
mp 67∼68°C

| Analysis for C₁₇H₁₇F₇O₃ | | |
|---|---|---|
| Calculated % | C, 50.75; | H, 4.26 |
| Found % | C, 50.85; | H, 4.38 |

### Example 27: Methyl 4-[4-(5,5,6,6,7,7,7-Heptafluoroheptanoyl)phenyl]butyrate

Appearance colorless plates (i-Pr₂O)
mp 44.5∼45.5°C

| Analysis for C₁₈H₁₉F₇O₃ | | |
|---|---|---|
| Calculated % | C, 51.93; | H, 4.60 |
| Found % | C, 51.86; | H, 4.65 |

### Example 28: Methyl 4-[4-(4,4,5,5,6,6,7,7,7-Nonafluoroheptanoyl)phenyl]butyrate

Appearance colorless needles (i-Pr₂O)
mp 67∼68.5°C

| Analysis for C₁₈H₁₇F₉O₃ | | |
|---|---|---|
| Calculated % | C, 47.80; | H, 3.79 |
| Found % | C, 47.64; | H, 3.83 |

### Example 29: Methyl 4-[4-(6,6,6-Trifluoro-5-methylhexanoyl)phenyl]butyrate

Appearance pale yellow oil
IR spectrum ν (liq) cm⁻¹ : 1740, 1686
Mass spectrum m/z : 344 (M⁺)
NMR spectrum δ (CDCl₃) ppm : 1.14(3H, d, J=7.5Hz), 1.36-1.47(1H, m), 1.69-1.78(2H, m), 1.86-1.94(1H, m), 1.98(2H, qn, J=7.5Hz), 2.13-2.24(1H, m), 2.34(2H, t, J=7.5Hz), 2.72(2H, t, J=7.5Hz), 2.97(2H, t, J=7Hz), 3.67(3H, s), 7.33(2H, d, J=8.5Hz), 7.88(2H, d, J=8.5Hz)

### Example 30: Methyl 4-[4-(5,6,6,6-Tetrafluoro-5-trifluoromethylhexanoyl)phenyl]butyrate

Appearance colorless oil
IR spectrum ν (liq) cm⁻¹ : 1738, 1688
Mass spectrum m/z : 416 (M⁺)
NMR spectrum δ (CDCl₃) ppm : 1.98(2H, qn, J=7.5Hz), 2.03-2.08(2H, m), 2.16-2.24(2H, m), 2.34(2H, t, J=7.5Hz), 2.72(2H, t, J=7.5Hz), 3.05(2H, t, J=6.5Hz), 3.67(3H, s), 7.28(2H, d, J=8Hz), 7.88(2H, d, J=8Hz)

### Example 31: Methyl 4-[4-(4,4,5,5,6,7,7,7-Octafluoro-6-trifluoromethylheptanoyl)phenyl]butyrate

Appearance colorless feathers (i-Pr₂O)
mp 63∼65°C

| Analysis for C₁₉H₁₇F₁₁O₃ | | |
|---|---|---|
| Calculated % | C, 45.43; | H, 3.41 |
| Found % | C, 45.30; | H, 3.39 |

### Example 32: Methyl 4-[4-(6-Bromohexanoyl)phenyl]butyrate

To a solution of 25.1 g of methyl 4-phenylbutyrate in 80 ml of carbon disulfide, 37.6 g of anhydrous aluminum chloride was added under ice-cooling, and then 6-bromohexanoyl chloride, which was prepared from 27.5 g of 6-bromohexanoic acid and 12.4 ml of thionyl chloride in an usual manner, was added dropwise and stirring was continued at 35°C for 3 hours. The reaction mixture was poured into ice-water and extracted with diethyl ether. The ether layer was washed successively with water, aqueous potassium carbonate solution and water, dried, and then the solvent was removed. The resulting residue was purified by column chromatography on silica gel (ethyl acetate-n-hexane=1:4) to obtain 26.2 g of colorless crystals. Recrystallization from diisopropyl ether gave colorless needles, mp 43.5∼44°C.

| Analysis for C₁₇H₂₃BrO₃ | | |
|---|---|---|
| Calculated % | C, 57.47; | H, 6.53 |
| Found % | C, 57.49; | H, 6.59 |

### Example 33: 4-[4-(6-Fluorohexanoyl)phenyl]butyric Acid

A mixture of 12.9 g of methyl 4-[4-(6-bromohexanoyl)phenyl]butyrate and 6.30 g of potassium fluoride in 35 ml of ethylene glycol was stirred at 130°C for 18 hours. After cooling, the reaction mixture was diluted with water and extracted with diethyl ether. The ether layer was washed with water, dried, and then the solvent was removed to obtain a pale yellow oil.

The resulting oil was added with 20 ml of 2N aqueous sodium hydroxide solution and 2 ml of tetrahydrofuran, and the mixture was stirred at room temperature for 2 hours. After the reaction mixture was washed with diethyl ether, the aqueous layer was acidified with dilute hydrochloric acid and extracted with diethyl ether. The ether layer was washed with water, dried, and then the solvent was removed. The residue was purified by column chromatography on silica gel (methylene chloridemethanol=10:1) to obtain 4.75 g of colorless crystals. Recrystallization from diisopropyl ether gave colorless needles, mp 47.5∼48°C.

| Analysis for C₁₆H₂₁FO₃ | | |
|---|---|---|
| Calculated % | C, 68.55; | H, 7.55 |
| Found % | C, 68.62; | H, 7.65 |

### Example 34: Methyl 4-[4-(6-Fluorohexanoyl)phenyl]butyrate

To a solution of 8.58 g of 4-[4-(6-fluorohexanoyl)phenyl]butyric acid in 25 ml of methanol, 0.17 ml of conc. sulfuric acid was added, and the mixture was refluxed for 2 hours. After the reaction solvent was removed under reduced pressure, water was added to the residue and the mixture was extracted with diethyl ether. The ether layer was washed successively with aqueous potassium carbonate solution and water, dried, and then the solvent was removed. The resulting residue was crystallized in cold diisopropyl ether and the crystals were collected by filtration to obtain 7.77 g of colorless crystals having low melting point.
IR spectrum ν (liq) cm⁻¹ : 1738, 1684
Mass spectrum m/z : 294 (M⁺)
NMR spectrum δ (CDCl₃) ppm : 1.47-1.54(2H, m), 1.70-1.82(4H, m), 1.98(2H, qn, J=7.5Hz), 2.34(2H, t, J=7.5Hz), 2.71(2H, t, J=7.5Hz), 2.97(2H, t, J=7.5Hz), 3.67(3H, s), 4.46(2H, dt, J=47.5, 6Hz), 7.27(2H, d, J=8Hz), 7.89(2H, d, J=8Hz)

### Example 35: Methyl 4-[4-(5,5,6,6,6-Pentafluoro-1-hydroxyiminohexyl)phenyl]butyrate

To a solution of 30.0 g of methyl 4-[4-(5,5,6,6,6-pentafluorohexanoyl)phenyl]butyrate in 90 ml of methanol, 6.50 g of hydroxylamine hydrochloride and 7.34 ml of pyridine were added, and the mixture was refluxed for 1 hour. After the reaction solvent was removed under reduced pressure, water was added to the resulting residue and the mixture was extracted with toluene. The toluene layer was washed successively with dilute hydrochloric acid, aqueous potassium carbonate solution and water, dried, and then the solvent was removed to obtain 29.9 g of pale yellowish brown crystals. Recrystallization from a mixture of diisopropyl ether and n-hexane gave colorless needles, mp 59∼60°C.

| Analysis for C₁₇H₂₀F₅NO₃ | | | |
|---|---|---|---|
| Calculated % | C, 53.54; | H, 5.29; | N, 3.67 |
| Found % | C, 53.38; | H, 5.20; | N, 3.64 |

### Example 36: Methyl 4-[5-(5,5,6,6,6-Pentafluoro-1-hydroxyhexyl)-2-thienyl]butyrate

To a solution of 9.97 g of methyl 4-[5-(5,5,6,6,6-pentafluorohexanoyl)-2-thienyl]butyrate in 100 ml of methanol, 1.01 g of sodium borohydride was added portionwise under ice-cooling, and stirring was continued at room temperature for 2 hours. After the reaction solvent was removed under reduced pressure, water was added to the residue and the mixture was extracted with diethyl ether. The ether layer was washed with water, dried, and then the solvent was removed to obtain 9.55 g of pale brown oil.
IR spectrum ν (liq) cm⁻¹ : 3456 (br) , 1738
Mass spectrum m/z : 374 (M⁺)
NMR spectrum δ (CDCl₃) ppm : 1.61-1.70(1H, m), 1.75-2.02(5H, m), 1.96(1H, d, J=4Hz), 1.99(2H, qn, J=7.5Hz), 2.37(2H, t, J=7.5Hz), 2.83(2H, t, J=7.5Hz), 3.67(3H, s), 4.84-4.87(1H, m), 6.64(1H, d, J=3.5Hz), 6.78(1H, d, J=3.5Hz)

The compounds of Example 37 through 59 were obtained in the same manner as described in Example 36.

### Example 37: Methyl 5-[5-(5,5,6,6,6-Pentafluoro-1-hydroxyhexyl)-2-thienyl]valerate

Appearance pale brown oil
IR spectrum ν (liq) cm⁻¹ : 3460 (br) , 1738
NMR spectrum δ (CDCl₃) ppm : 1.61-1.73(5H, m), 1.74-1.97(3H, m), 1.93(1H, d, J=3.5Hz), 2.01-2.13(2H, m), 2.34(2H, t, J=7Hz), 2.80(2H, t, J=6.5Hz), 3.67(3H, s), 4.84-4.87(1H, m), 6.63(1H, d, J=3.5Hz), 6.78(1H, d, J=3.5Hz)

### Example 38: Methyl 4-[5-(5,5,5-Trifluoro-1-hydroxypentyl)-2-thienyl]butyrate

Appearance pale yellow oil
IR spectrum ν (liq) cm⁻¹ : 3464 (br) , 1738
Mass spectrum m/z : 324 (M⁺)
NMR spectrum δ (CDCl₃) ppm : 1.59-1.66(1H, m), 1.71-1.80(1H, m), 1.81-1.93(2H, m), 1.93(1H, d, J=3.5Hz), 1.99(2H, qn, J=7.5Hz), 2.08-2.17(2H, m), 2.37(2H, t, J=7.5Hz), 2.83(2H, t, J=7.5Hz), 3.67(3H, s), 4.83-4.86(1H, m), 6.64(1H, d, J=3.5Hz), 6.78(1H, d, J=3.5Hz)

### Example 39: Methyl 4-[5-(6,6,6-Trifluoro-1-hydroxyhexyl)-2-thienyl]butyrate

Appearance pale yellow oil
IR spectrum ν (liq) cm⁻¹ : 3432 (br) , 1738
Mass spectrum m/z ; 338 (M⁺)
NMR spectrum δ (CDCl₃) ppm : 1.36-1.46(1H, m), 1.49-1.63(3H, m), 1.76-1.92(3H, m), 1.99(2H, qn, J=7.5Hz), 2.04-2.12(2H, m), 2.37(2H, t, J=7.5Hz), 2.83(2H, t, J=7.5Hz), 3.68(3H, s), 4.81-4.85(1H, m), 6.64(1H, d, J=3.5Hz), 6.77(1H, d, J=3.5Hz)

### Example 40: Methyl 4-[5-(7,7,7-Trifluoro-1-hydroxyheptyl)-2-thienyl]butyrate

Appearance pale yellow oil
IR spectrum ν (liq) cm⁻¹ : 3464 (br) , 1738
Mass spectrum m/z : 352 (M⁺)
NMR spectrum δ (CDCl₃) ppm : 1.32-1.51(4H, m), 1.53-1.59(2H, m), 1.75-1.89(2H, m), 1.89(1H, d, J=4Hz), 1.99(2H, qn, J=7.5Hz), 2.02-2.10(2H, m), 2.37(2H, t, J=7.5Hz), 2.83(2H, t, J=7.5Hz), 3.67(3H, s), 4.80-4.84(1H, m), 6.63(1H, d, J=3.5Hz), 6.77(1H, d, J=3.5Hz)

### Example 41: Methyl 4-[5-(6,6,7,7,7-Pentafluoro-1-hydroxyheptyl)-2-thienyl]butyrate

Appearance pale brown oil
IR spectrum ν (liq) cm⁻¹ : 3464 (br) , 1740
Mass spectrum m/z : 388 (M⁺)
NMR spectrum δ (CDCl₃) ppm : 1.37-1.46(1H, m), 1.50-1.66(3H, m), 1.77-1.92(2H, m), 1.93(1H, d, J=4.5Hz), 1.96-2.07(4H, m), 2.37(2H, t, J=7.5Hz), 2.83(2H, t, J=7.5Hz), 3.67(3H, s), 4.82-4.84(1H, m), 6.64(1H, d, J=3.5Hz), 6.77(1H, d, J=3.5Hz)

### Example 42: Methyl 4-[5-(4,4,5,5,6,6,6-Heptafluoro-1-hydroxyhexyl)-2-thienyl]butyrate

Appearance pale yellow oil
IR spectrum ν (liq) cm⁻¹ : 3464 (br) , 1740
Mass spectrum m/z : 410 (M⁺)
NMR spectrum δ (CDCl₃) ppm : 1.99(2H, qn, J=7.5Hz), 2.00(1H, d, J=4.5Hz), 2.06-2.23(3H, m), 2.24-2.40(1H, m), 2.37(2H, t, J=7.5Hz), 2.84(2H, t, J=7.5Hz), 3.68(3H, s), 4.90-4.93(1H, m), 6.60(1H, d, J=3.5Hz), 6.80(1H, d J=3.5Hz)

### Example 43: Methyl 4-[5-(5,5,6,6,7,7,7-Heptafluoro-1-hydroxyheptyl)-2-thienyl]butyrate

Appearance pale orange oil
IR spectrum ν (liq) cm⁻¹ : 3444 (br) , 1738
Mass spectrum m/z : 424 (M⁺)
NMR spectrum δ (CDCl₃) ppm : 1.62-1.70(1H, m), 1.76-2.02(6H, m), 2.04-2.16(2H, m), 2.37(2H, t, J=7.5Hz), 2.83(2H, t, J=7.5Hz), 3.67(3H, s), 4.84-4.88(1H, m), 6.64(1H, d, J=3.5Hz), 6.78(1H, d, J=3.5Hz)

### Example 44: Methyl 4-[5-(4,4,5,5,6,6,7,7,7-Nonafluoro-1-hydroxyheptyl)-2-thienyl]butyrate

Appearance pale yellow oil
IR spectrum ν (liq) cm⁻¹ : 3464 (br) , 1738
Mass spectrum m/z : 460 (M⁺)
NMR spectrum δ (CDCl₃) ppm : 1.99(2H, qn, J=7.5Hz), 2.00(1H, d, J=3Hz), 2.06-2.24(3H, m), 2.25-2.39(1H, m), 2.37(2H, t, J=7.5Hz), 2.84(2H, t, J=7.5Hz), 3.68(3H, s), 4.90-4.93(1H, m), 6.66(1H, d, J=3.5Hz), 6.81(1H, d, J=3.5Hz)

### Example 45: Methyl 4-[5-(6,6,6-Trifluoro-1-hydroxy-5-methylhexyl)-2-thienyl]butyrate

Appearance pale yellow oil
IR spectrum ν (liq) cm⁻¹ : 3456 (br) , 1740
Mass spectrum m/z : 352 (M⁺)
NMR spectrum δ (CDCl₃) ppm : 1.08(1.5H, d, J=6.5Hz), 1.09(1.5H, d, J=6.5Hz), 1.29-1.38(1.5H, m), 1.45-1.51(1H, m), 1.55-1.63(0.5H, m), 1.67-1.89(3H, m), 1.90(0.5H, d, J=3.5Hz), 1.91(0.5H, d, J=3.5Hz), 1.99(2H, qn, J=7.5Hz), 2.08-2.18(1H, m), 2.37(2H, t, J=7.5Hz), 2.83(2H, t, J=7.5Hz), 3.68(3H, s), 4.82-4.86(1H, m), 6.64(1H, d, J=3Hz), 6.77(1H, d, J=3Hz)

### Example 46: Methyl 4-[5-(5,6,6,6-Tetrafluoro-5-trifluoromethyl-1-hydroxyhexyl)-2-thienyl]butyrate

Appearance pale yellow oil
IR spectrum ν (liq) cm⁻¹ : 3432 (br) , 1738
Mass spectrum m/z : 424 (M⁺)
NMR spectrum δ (CDCl₃) ppm : 1.60-1.70(1H, m), 1.73-1.94(3H, m), 1.96(1H, d, J=3.5Hz), 1.99(2H, qn, J=7.5Hz), 2.06-2.17(2H, m), 2.37(2H, t, J=7.5Hz), 2.83(2H, t, J=7.5Hz), 3.67(3H, s), 4.84-4.88(1H, m), 6.64(1H, d, J=3Hz), 6.78(1H, d, J=3Hz)

### Example 47: Methyl 4-[5-(4,4,5,5,6,7,7,7-Octafluoro-6-trifluoromethyl-1-hydroxyheptyl)-2-thienyl]butyrate

Appearance pale yellow oil
IR spectrum ν (liq) cm⁻¹ : 3464 (br) , 1740
Mass spectrum m/z : 510 (M⁺)
NMR spectrum δ (CDCl₃) ppm : 1.96-2.02(3H, m), 2.05-2.25(3H, m), 2.26-2.39(1H, m), 2.37(2H, t, J=7.5Hz), 2.84(2H, t, J=7.5Hz), 3.68(3H, s), 4.90-4.93(1H, m), 6.66(1H, d, J=3.5Hz), 6.81(1H, d, J=3.5Hz)

### Example 48: Methyl 4-[5-(6-Fluoro-1-hydroxyhexyl)-2-thienyl]butyrate

Appearance pale yellow oil
IR spectrum ν (liq) cm⁻¹ : 3464 (br) , 1738
Mass spectrum m/z : 302 (M⁺)
NMR spectrum δ (CDCl₃) ppm : 1.36-1.55(4H, m), 1.65-1.88(4H, m), 1.90(1H, d, J=4Hz), 1.99(2H, qn, J=7.5Hz), 2.37(2H, t, J=7.5Hz), 2.83(2H, t, J=7.5Hz), 3.67(3H, s), 4.43(2H, dt, J=47.5, 6Hz), 4.81-4.85(1H, m), 6.63(1H, d, J=3.5Hz), 6.77(1H, d, J=3.5Hz)

### Example 49: Methyl 4-[4-(5,5,6,6,6-Pentafluoro-1-hydroxyhexyl)phenyl]butyrate

Appearance colorless oil
IR spectrum ν (liq) cm⁻¹ : 3448 (br) , 1738
Mass spectrum m/z : 368 (M⁺)
NMR spectrum δ (CDCl₃) ppm : 1.59-1.66(1H, m), 1.74-1.78(2H, m), 1.81(1H, d, J=3Hz), 1.83-1.89(1H, m), 1.95(2H, qn, J=7.5Hz), 2.01-2.10(2H, m), 2.33(2H, t, J=7.5Hz), 2.65(2H, t, J=7.5Hz), 3.67(3H, s), 4.66-4.69(1H, m), 7.17(2H, d, J=8Hz), 7.26(2H, d, J=8Hz)

### Example 50: Methyl 5-[4-(5,5,6,6,6-Pentafluoro-1-hydroxyhexyl)phenyl]valerate

Appearance colorless oil
IR spectrum ν (liq) cm⁻¹ : 3464 (br) , 1740
NMR spectrum δ (CDCl₃) ppm : 1.59-1.71(5H, m), 1.73-1.89(4H, m), 1.99-2.11(2H, m), 2.33(2H, t, J=7Hz), 2.63(2H, t, J=7Hz), 3.66(3H, s), 4.66-4.69(1H, m), 7.17(2H, d, J=8.5Hz), 7.25(2H, d, J=8.5Hz)

### Example 51: Methyl 4-[4-(6,6,6-Trifluoro-1-hydroxyhexyl)phenyl]butyrate

Appearance colorless oil
IR spectrum ν (liq) cm⁻¹ : 3448 (br) , 1740
Mass spectrum m/z : 332 (M⁺)
NMR spectrum δ (CDCl₃) ppm : 1.36-1.41(1H, m), 1.46-1.61(3H, m), 1.67-1.74(1H, m), 1.78(1H, d, J=3.5Hz), 1.79-1.85(1H, m), 1.95(2H, qn, J=7.5Hz), 2.00-2.10(2H, m), 2.33(2H, t, J=7.5Hz), 2.64(2H, t, J=7.5Hz), 3.67(3H, s), 4.63-4.66(1H, m), 7.16(2H, d, J=8Hz), 7.25(2H, d, J=8Hz)

### Example 52: Methyl 4-[4-(6,6,7,7,7-Pentafluoro-1-hydroxyheptyl)phenyl]butyrate

Appearance pale yellow oil
IR spectrum ν (liq) cm⁻¹ : 3456 (br) , 1740
Mass spectrum m/z : 382 (M⁺)
NMR spectrum δ (CDCl₃) ppm : 1.33-1.42(1H, m), 1.46-1.56(1H, m), 1.57-1.64(2H, m), 1.67-1.76(1H, m), 1.79-1.87(2H, m), 1.92-2.04(4H, m), 2.33(2H, t, J=7.5Hz), 2.64(2H, t, J=7.5Hz), 3.67(3H, s), 4.64-4.67(1H, m), 7.17(2H, d, J=7.5Hz), 7.25(2H, d, J=7.5Hz)

### Example 53: Methyl 4-[4-(4,4,5,5,6,6,6-Heptafluoro-1-hydroxyhexyl)phenyl]butyrate

Appearance pale yellow oil
IR spectrum ν (liq) cm⁻¹ : 3464 (br) , 1738
Mass spectrum m/z : 404 (M⁺)
NMR Spectrum δ (CDCl₃) ppm : 1.88(1H, d, J=3Hz), 1.92-2.20(6H, m), 2.22-2.37(1H, m), 2.33(2H, t, J=7.5Hz), 2.65(2H, t, J=7.5Hz), 3.67(3H, s), 4.71-4.75(1H, m), 7.19(2H, d, J=8Hz), 7.27(2H, d, J=8Hz)

### Example 54: Methyl 4-[4-(5,5,6,6,7,7,7-Heptafluoro-1-hydroxyheptyl)phenyl]butyrate

Appearance pale yellow oil
IR spectrum ν (liq) cm⁻¹ : 3440 (br) , 1738
NMR spectrum δ (CDCl₃) ppm : 1.60-1.67(1H, m), 1.73-1.89(3H,m), 1.92-1.98(3H, m), 2.01-2.14(2H, m), 2.33(2H, t, J=7.5Hz), 2.64(2H, t, J=7.5Hz), 3.66(3H, s), 4.66-4.68(1H, m), 7.17(2H, d, J=8Hz), 7.26(2H, d, J=8Hz)

### Example 55: Methyl 4-[4-(4,4,5,5,6,6,7,7,7-Nonafluoro-1-hydroxyheptyl)phenyl]butyrate

Appearance colorless oil
IR spectrum ν (liq) cm⁻¹ : 3464 (br) , 1740
Mass spectrum m/z : 454 (M⁺)
NMR spectrum δ (CDCl₃) ppm : 1.89(1H, d, J=3.5Hz), 1.92-2.22(5H, m), 2.24-2.38(1H, m), 2.33(2H, t, J=7.5Hz), 2.65(2H, t, J=7.5Hz), 3.67(3H, s), 4.72-4.75(1H, m), 7.19(2H, d, J=8Hz), 7.27(2H, d, J=8Hz)

### Example 56: Methyl 4-[4-(6,6,6-Trifluoro-1-hydroxy-5-methylhexyl)phenyl]butyrate

Appearance pale yellow oil
IR spectrum ν (liq) cm⁻¹ : 3448 (br) , 1738
Mass spectrum m/z : 346 (M⁺)
NMR spectrum δ (CDCl₃) ppm : 1.06(1.5H, d, J=6Hz), 1.07(1.5H, d, J=6Hz), 1.24-1.36(1.5H, m), 1.42-1.48(1H, m), 1.53-1.61(0.5H, m), 1.64-1.74(2H, m), 1.76-1.85(2H, m), 1.95(2H, qn, J=7.5Hz), 2.04-2.17(1H, m), 2.33(2H, t, J=7.5Hz), 2.65(2H, t, J=7.5Hz), 3.67(3H, s) 4.62-4.68(1H, m), 7.16(2H, d, J=8Hz), 7.25(2H, d, J=8Hz)

### Example 57: Methyl 4-[4-(5,6,6,6-Tetrafluoro-5-trifluoromethyl-1-hydroxyhexyl)phenyl]butyrate

Appearance colorless oil
IR spectrum ν (liq) cm⁻¹ : 3464 (br) , 1738
Mass spectrum m/z : 418 (M⁺)
NMR spectrum δ (CDCl₃) ppm : 1.58-1.67(1H, m), 1.71-1.86(3H, m), 1.80(1H, d, J=3Hz), 1.95(2H, qn, J=7.5Hz), 2.05-2.15(2H, m), 2.33(2H, t, J=7.5Hz), 2.65(2H, t, J=7.5Hz), 3.67(3H, s), 4.66-4.69(1H, m), 7.18(2H, d, J=7.5Hz), 7.25(2H, d, J=7.5Hz)

### Example 58: Methyl 4-[4-(4,4,5,5,6,7,7,7-Octafluoro-6-trifluoromethyl-1-hydroxyheptyl)phenyl]butyrate

Appearance colorless oil
IR spectrum ν (liq) cm⁻¹ : 3460 (br) , 1740
NMR spectrum δ (CDCl₃) ppm : 1.88(1H, d, J=3Hz), 1.92-2.23(5H, m), 2.24-2.34(1H, m), 2.33(2H, t, J=7.5Hz), 2.65(2H, t, J=7.5Hz), 3.67(3H, s), 4.71-4.75(1H, m), 7.19(2H, d, J=8Hz), 7.27(2H, d, J=8Hz)

### Example 59: Methyl 4-[4-(6-Fluoro-1-hydroxyhexyl)phenyl]butyrate

Appearance colorless oil
IR spectrum ν (liq) cm⁻¹ : 3444 (br) , 1738
Mass spectrum m/z : 296 (M⁺)
NMR spectrum δ (CDCl₃) ppm : 1.29-1.35(1H, m), 1.40-1.51(3H, m), 1.63-1.74(3H, m), 1.78(1H, d, J=3Hz), 1.79-1.84(1H, m), 1.95(2H, qn, J=7.5Hz), 2.33(2H, t, J=7.5Hz), 2.64(2H, t, J=7.5Hz), 3.67(3H, s), 4.42(2H, dt, J=47, 6Hz), 4.63-4.66(1H, m), 7.16(2H, d, J=8Hz), 7.26(2H, d, J=8Hz)

### Example 60: Methyl 4-[5-(1-Azido-5,5,6,6,6-pentafluorohexyl)-2-thienyl]butyrate

To a solution of 9.15 g of methyl 4-[5-(5,5,6,6,6-pentafluoro-1-hydroxyhexyl)-2-thienyl]butyrate in 45 ml of dry diethyl ether, 1.96 ml of thionyl chloride was added dropwise under ice-cooling, and stirring was continued at room temperature for 1 hour. The reaction solvent was removed under reduced pressure, and the residue obtained was added with 20 ml of N,N-dimethylformamide and 3.17 g of sodium azide, and then the mixture was stirred at 40°C for 1 hour. The reaction solution was diluted with water and extracted with diethyl ether. The ether layer was washed with water, dried, and then the solvent was removed to obtain 9.19 g of pale brown oil.
IR spectrum ν (liq) cm⁻¹ : 2104, 1738
Mass spectrum m/z : 399 (M⁺)
NMR spectrum δ (CDCl₃) ppm : 1.60-1.69(1H, m), 1.70-1.83(1H, m), 1.84-2.10(6H, m), 2.38(2H, t, J=7.5Hz), 2.85(2H, t, J=7.5Hz), 3.68(3H, s), 4.61(1H, t, J=7Hz), 6.68(1H, d, J=3.5Hz), 6.84(1H, d, J=3.5Hz)

The compounds of Example 61 through 72 were obtained in the same manner as described in Example 60.

### Example 61: Methyl 5-[5-(1-Azido-5,5,6,6,6-pentafluorohexyl)-2-thienyl]valerate

Appearance pale yellow oil
IR spectrum ν (liq) cm⁻¹ : 2104 , 1738
NMR spectrum δ (CDCl₃) ppm : 1.61-1.81(6H, m), 1.84-1.97(2H, m), 2.00-2.11(2H, m), 2.35(2H, t, J=7Hz), 2.82(2H, t, J=6.5Hz), 3.67(3H, s), 4.60(1H, t, J=7Hz), 6.67(1H, d, J=3.5Hz), 6.83(1H, d, J=3.5Hz)

### Example 62: Methyl 4-[5-(1-Azido-5,5,5-trifluoropentyl)-2-thienyl]butyrate

Appearance pale brown oil
IR spectrum ν (liq) cm⁻¹ : 2104 , 1738
Mass spectrum m/z : 349 (M⁺)
NMR spectrum δ (CDCl₃) ppm : 1.58-1.65(1H, m), 1.67-1.77(1H, m), 1.83-1.92(2H, m), 2.00(2H, qn, J=7.5Hz), 2.07-2.16(2H, m), 2.38(2H, t, J=7.5Hz), 2.85(2H, t, J=7.5Hz), 3.68(3H, s), 4.59(1H, t, J=7.5Hz), 6.68(1H, d, J=3.5Hz), 6.83(1H, d, J=3.5Hz)

### Example 63: Methyl 4-[5-(1-Azido-6,6,6-trifluorohexyl)-2-thienyl]butyrate

Appearance pale reddish brown oil
IR spectrum ν (liq) cm⁻¹ : 2104 , 1738
Mass spectrum m/z : 363 (M⁺)
NMR spectrum δ (CDCl₃) ppm : 1.38-1.45(1H, m), 1.47-1.62(3H, m), 1.78-1.91(2H, m), 2.00(2H, qn, J=7.5Hz), 2.04-2.13(2H, m), 2.38(2H, t, J=7.5Hz), 2.85(2H, t, J=7.5Hz), 3.68(3H, s), 4.57(1H, t, J=7.5Hz), 6.67(1H, d, J=3Hz), 6.82(1H, d, J=3Hz)

### Example 64: Methyl 4-[5-(1-Azido-7,7,7-trifluoroheptyl)-2-thienyl]butyrate

Appearance pale brown oil
IR spectrum ν (liq) cm⁻¹ : 2104 , 1738
Mass spectrum m/z : 377 (M⁺)
NMR spectrum δ (CDCl₃) ppm : 1.32-1.49(4H, m), 1.54-1.60(2H, m), 1.80-1.87(2H, m), 1.97-2.10(4H, m), 2.38(2H, t, J=7.5Hz), 2.84(2H, t, J=7.5Hz), 3.68(3H, s), 4.55(1H, t, J=7.5Hz), 6.67(1H, d, J=3.5Hz), 6.81(1H, d, J=3.5Hz)

### Example 65: Methyl 4-[5-(1-Azido-6,6,7,7,7-pentafluoroheptyl)-2-thienyl]butyrate

Appearance pale brown oil
IR spectrum ν (liq) cm⁻¹ : 2104 , 1740
Mass spectrum m/z : 413 (M⁺)
NMR spectrum δ (CDCl₃) ppm : 1.37-1.47(1H, m), 1.48-1.57(1H, m), 1.59-1.66(1H, m), 1.72-1.92(3H, m), 1.95-2.10(4H, m), 2.38(2H, t, J=7.5Hz), 2.85(2H, t, J=7.5Hz), 3.68(3H, s), 4.58(1H, t, J=7.5Hz), 6.68(1H, d, J=3.5Hz), 6.82(1H, d, J=3.5Hz)

### Example 66: Methyl 4-[5-(1-Azido-4,4,5,5,6,6,6-heptafluorohexyl)-2-thienyl]butyrate

Appearance pale brown oil
IR spectrum ν (liq) cm⁻¹ : 2108 , 1738
Mass spectrum m/z : 435 (M⁺)
NMR spectrum δ (CDCl₃) ppm : 2.01(2H, qn, J=7.5Hz), 2.07-2.32(7H, m), 2.38(2H, t, J=7.5Hz), 2.86(2H, t, J=7.5Hz), 3.68(3H, s), 4.69(1H, t, J=7Hz), 6.70(1H, d, J=3.5Hz), 6.86(1H, d, J=3.5Hz)

### Example 67: Methyl 4-[5-(1-Azido-5,5,6,6,7,7,7-heptafluoroheptyl)-2-thienyl]butyrate

Appearance pale yellow oil
IR spectrum ν (liq) cm⁻¹ : 2108 , 1738
Mass spectrum m/z : 449 (M⁺)
NMR spectrum δ (CDCl₃) ppm : 1.62-1.71(1H, m), 1.74-1.83(1H, m), 1.84-1.94(2H, m), 1.97-2.15(4H, m), 2.38(2H, t, J=7.5Hz), 2.85(2H, t, J=7.5Hz), 3.68(3H, s), 4.61(1H, t, J=7.5Hz), 6.68(1H, d, J=3.5Hz), 6.84(1H, d, J=3.5Hz)

### Example 68: Methyl 4-[5-(1-Azido-4,4,5,5,6,6,7,7,7-nonafluoroheptyl)-2-thienyl]butyrate

Appearance pale brown oil
IR spectrum ν (liq) cm⁻¹ : 2108 , 1740
Mass spectrum m/z : 485 (M⁺)
NMR spectrum δ (CDCl₃) ppm : 2.01(2H, qn, J=7.5Hz), 2.08-2.33(4H, m), 2.38(2H, t, J=7.5Hz), 2.86(2H, t, J=7.5Hz), 3.68(3H, s), 4.69(1H, t, J=7Hz), 6.70(1H, d, J=3.5Hz), 6.86(1H, d, J=3.5Hz)

### Example 69: Methyl 4-[5-(1-Azido-6,6,6-trifluoro-5-methylhexyl)-2-thienyl]butyrate

Appearance pale red oil
IR spectrum ν (liq) cm⁻¹ : 2104 , 1738
Mass spectrum m/z : 377 (M⁺)
NMR spectrum δ (CDCl₃) ppm : 1.08(1.5H, d, J=6.5Hz), 1.09(1.5H, d, J=6.5Hz), 1.27-1.39(1.5H, m), 1.41-1.53(1H, m), 1.54-1.62(0.5H, m), 1.66-1.73(1H, m), 1.76-1.92(2H, m), 2.00(2H, qn, J=7.5Hz), 2.07-2.21(1H, m), 2.38(2H, t, J=7.5Hz), 2.85(2H, t, J=7.5Hz), 3.68(3H, s), 4.56-4.59(1H, m), 6.67(1H, d, J=3.5Hz), 6.82(1H, d, J=3.5Hz)

### Example 70: Methyl 4-[5-(1-Azido-5,6,6,6-tetrafluoro-5-trifluoromethylhexyl)-2-thienyl]butyrate

Appearance pale yellow oil
IR spectrum ν (liq) cm⁻¹ : 2104 , 1740
Mass spectrum m/z : 449 (M⁺)
NMR spectrum δ (CDCl₃) ppm : 1.58-1.67(1H, m), 1.70-1.93(3H, m), 2.00(2H, qn, J=7.5Hz), 2.05-2.14(2H, m), 2.38(2H, t, J=7.5Hz), 2.85(2H, t, J=7.5Hz), 3.68(3H, s), 4.59-4.62(1H, m), 6.68(1H, d, J=3Hz), 6.83(1H, d, J=3Hz)

### Example 71: Methyl 4-[5-(1-Azido-4,4,5,5,6,7,7,7-octafluoro-6-trifluoromethylheptyl)-2-thienyl]butyrate

Appearance pale yellow oil
IR spectrum ν (liq) cm⁻¹ : 2108 , 1738
Mass spectrum m/z : 535 (M⁺)
NMR spectrum δ (CDCl₃) ppm : 2.01(2H, qn, J=7.5Hz), 2.07-2.35(4H, m), 2.38(2H, t, J=7.5Hz), 2.86(2H, t, J=7.5Hz), 3.68(3H, s), 4.68(1H, t, J=7Hz), 6.70(1H, d, J=3.5Hz), 6.86(1H, d, J=3.5Hz)

### Example 72: Methyl 4-[5-(1-Azido-6-fluorohexyl)-2-thienyl]butyrate

Appearance pale brown oil
IR spectrum ν (liq) cm⁻¹ : 2104 , 1738
Mass spectrum m/z : 327 (M⁺)
NMR spectrum δ (CDCl₃) ppm : 1.39-1.53(4H, m), 1.64-1.91(4H, m), 2.00(2H, qn, J=7.5Hz), 2.38(2H, t, J=7.5Hz), 2.84(2H, t, J=7.5Hz), 3.68(3H, s), 4.43(2H, dt, J=47, 6Hz), 4.56(1H, t, J=7.5Hz), 6.67(1H, d, J=3Hz), 6.82(1H, d, J=3Hz)

### Example 73: Methyl 4-[4-(1-Azido-5,5,6,6,6-pentafluorohexyl)phenyl]butyrate

To a solution of 7.97 g of methyl 4-[4-(5,5,6,6,6-pentafluoro-1-hydroxyhexyl)phenyl]butyrate in 25 ml of methylene chloride, 2.05 ml of thionyl chloride was added, and stirring was continued at room temperature for 10 minutes and then at 40°C for 1 hour. The reaction solvent was removed under reduced pressure, and the residue obtained was added with 25 ml of N,N-dimethylformamide and 2.81 g of sodium azide, and the mixture was stirred at 60°C for 3 hours. The reaction mixture was diluted with water and extracted with diethyl ether. The ether layer was washed with water, dried, and then the solvent was removed to obtain 8.21 g of pale yellow oil.
IR spectrum ν (liq) cm⁻¹ : 2104 , 1738
Mass spectrum m/z : 393 (M⁺)
NMR spectrum δ (CDCl₃) ppm : 1.55-1.62(1H, m), 1.70-1.92(3H, m), 1.94-2.10(4H, m), 2.34(2H, t, J=7.5Hz), 2.66(2H, t, J=7.5Hz), 3.67(3H, s), 4.42(1H, dd, J=8, 6Hz), 7.21(4H,s)

The compounds of Example 74 through 83 were obtained in the same manner as described in Example 73.

### Example 74: Methyl 5-[4-(1-Azido-5,5,6,6,6-pentafluorohexyl)phenyl]valerate

Appearance pale yellow oil
IR spectrum ν (liq) cm⁻¹ : 2104 , 1738
NMR spectrum δ (CDCl₃) ppm : 1.54-1.93(8H, m), 1.98-2.09(2H, m), 2.34(2H, t, J=7.5Hz), 2.64(2H, t, J=7.5Hz), 3.67(3H, s), 4.42(1H, dd, J=8, 5.5Hz), 7.20(4H, s)

### Example 75: Methyl 4-[4-(1-Azido-6,6,6-trifluorohexyl)phenyl]butyrate

Appearance pale yellow oil
IR spectrum ν (liq) cm⁻¹ : 2104 , 1738
NMR spectrum δ (CDCl₃) ppm : 1.30-1.39(1H, m), 1.44-1.52(1H, m) 1.57(2H, qn, J=7.5Hz), 1.70-1.77(1H, m), 1.80-1.87(1H, m), 1.96(2H, q, J=7.5Hz), 2.00-2.10(2H, m), 2.34(2H, t, J=7.5Hz), 2.66(2H, t, J=7.5Hz), 3.67(3H, s), 4.77(1H, t, J=7.5Hz), 7.20(4H, s)

### Example 76: Methyl 4-[4-(1-Azido-6,6,7,7,7-pentafluoroheptyl)phenyl]butyrate

Appearance pale yellow oil
IR spectrum ν (liq) cm⁻¹ : 2104 , 1738
Mass spectrum m/z : 407 (M⁺)
NMR spectrum δ (CDCl₃) ppm : 1.31-1.40(1H, m), 1.44-1.53(1H, m), 1.57-1.63(2H, m), 1.72-1.79(1H, m), 1.81-1.89(1H, m), 1.93-2.05(4H, m), 2.34(2H, t, J=7.5Hz), 2.66(2H, t, J=7.5Hz), 3.67(3H, s), 4.39(1H, t, J=7Hz), 7.20(4H, s)

### Example 77: Methyl 4-[4-(1-Azido-4,4,5,5,6,6,6-heptafluorohexyl)phenyl]butyrate

Appearance pale brown oil
IR spectrum ν (liq) cm⁻¹ : 2108 , 1740
NMR spectrum δ (CDCl₃) ppm : 1.94-2.14(5H, m), 2.15-2.28(1H, m), 2.34(2H, t, J=7.5Hz), 2.67(2H, t, J=7.5Hz), 3.67(3H, s), 4.48-4.51(1H, m) 7.23(4H, s)

### Example 78: Methyl 4-[4-(1-Azido-5,5,6,6,7,7,7-heptafluoroheptyl)phenyl]butyrate

Appearance pale yellow oil
IR spectrum ν (liq) cm⁻¹ : 2108 , 1740
NMR spectrum δ (CDCl₃) ppm : 1.56-1.65(1H, m), 1.70-1.92(3H, m), 1.97(2H, qn, J=7.5Hz), 2.00-2.18(2H, m), 2.34(2H, t, J=7.5Hz), 2.66(2H, t, J=7.5Hz), 3.67(3H, s), 4.42(1H, dd, J=8, 6Hz), 7.21(4H, s)

### Example 79: Methyl 4-[4-(1-Azido-4,4,5,5,6,6,7,7,7-nonafluoroheptyl)phenyl]butyrate

Appearance colorless oil
IR spectrum ν (liq) cm⁻¹ : 2108 , 1740
NMR spectrum δ (CDCl₃) ppm : 1.92-2.12(5H, m), 2.17-2.23(1H, m), 2.34(2H, t, J=7.5Hz), 2.67(2H, t, J=7.5Hz), 3.67(3H, s), 4.48-4.51(1H, m), 7.23(4H,s)

### Example 80: Methyl 4-[4-(1-Azido-6,6,6-trifluoro-5-methylhexyl)phenyl]butyrate

Appearance pale yellow oil
IR spectrum ν (liq) cm⁻¹ : 2104 , 1740
NMR spectrum δ (CDCl₃) ppm : 1.06(1.5H, d, J=7Hz), 1.07(1.5H, d, J=7Hz), 1.22-1.35(1.5H, m), 1.39-1.45(1H, m), 1.50-1.59(0.5H, m), 1.63-1.86(3H, m), 1.96(2H, qn, J=7.5Hz), 2.04-2.16(1H, m), 2.34(2H, t, J=7.5Hz), 2.66(2H, t, J=7.5Hz), 3.67(3H, s), 4.37-4.41(1H, m), 7.20(4H, s)

### Example 81: Methyl 4-[4-(1-Azido-5,6,6,6-tetrafluoro-5-trifluoromethylhexyl)phenyl]butyrate

Appearance pale yellow oil
IR spectrum ν (liq) cm⁻¹ : 2104 , 1740
NMR spectrum δ (CDCl₃) ppm : 1.52-1.64(1H, m), 1.67-1.80(2H, m), 1.81-1.90(1H, m), 1.96(2H, qn, J=7.5Hz), 2.02-2.13(2H, m), 2.34(2H, t, J=7.5Hz), 2.65(2H, t, J=7.5Hz), 3.67(3H, s), 4.42(1H, dd, J=8, 5.5Hz), 7.21(4H, s)

### Example 82: Methyl 4-[4-(1-Azido-4,4,5,5,6,7,7,7-octafluoro-6-trifluoromethylheptyl)-phenyl]butyrate

Appearance pale yellow oil
IR spectrum ν (liq) cm⁻¹ : 2104 , 1740
NMR spectrum δ (CDCl₃) ppm : 1.92-2.14(5H, m), 2.18-2.24(1H, m), 2.34(2H, t, J=7.5Hz), 2.67(2H, t, J=7.5Hz), 3.67(3H, s), 4.48-4.51(1H, m), 7.23(4H, s)

### Example 83: Methyl 4-[4-(1-Azido-6-fluorohexyl)phenyl]butyrate

Appearance pale yellow oil
IR spectrum ν (liq) cm⁻¹ : 2100 , 1738
NMR spectrum δ (CDCl₃) ppm : 1.28-1.37(1H, m), 1.40-1.46(3H, m), 1.62-1.76(3H, m), 1.80-1.90(1H, m), 1.96(2H, qn, J=7.5Hz), 2.34(2H, t, J=7.5Hz), 2.66(2H, t, J=7.5Hz), 3.67(3H, s), 4.38(1H, t, J=7Hz), 4.42(2H, dt, J=47.5, 6Hz), 7.19(2H, d, J=8.5Hz), 7.21(2H, d, J=8.5Hz)

### Example 84: Methyl 4-[5-(1-Amino-5,5,6,6,6-pentafluorohexyl)-2-thienyl]butyrate

To a solution of 9.12 g of methyl 4-[5-(1-azido-5,5,6,6,6-pentafluorohexyl)-2-thienyl]butyrate in 90 ml of dry tetrahydrofuran, 7.76 g of triphenylphosphine was added, and the mixture was stirred at 50°C for 1 hour. Then, 20.5 ml of water was added to the reaction mixture and stirring was continued at 50°C for 16 hours. After the reaction solvent was removed under reduced pressure, the residue was acidified with dilute hydrochloric acid and washed with diethyl ether. The acidic aqueous layer was made alkaline with potassium carbonate and extracted with diethyl ether. The ether layer was washed with water, dried, and then the solvent was removed to obtain 5.75 g of pale yellow oil.
IR spectrum ν (liq) cm⁻¹ : 3384 , 1738
Mass spectrum m/z : 373 (M⁺)
NMR spectrum δ (CDCl₃) ppm : 1.48-1.64(3H, m), 1.67-1.72(1H, m), 1.77(2H, qn, J=7.5Hz), 1.96-2.08(4H, m), 2.37(2H, t, J=7.5Hz), 2.82(2H, t, J=7.5Hz), 3.68(3H, s), 4.11(1H, t, J=6.5Hz), 6.61(1H, d, J=3.5Hz), 6.70(1H, d, J=3.5Hz)

The compounds of Example 85 through 107 were obtained in the same manner as described in Example 84.

### Example 85: Methyl 5-[5-(1-Amino-5,5,6,6,6-pentafluorohexyl)-2-thienyl]valerate

Appearance colorless oil
IR spectrum ν (liq) cm⁻¹ : 3384 , 1738
NMR spectrum δ (CDCl₃) ppm : 1.54-1.75(8H, m), 1.76(2H, qn, J=7Hz), 1.97-2.08(2H, m), 2.34(2H, t, J=7.5Hz), 2.79(2H, t, J=6.5Hz), 3.67(3H, s), 4.11(1H, t, J=6.5Hz), 6.60(1H, d, J=3.5Hz), 6.69(1H, d, J=3.5Hz)

### Example 86: Methyl 4-[5-(1-Amino-5,5,5-trifluoropentyl)-2-thienyl]butyrate

Appearance pale yellow oil
IR spectrum ν (liq) cm⁻¹ : 3384 , 1738
Mass spectrum m/z : 323 (M⁺)
NMR spectrum δ (CDCl₃) ppm : 1.48-1.70(4H, m), 1.74-1.78(2H, m), 1.99(2H, qn, J=7.5Hz), 2.04-2.13(2H, m), 2.37(2H, t, J=7.5Hz), 2.82(2H, t, J=7.5Hz), 3.67(3H, s), 4.09(1H, t, J=6.5Hz), 6.61(1H, d, J=3.5Hz), 6.69(1H, d, J=3.5Hz)

### Example 87: Methyl 4-[5-(1-Amino-6,6,6-trifluorohexyl)-2-thienyl]butyrate

Appearance pale yellow oil
IR spectrum ν (liq) cm⁻¹ : 3384 , 1738
Mass spectrum m/z : 337 (M⁺)
NMR spectrum δ (CDCl₃) ppm : 1.32-1.48(2H, m), 1.49-1.60(4H, m), 1.72(2H, q, J=7.5Hz), 1.98(2H, qn, J=7.5Hz), 2.03-2.10(2H, m), 2.37(2H, t, J=7.5Hz), 2.82(2H, t, J=7.5Hz), 3.67(3H, s), 4.08(1H, t, J=7Hz), 6.60(1H, d, J=3Hz), 6.68(1H, d, J=3Hz)

### Example 88: Methyl 4-[5-(1-Amino-7,7,7-trifluoroheptyl)-2-thienyl]butyrate

Appearance pale yellow oil
IR spectrum ν (liq) cm⁻¹ : 3384 , 1740
Mass spectrum m/z : 351 (M⁺)
NMR spectrum δ (CDCl₃) ppm : 1.31-1.43(4H, m), 1.52-1.61(4H, m), 1.68-1.72(2H, m), 1.96-2.09(4H, m), 2.37(2H, t, J=7.5Hz), 2.82(2H, t, J=7.5Hz), 3.67(3H, s), 4.07(1H, t, J=6.5Hz), 6.60(1H, d, J=3.5Hz), 6.68(1H, d, J=3.5Hz)

### Example 89: Methyl 4-[5-(1-Amino-6,6,7,7,7-pentafluoroheptyl)-2-thienyl]butyrate

Appearance pale orange oil
IR spectrum ν (liq) cm⁻¹ : 3420 , 1738
Mass spectrum m/z : 387 (M⁺)
NMR spectrum δ (CDCl₃) ppm : 1.32-1.50(2H, m), 1.52-1.67(4H, m), 1.73(2H, q, J=7.5Hz), 1.94-2.06(4H, m), 2.37(2H, t, J=7.5Hz), 2.82(2H, t, J=7.5Hz), 3.68(3H, s), 4.09(1H, t, J=7Hz), 6.61(1H, d, J=3Hz), 6.69(1H, d, J=3Hz)

### Example 90: Methyl 4-[5-(1-Amino-4,4,5,5,6,6,6-heptafluorohexyl)-2-thienyl]butyrate

Appearance pale yellow oil
IR spectrum ν (liq) cm⁻¹ : 3392 , 1738
Mass spectrum m/z : 409 (M⁺)
NMR spectrum δ (CDCl₃) ppm : 1.55(2H, s), 1.94-2.28(6H, m), 2.37(2H, t, J=7.5Hz), 2.83(2H, t, J=7.5Hz), 3.68(3H, s), 4.15(1H, t, J=7Hz), 6.63(1H, d, J=3.5Hz), 6.71(1H, d, J=3.5Hz)

### Example 91: Methyl 4-[5-(1-Amino-5,5,6,6,7,7,7-heptafluoroheptyl)-2-thienyl]butyrate

Appearance pale yellow oil
IR spectrum ν (liq) cm⁻¹ : 3388 , 1738
NMR spectrum δ (CDCl₃) ppm : 1.53-1.67(3H, m), 1.68-1.80(3H, m), 1.99(2H, qn, J=7.5Hz), 2.03-2.11(2H, m), 2.37(2H, t, J=7.5Hz), 2.82(2H, t, J=7.5Hz), 3.67(3H, s), 4.11(1H, t, J=6.5Hz), 6.61(1H, d, J=3.5Hz), 6.70(1H, d, J=3.5Hz)

### Example 92: Methyl 4-[5-(1-Amino-4,4,5,5,6,6,7,7,7-nonafluoroheptyl)-2-thienyl]butyrate

Appearance pale brown oil
IR spectrum ν (liq) cm⁻¹ : 3380 , 1738
Mass spectrum m/z : 459 (M⁺)
NMR spectrum δ (CDCl₃) ppm : 1.99(2H, qn, J=7.5Hz), 2.02-2.27(6H, m), 2.37(2H, t, J=7.5Hz), 2.82(2H, t, J=7.5Hz), 3.67(3H, s), 4.21-4.27(1H, m), 6.64(1H, d, J=3.5Hz), 6.79(1H, d, J=3.5Hz)

### Example 93: Methyl 4-[5-(1-Amino-6,6,6-trifluoro-5-methylhexyl)-2-thienyl]butyrate

Appearance pale red oil
IR spectrum ν (liq) cm⁻¹ : 3384 , 1738
Mass spectrum m/z : 351 (M⁺)
NMR spectrum δ (CDCl₃) ppm : 1.06(1.5H, d, J=7Hz), 1.07(1.5H, d, J=7Hz), 1.22-1.77(8H, m), 1.98(2H, qn, J=7.5Hz), 2.04-2.17(1H, m), 2.37(2H, t, J=7.5Hz), 2.82(2H, t, J=7.5Hz), 3.67(3H, s), 4.09(1H, t, J=6Hz), 6.61(1H, d, J=3.5Hz), 6.69(1H, d, J=3.5Hz)

### Example 94: Methyl 4-[5-(1-Amino-5,6,6,6-tetrafluoro-5-trifluoromethylhexyl)-2-thienyl]butyrate

Appearance pale yellow oil
IR spectrum ν (liq) cm⁻¹ : 3384 , 1738
Mass spectrum m/z : 423 (M⁺)
NMR spectrum δ (CDCl₃) ppm : 1.74-1.86(6H, m), 1.98(2H, qn, J=7.5Hz), 2.02-2.12(2H, m), 2.37(2H, t, J=7.5Hz), 2.82(2H, t, J=7.5Hz), 3.67(3H, s), 4.12(1H, t, J=6.5Hz), 6.61(1H, d, J=3.5Hz), 6.70(1H, d, J=3.5Hz)

### Example 95: Methyl 4-[5-(1-Amino-4,4,5,5,6,7,7,7-octafluoro-6-trifluoromethylheptyl)-2-thienyl]butyrate

Appearance colorless oil
IR spectrum ν (liq) cm⁻¹ : 3388 , 1738
Mass spectrum m/z : 509 (M⁺)
NMR spectrum δ (CDCl₃) ppm : 1.56(2H, brs), 1.92-2.30(6H, m), 2.37(2H, t, J=7.5Hz), 2.83(2H, t, J=7.5Hz), 3.67(3H, s), 4.15(1H, t, J=6.5Hz), 6.62(1H, d, J=3Hz), 6.71(1H, d, J=3Hz)

### Example 96: Methyl 4-[5-(1-Amino-6-fluorohexyl)-2-thienyl]butyrate

Appearance pale yellow oil
IR spectrum ν (liq) cm⁻¹ : 3384 , 1738
Mass spectrum m/z : 301 (M⁺)
NMR spectrum δ (CDCl₃) ppm : 1.28-1.46(4H, m), 1.59(2H,s), 1.63-1.75(4H, m), 1.98(2H, qn, J=7.5Hz), 2.37(2H, t, J=7.5Hz), 2.82(2H, t, J=7.5Hz), 3.67(3H, s), 4.08(1H, t, J=6.5Hz), 4.42(2H, dt, J=47.5, 6Hz), 6.60(1H, d, J=3.5Hz), 6.69(1H, d, J=3.5Hz)

### Example 97: Methyl 4-[4-(1-Amino-5,5,6,6,6-pentafluorohexyl)phenyl]butyrate

Appearance pale yellow oil
IR spectrum ν (liq) cm⁻¹ : 3384 , 1738
NMR spectrum δ (CDCl₃) ppm : 1.43-1.77(6H, m), 1.92-2.03(4H, m), 2.33(2H, t, J=7.5Hz), 2.64(2H, t, J=7.5Hz), 3.67(3H, s), 3.87(1H, t, J=7Hz), 7.15(2H, d, J=8.5Hz), 7.21(2H, d, J=8.5Hz)

### Example 98: Methyl 5-[4-(1-Amino-5,5,6,6,6-pentafluorohexyl)phenyl]valerate

Appearance pale yellow oil
IR spectrum ν (liq) cm⁻¹ : 3384 , 1740
NMR spectrum δ (CDCl₃) ppm : 1.43-1.77(10H, m), 1.95-2.05(2H, m), 2.34(2H, t, J=7Hz), 2.62(2H, t, J=7Hz), 3.66(3H, s), 3.87(1H, t, J=6.5Hz), 7.14(2H, d, J=8Hz), 7.20(2H, d, J=8Hz)

### Example 99: Methyl 4-[4-(1-Amino-6,6,6-trifluorohexyl)phenyl]butyrate

Appearance pale yellow oil
IR spectrum ν (liq) cm⁻¹ : 3380 , 1738
NMR spectrum δ (CDCl₃) ppm : 1.24-1.31(1H, m), 1.37-1.46(1H, m), 1.46-1.72(6H, m), 1.95(2H, qn, J=7.5Hz), 2.00-2.08(2H, m), 2.33(2H, t, J=7.5Hz), 2.64(2H, t, J=7.5Hz), 3.67(3H, s), 3.85(1H, t, J=6.5Hz), 7.14(2H, d, J=8Hz), 7.21(2H, d, J=8Hz)

### Example 100: Methyl 4-[4-(1-Amino-6,6,7,7,7-pentafluoroheptyl)phenyl]butyrate

Appearance pale yellow oil
IR spectrum ν (liq) cm⁻¹ : 3384 , 1738
NMR spectrum δ (CDCl₃) ppm : 1.24-1.33(1H, m), 1.37-1.46(1H, m), 1.47-1.75(6H, m), 1.94-2.03(4H, m), 2.33(2H, t, J=7.5Hz), 2.64(2H, t, J=7.5Hz), 3.67(3H, s), 3.85(1H, t, J=7Hz), 7.14(2H, d, J=8Hz), 7.21(2H, d, J=8Hz)

### Example 101: Methyl 4-[4-(1-Amino-4,4,5,5,6,6,6-heptafluorohexyl)phenyl]butyrate

Appearance pale yellow oil
IR spectrum ν (liq) cm⁻¹ : 3384 , 1738
NMR spectrum δ (CDCl₃) ppm : 1.47(2H, brs), 1.91-2.08(5H, m), 2.09-2.23(1H, m), 2.33(2H, t, J=7.5Hz), 2.64(2H, t, J=7.5Hz), 3.67(3H, s), 3.91(1H, t, J=6.5Hz), 7.12(2H, d, J=8Hz), 7.22(2H, d, J=8Hz)

### Example 102: Methyl 4-[4-(1-Amino-5,5,6,6,7,7,7-heptafluoroheptyl)phenyl]butyrate

Appearance pale yellow oil
IR spectrum ν (liq) cm⁻¹ : 3384 , 1738
NMR spectrum δ (CDCl₃) ppm : 1.38-1.58(2H, m), 1.64-1.77(4H, m), 1.95(2H, qn, J=7.5Hz), 1.98-2.09(2H, m), 2.33(2H, t, J=7.5Hz), 2.64(2H, t, J=7.5Hz), 3.67(3H, s), 3.88(1H, t, J=6.5Hz), 7.15(2H, d, J=8Hz), 7.21(2H, d, J=8Hz)

### Example 103: Methyl 4-[4-(1-Amino-4,4,5,5,6,6,7,7,7-nonafluoroheptyl)phenyl]butyrate

Appearance pale yellow oil
IR spectrum ν (liq) cm⁻¹ : 3384 , 1738
NMR spectrum δ (CDCl₃) ppm : 1.53(2H, brs), 1.91-2.09(5H, m), 2.10-2.24(1H, m), 2.33(2H, t, J=7.5Hz), 2.64(2H, t, J=7.5Hz), 3.67(3H, s), 3.92(1H, t, J=6.5Hz), 7.17(2H, d, J=8Hz), 7.22(2H, d, J=8Hz)

### Example 104: Methyl 4-[4-(1-Amino-6,6,6-trifluoro-5-methylhexyl)phenyl]butyrate

Appearance pale yellow oil
IR spectrum ν (liq) cm⁻¹ : 3388 , 1738
NMR spectrum δ (CDCl₃) ppm : 1.03(1.5H, d, J=7Hz), 1.05(1.5H, d, J=7Hz), 1.12-1.23(0.5H, m), 1.25-1.38(2H, m), 1.42-1.57(2.5H, m), 1.58-1.72(3H, m), 1.95(2H, qn, J=7.5Hz), 2.03-2.13(1H, m), 2.33(2H, t, J=7.5Hz), 2.64(2H, t, J=7.5Hz), 3.67(3H, s), 3.83-3.90(1H, m), 7.14(2H, d, J=8Hz), 7.21(2H, d, J=8Hz)

### Example 105: Methyl 4-[4-(1-Amino-5,6,6,6-tetrafluoro-5-trifluoromethylhexyl)phenyl]butyrate

Appearance pale yellow oil
IR spectrum ν (liq) cm⁻¹ : 3372 , 1738
NMR spectrum δ (CDCl₃) ppm : 1.40-1.57(3H, m), 1.60-1.75(3H, m), 1.95 (2H, qn, J=7.5Hz), 2.00-2.10(2H, m), 2.33(2H, t, J=7.5Hz), 2.64(2H, t, J=7.5Hz), 3.67(3H, s), 3.87(1H, t, J=6.5Hz), 7.15(2H, d, J=8Hz), 7.21(2H, d, J=8Hz)

### Example 106: Methyl 4-[4-(1-Amino-4,4,5,5,6,7,7,7-octafluoro-6-trifluoromethylheptyl)phenyl]butyrate

Appearance pale yellow oil
IR spectrum ν (liq) cm⁻¹ : 3380 , 1738
NMR spectrum δ (CDCl₃) ppm : 1.48(2H, brs), 1.91-2.25(6H, m), 2.33(2H, t, J=7.5Hz), 2.64(2H, t, J=7.5Hz), 3.67(3H, s), 3.91(1H, t, J=6.5Hz), 7.16(2H, d, J=8Hz), 7.21(2H, d, J=8Hz)

### Example 107: Methyl 4-[4-(1-Amino-6-fluorohexyl)phenyl]butyrate

Appearance colorless oil
IR spectrum ν (liq) cm⁻¹ : 3384 , 1738
NMR spectrum δ (CDCl₃) ppm : 1.20-1.27(1H, m), 1.33-1.43(3H, m), 1.56(2H, s), 1.61-1.68(4H, m), 1.95(2H, qn, J=7.5Hz), 2.33(2H, t, J=7.5Hz), 2.63(2H, t, J=7.5Hz), 3.67(3H, s), 3.85(1H, t, J=7Hz), 4.40(2H, dt, J=47, 6Hz), 7.13(2H, d, J=8Hz), 7.21(2H, d, J=8Hz)

### Example 108: Methyl 4-[4-(1-Amino-5,5,6,6,6-pentafluorohexyl)phenyl]butyrate

To a solution of 30.0 g of methyl 4-[4-(5,5,6,6,6-pentafluoro-1-hydroxyiminohexyl)phenyl]butyrate in 300 ml of methanol containing 1% ammonia, 9 ml of Raney nickel was added, and hydrogenation was carried out under hydrogen pressure of 60kg/cm² at 50°C for 12 hours. After the catalyst was filtered off, the filtrate was concentrated under reduced pressure, and the residue was dissolved in toluene and washed with water. The toluene layer was dried and the solvent was removed to obtain 28.8 g of colorless oil. The product was identical to the compound obtained in Example 97.

### Example 109: Methyl 4-[5-[1-(4-Chlorophenylsulfonylamino)-5,5,6,6,6-pentafluorohexyl]-2-thienyl]butyrate

To a solution of 2.00 g of methyl 4-[5-(1-amino-5,5,6,6,6-pentafluorohexyl)-2-thienyl]butyrate and 0.85 ml of triethylamine in 8 ml of methylene chloride, 1.13 g of 4-chlorobenzenesulfonyl chloride was added under ice-cooling, and stirring was continued at room temperature for 1 hour. The reaction solution was washed successively with dilute hydrochloric acid and water, dried, and then the solvent was removed. The resulting residue was purified by column chromatography on silica gel (methylene chloride-n-hexane=2:1) to obtain 2.44 g of pale yellow viscous oil.
IR spectrum ν (liq) cm⁻¹ : 3272 , 1738
Mass spectrum m/z : 547, 549 (3:1, M⁺)
NMR spectrum δ (CDCl₃) ppm : 1.51-1.60(1H, m), 1.62-1.71(1H, m), 1.81-2.06(6H, m), 2.32(2H, t, J=7.5Hz), 2.71(2H, t, J=7.5Hz), 3.69(3H, s), 4.54(1H, q, J=7.5Hz), 4.83(1H, d, J=8Hz), 6.46(1H, d, J=3.5Hz), 6.48(1H, d, J=3.5Hz), 7.35(1H, d, J=8.5Hz), 7.64(1H, d, J=8.5Hz)

The compounds of Example 110 through 161 were obtained in the same manner as described in Example 109.

### Example 110: Methyl 4-[5-[5,5,6,6,6-Pentafluoro-1-(4-fluorophenylsulfonylamino)hexyl]-2-thienyl]butyrate

Appearance pale yellow viscous oil
IR spectrum ν (liq) cm⁻¹ : 3274 , 1738
Mass spectrum m/z : 531 (M⁺)
NMR spectrum δ (CDCl₃) ppm : 1.50-1.59(1H, m), 1.62-1.69(1H, m), 1.86-2.06(6H, m), 2.31(2H, t, J=7.5Hz), 2.72(2H, t, J=7.5Hz), 3.69(3H, s), 4.54(1H, q, J=7.5Hz), 4.72(1H, d, J=8Hz), 6.46(1H, d, J=3.5Hz), 6.47(1H, d, J=3.5Hz), 7.07(2H, t, J=8.5Hz), 7.73(2H, dd, J=8.5, 5Hz)

### Example 111: Methyl 4-[5-[5,5,6,6,6-Pentafluoro-1-(4-nitrophenylsulfonylamino)hexyl]-2-thienyl]butyrate

Appearance colorless needles (i-Pr₂O)
mp 84.5∼85°C

| Analysis for C₂₁H₂₃F₅N₂O₆S₂ | | | |
|---|---|---|---|
| Calculated % | C, 45.16; | H, 4.15; | N, 5.02 |
| Found % | C, 45.05; | H, 4.11; | N, 5.17 |

### Example 112: Methyl 5-[5-[1-(4-Chlorophenylsulfonylamino)-5,5,6,6,6-pentafluorohexyl]-2-thienyl]valerate

Appearance colorless plates (i-Pr₂O)
mp 70.5∼72°C

| Analysis for C₂₂H₂₅ClF₅NO₄S₂ | | | |
|---|---|---|---|
| Calculated % | C, 47.02; | H, 4.48; | N, 2.49 |
| Found % | C, 46.96; | H, 4.41; | N, 2.56 |

### Example 113: Methyl 5-[5-[5,5,6,6,6-Pentafluoro-1-(4-fluorophenylsulfonylamino)hexyl]-2-thienyl]valerate

Appearance pale yellow viscous oil
IR spectrum ν (liq) cm⁻¹ : 3272 , 1738
NMR spectrum δ (CDCl₃) ppm : 1.49-1.71(6H, m), 1.81-2.06(4H, m), 2.33(2H, t, J=7Hz), 2.68(2H, t, J=7Hz), 3.68(3H, s), 4.53(1H, q, J=7.5Hz), 4.83(1H, d, J=8Hz), 6.44(1H, d, J=3.5Hz), 6.46(1H, d, J=3.5Hz), 7.06(2H, t, J=8.5Hz), 7.73(2H, dd, J=8.5, 5Hz)

### Example 114: Methyl 4-[5-[1-(4-Chlorophenylsulfonylamino)-5,5,5-trifluoropentyl]-2-thienyl]butyrate

Appearance pale pink plates (i-Pr₂O)
mp 53.5∼54.5°C

| Analysis for C₂₀H₂₃ClF₃NO₄S₂ | | | |
|---|---|---|---|
| Calculated % | C, 4.66; | H, 48.24; | N, 2.81 |
| Found % | C, 4.72; | H, 48.10; | N, 2.79 |

### Example 115: Methyl 4-[5-[1-(4-Chlorophenylsulfonylamino)-6,6,6-trifluorohexyl]-2-thienyl]butyrate

Appearance colorless viscous oil
IR spectrum ν (liq) cm⁻¹ : 3272 , 1740
Mass spectrum m/z : 511, 513 (3:1, M⁺)
NMR spectrum δ (CDCl₃) ppm : 1.26-1.38(1H, m), 1.39-1.47(1H, m), 1.54(2H, qn, J=8Hz), 1.73-1.80(1H, m), 1.82-1.91(3H, m), 1.97-2.06(2H, m), 2.32(2H, t, J=7.5Hz), 2.71(2H, t, J=7.5Hz), 3.69(3H, s), 4.53(1H, q, J=7.5Hz), 4.83(1H, d, J=8Hz), 6.44(1H, d, J=3.5Hz), 6.46(1H, d, J=3.5Hz), 7.35(2H, t, J=8.5Hz), 7.63(2H, d, J=8.5Hz)

### Example 116: Methyl 4-[5-[6,6,6-Trifluoro-1-(4-fluorophenylsulfonylamino)hexyl]-2-thienyl]butyrate

Appearance colorless viscous oil
IR spectrum ν (liq) cm⁻¹ : 3276 , 1738
Mass spectrum m/z : 495 (M⁺)
NMR spectrum δ (CDCl₃) ppm : 1.27-1.36(1H, m), 1.38-1.47(1H, m), 1.54(2H, qn, J=8Hz), 1.74-1.82(1H, m), 1.84-1.91(3H, m), 1.97-2.07(2H, m), 2.31(2H, t, J=7.5Hz), 2.71(2H, t, J=7.5Hz), 3.69(3H, s), 4.52(1H, q, J=7.5Hz), 4.80(1H, d, J=8Hz), 6.44(1H, d, J=3.5Hz), 6.46(1H, d, J=3.5Hz), 7.06(2H, t, J=8.5Hz), 7.72(2H, dd, J=8.5, 5Hz)

### Example 117: Methyl 4-[5-[6,6,6-Trifluoro-1-(4-nitrophenylsulfonylamino)hexyl]-2-thienyl]butyrate

Appearance pale yellow viscous oil
IR spectrum ν (liq) cm⁻¹ : 3276 , 1738
NMR spectrum δ (CDCl₃) ppm : 1.30-1.40(1H, m), 1.42-1.50(1H, m), 1.52-1.60(2H, m), 1.77-1.94(4H, m), 2.00-2.10(2H, m), 2.28(2H, t, J=7.5Hz), 2.66(2H, t, J=7.5Hz), 3.69(3H, s), 4.62(1H, q, J=7.5Hz), 4.93(1H, d, J=7.5Hz), 6.42(1H, s), 6.49(1H, d, J=3Hz), 7.84(2H, d, J=9Hz), 8.19(2H, d, J=9Hz)

### Example 118: Methyl 4-[5-[1-(4-Chlorophenylsulfonylamino)-7,7,7-trifluoroheptyl]-2-thienyl]butyrate

Appearance colorless viscous oil
IR spectrum ν (liq) cm⁻¹ : 3280 , 1738
Mass spectrum m/z : 525, 527 (3:1, M⁺)
NMR spectrum δ (CDCl₃) ppm : 1.24-1.38(4H, m), 1.47-1.53(2H, m), 1.74-1.90(4H, m), 1.97-2.05(2H, m), 2.32(2H, t, J=7.5Hz), 2.71(2H, t, J=7.5Hz), 3.69(3H, s), 4.53(1H, q, J=7.5Hz), 4.72(1H, d, J=8Hz), 6.44(1H, d, J=3.5Hz), 6.47(1H, d, J=3.5Hz), 7.34(2H, d, J=8.5Hz), 7.62(2H, d, J=8.5Hz)

### Example 119: Methyl 4-[5-[7,7,7-Trifluoro-1-(4-fluorophenylsulfonylamino)heptyl]-2-thienyl]butyrate

Appearance colorless viscous oil
IR spectrum ν (liq) cm⁻¹ : 3284 , 1738
Mass spectrum m/z : 509 (M⁺)
NMR spectrum δ (CDCl₃) ppm : 1.24-1.39(4H, m), 1.47-1.53(2H, m), 1.72-1.91(4H, m), 1.97-2.07(2H, m), 2.31(2H, t, J=7.5Hz), 2.71(2H, t, J=7.5Hz), 3.69(3H, s), 4.53(1H, q, J=7.5Hz), 4.72(1H, d, J=8Hz), 6.44(1H, d, J=3Hz), 6.46(1H, d, J=3Hz), 7.05(2H, t, J=8.5Hz), 7.71(2H, dd, J=8.5, 5Hz)

### Example 120: Methyl 4-[5-[1-(4-Chlorophenylsulfonylamino)-6,6,7,7,7-pentafluoroheptyl]-2-thienyl]butyrate

Appearance colorless plates (i-Pr₂O-n-Hexane)
mp 59.5∼60.5°C

| Analysis for C₂₂H₂₅ClF₅NO₄S₂ | | | |
|---|---|---|---|
| Calculated % | C, 47.02; | H, 4.48; | N, 2.49 |
| Found % | C, 46.96; | H, 4.45; | N, 2.53 |

### Example 121: Methyl 4-[5-[6,6,7,7,7-Pentafluoro-1-(4-fluorophenylsulfonylamino)heptyl]-2-thienyl]butyrate

Appearance pale yellow viscous oil
IR spectrum ν (liq) cm⁻¹ : 3284 , 1738
Mass spectrum m/z : 545 (M⁺)
NMR spectrum δ (CDCl₃) ppm : 1.27-1.37(1H, m), 1.39-1.49(1H, m), 1.54-1.60(2H, m), 1.75-1.83(1H, m), 1.84-2.02(5H, m), 2.31(2H, t, J=7.5Hz), 2.71(2H, t, J=7.5Hz), 3.69(3H, s), 4.53(1H, q, J=7.5Hz), 4.73(1H, d, J=7.5Hz), 6.45(1H, d, J=3.5Hz), 6.46(1H, d, J=3.5Hz), 7.06(2H, t, J=8.5Hz), 7.72(2H, dd, J=8.5, 5Hz)

### Example 122: Methyl 4-[5-[1-(4-Chlorophenylsulfonylamino)-4,4,5,5,6,6,6-heptafluorohexyl]-2-thienyl]butyrate

Appearance colorless needles (i-Pr₂O)
mp 79∼80°C

| Analysis for C₂₁H₂₁ClF₇NO₄S₂ | | | |
|---|---|---|---|
| Calculated % | C, 43.19; | H, 3.62; | N, 2.40 |
| Found % | C, 43.07; | H, 3.45; | N, 2.40 |

### Example 123: Methyl 4-[5-[4,4,5,5,6,6,6-Heptafluoro-1-(4-fluorophenylsulfonylamino)hexyl]-2-thienyl]butyrate

Appearance pale yellow viscous oil
IR spectrum ν (liq) cm⁻¹ : 3272 , 1738
Mass spectrum m/z : 567 (M⁺)
NMR spectrum δ (CDCl₃) ppm : 1.89(2H, qn, J=7.5Hz), 2.00-2.28(4H, m), 2.31(2H, t, J=7.5Hz), 2.72(2H, t, J=7.5Hz), 3.69(3H, s), 4.56(1H, q, J=7.5Hz), 4.82(1H, d, J=8.5Hz), 6.48(2H, s), 7.09(2H, t, J=8.5Hz), 7.75(2H, dd, J=8.5, 5Hz)

### Example 124: Methyl 4-[5-[1-(4-Chlorophenylsulfonylamino)-5,5,6,6,7,7,7-heptafluoroheptyl]-2-thienyl]butyrate

Appearance colorless viscous oil
IR spectrum ν (liq) cm⁻¹ : 3272 , 1738
NMR spectrum δ (CDCl₃) ppm : 1.52-1.61(1H, m), 1.63-1.72(1H, m), 1.82-2.09(6H, m), 2.32(2H, t, J=7.5Hz), 2.72(2H, t, J=7.5Hz), 3.69(3H, s), 4.54(1H, q, J=7.5Hz), 4.77(1H, d, J=8Hz), 6.46(1H, d, J=3.5Hz), 6.48(1H, d, J=3.5Hz), 7.36(2H, d, J=8.5Hz), 7.64(2H, d, J=8.5Hz)

### Example 125: Methyl 4-[5-[5,5,6,6,7,7,7-Heptafluoro-1-(4-fluorophenylsulfonylamino)heptyl]-2-thienyl]butyrate

Appearance colorless viscous oil
IR spectrum ν (liq) cm⁻¹ : 3288 , 1738
Mass spectrum m/z : 581 (M⁺)
NMR spectrum δ (CDCl₃) ppm : 1.52-1.61(1H, m), 1.63-1.72(1H, m), 1.82-2.09(6H, m), 2.31(2H, t, J=7.5Hz), 2.71(2H, t, J=7.5Hz), 3.68(3Hz, s), 4.54(1H, q, J=7.5Hz), 4.78(1H, d, J=8Hz), 6.46(1H, d, J=3.5Hz), 6.47(1H, d, J=3.5Hz), 7.07(2H, t, J=8.5Hz), 7.73(2H, dd, J=8.5, 5Hz)

### Example 126: Methyl 4-[5-[4,4,5,5,6,6,7,7,7-Nonafluoro-1-(4-fluorophenylsulfonylamino)heptyl]-2-thienyl]butyrate

Appearance pale brown viscous oil
IR spectrum ν (liq) cm⁻¹ : 3272 , 1738
Mass spectrum m/z : 617 (M⁺)
NMR spectrum δ (CDCl₃) ppm : 1.89(2H, qn, J=7.5Hz), 2.01-2.39(4H, m), 2.31(2H, t, J=7.5Hz), 2.72(2H, t, J=7.5Hz), 3.69(3H, s), 4.56(1H, q, J=7.5Hz), 4.78(1H, d, J=8.5Hz), 6.48(2H, s), 7.09(2H, t, J=8.5Hz), 7.75(2H, dd, J=8.5, 5Hz)

### Example 127: Methyl 4-[5-[1-(4-Chlorophenylsulfonylamino)-6,6,6-trifluoro-5-methylhexyl]-2-thienyl]butyrate

Appearance pale yellow viscous oil
IR spectrum ν (liq) cm⁻¹ : 3284 , 1738
Mass spectrum m/z : 525, 527 (3:1, M⁺)
NMR spectrum δ (CDCl₃) ppm : 1.03(1.5H, d, J=7.5Hz), 1.05(1.5H, d, J=7.5Hz), 1.17-1.53(3H, m), 1.59-1.68(1H, m), 1.71-1.91(4H, m), 2.00-2.11(1H, m), 2.32(2H, t, J=7.5Hz), 2.71(2H, t, J=7.5Hz), 3.69(3H, s), 4.51-4.56(1H, m), 4.75(1H, d, J=8Hz), 6.40-6.50(2H, m), 7.35(2H, d, J=8.5Hz), 7.63(2H, d, J=8.5Hz)

### Example 128: Methyl 4-[5-[6,6,6-Trifluoro-1-(4-fluorophenylsulfonylamino)-5-methylhexyl]-2-thienyl]butyrate

Appearance pale yellow viscous oil
IR spectrum ν (liq) cm⁻¹ : 3280 , 1738
Mass spectrum m/z : 509 (M⁺)
NMR spectrum δ (CDCl₃) ppm : 1.03(1.5H, d, J=7.5Hz), 1.05(1.5H, d, J=7.5Hz), 1.18-1.52(3H, m), 1.59-1.67(1H, m), 1.71-1.91(4H, m), 2.01-2.13(1H, m), 2.31(2H t, J=7.5Hz), 2.71(2H, t, J=7.5Hz), 3.69(3H, s), 4.51-4.56(1H, m), 4.75(1H, d, J=8Hz), 6.42-6.47(2H, m), 7.06(2H, t, J=8.5Hz), 7.72(2H, dd, J=8.5, 5Hz)

### Example 129: Methyl 4-[5-[1-(4-Chlorophenylsulfonylamino)-5,6,6,6-tetrafluoro-5-trifluoromethylhexyl]-2-thienyl]butyrate

Appearance pale yellow viscous oil
IR spectrum ν (liq) cm⁻¹ : 3272 , 1738
Mass spectrum m/z : 597, 599 (3:1, M⁺)
NMR spectrum δ (CDCl₃) ppm : 1.48-1.68(2H, m), 1.78-1.96(4H, m), 2.01-2.98(2H, m), 2.32(2H, t, J=7.5Hz), 2.72(2H, t, J=7.5Hz), 3.69(3H, s), 4.54(1H, q, J=7.5Hz), 4.79(1H, d, J=8Hz), 6.46(1H, d, J=3.5Hz), 6.48(1H, d, J=3.5Hz), 7.36(2H, d, J=8.5Hz), 7.64(2H, d, J=8.5Hz)

### Example 130: Methyl 4-[5-[5,6,6,6-Tetrafluoro-5-trifluoromethyl-1-(4-fluorophenylsulfonylamino)hexyl]-2-thienyl]butyrate

Appearance pale yellow viscous oil
IR spectrum ν (liq) cm⁻¹ : 3280 , 1738
Mass spectrum m/z : 581 (M⁺)
NMR spectrum δ (CDCl₃) ppm : 1.48-1.68(2H, m), 1.78-1.94(4H, m), 2.01-2.08(2H, m), 2.31(2H, t, J=7.5Hz), 2.71(2H, t, J=7.5Hz), 3.69(3H, s), 4.53(1H, q, J=7.5Hz), 4.78(1H, d, J=8Hz), 6.46(1H, d, J=3.5Hz), 6.47(1H, d, J=3.5Hz), 7.07(2H, t, J=9Hz), 7.73(2H, dd, J=9, 5Hz)

### Example 131: Methyl 4-[5-[4,4,5,5,6,7,7,7-Octafluoro-6-trifluoromethyl-1-(4-fluorophenylsulfonylamino)heptyl]-2-thienyl]butyrate

Appearance pale yellow viscous oil
IR spectrum ν (liq) cm⁻¹ : 3272 , 1738
Mass spectrum m/z : 667 (M⁺)
NMR spectrum δ (CDCl₃) ppm : 1.89(2H, qn, J=7.5Hz), 1.99-2.30(4H, m), 2.31(2H, t, J=7.5Hz), 2.72(2H, t, J=7.5Hz), 3.69(3H, s), 4.56(1H, q, J=7.5Hz), 4.76(1H, d, J=8.5Hz), 6.48(2H, s), 7.09(2H, t, J=9Hz), 7.75(2H, dd, J=9, 5Hz)

### Example 132: Methyl 4-[5-[1-(4-Chlorophenylsulfonylamino)-6-fluorohexyl]-2-thienyl]butyrate

Appearance pale yellow viscous oil
IR spectrum ν (liq) cm⁻¹ : 3284 , 1738
Mass spectrum m/z : 475, 477 (3:1, M⁺)
NMR spectrum δ (CDCl₃) ppm : 1.23-1.30(1H, m), 1.34-1.42(3H, m), 1.58-1.69(2H, m), 1.72-1.91(4H, m), 2.32(2H, t, J=7.5Hz), 2.70(2H, t, J=7.5Hz), 3.69(3H, s), 4.40(2H, dt, J=47, 6Hz), 4.54(1H, q, J=7.5Hz), 4.76(1H, d, J=7.5Hz), 6.44(1H, d, J=3.5Hz), 6.47(1H, d, J=3.5Hz), 7.34(2H, d, J=9Hz), 7.62(2H, d, J=9Hz)

### Example 133: Methyl 4-[5-[6-Fluoro-1-(4-fluorophenylsulfonylamino)hexyl]-2-thienyl]butyrate

Appearance pale yellow viscous oil
IR spectrum ν (liq) cm⁻¹ : 3284 , 1738
Mass spectrum m/z : 459 (M⁺)
NMR spectrum δ (CDCl₃) ppm : 1.23-1.31(1H, m), 1.33-1.43(3H, m), 1.58-1.69(2H, m), 1.73-1.91(4H, m), 2.31(2H, t, J=7.5Hz), 2.70(2H, t, J=7.5Hz), 3.69(3H, s), 4.40(2H, dt, J=47, 6Hz), 4.54(1H, q, J=7.5Hz), 4.71(1H, d, J=7.5Hz), 6.44(1H, d, J=3.5Hz), 6.47(1H, d, J=3.5Hz), 7.05(2H, t, J=8.5Hz), 7.71(2H, dd, J=8.5, 5Hz)

### Example 134: Methyl 4-[5-[6-Fluoro-1-(4-nitrophenylsulfonylamino)hexyl]-2-thienyl]butyrate

Appearance pale yellow viscous oil
IR spectrum ν (liq) cm⁻¹ : 3280 , 1736
NMR spectrum δ (CDCl₃) ppm : 1.26-1.34(1H, m), 1.37-1.45(3H, m), 1.60-1.70(2H, m), 1.75-1.91(4H, m), 2.28(2H, t, J=7.5Hz), 2.65(2H, t, J=7.5Hz), 3.69(3H, s), 4.41(2H, dt, J=47.5, 6Hz), 4.63(1H, q, J=7.5Hz), 4.90(1H, d, J=7.5Hz), 6.41(1H, d, J=3.5Hz), 6.50(1H, d, J=3.5Hz), 7.84(2H, t, J=9Hz), 8.18(2H, d, J=9Hz)

### Example 135: Methyl 4-[4-[1-(4-Chlorophenylsulfonylamino)-5,5,6,6,6-pentafluorohexyl]phenyl]butyrate

Appearance pale yellow viscous oil
IR spectrum ν (liq) cm⁻¹ : 3280 , 1738
NMR spectrum δ (CDCl₃) ppm : 1.42-1.51(1H, m), 1.57-1.67(1H, m), 1.73-1.81(1H, m), 1.85-2.02(5H, m), 2.31(2H, t, J=7.5Hz), 2.57(2H, t, J=8Hz), 3.68(3H, s), 4.27(1H, q, J=7.5Hz), 5.08(1H, d, J=8Hz), 6.86(2H, d, J=8Hz), 6.97(2H, d, J=8Hz), 7.24(2H, d, J=8.5Hz), 7.52(2H, d, J=8.5Hz)

### Example 136: Methyl 4-[4-[5,5,6,6,6-Pentafluoro-1-(4-fluorophenylsulfonylamino)hexyl]phenyl]butyrate

Appearance pale yellow viscous oil
IR spectrum ν (liq) cm⁻¹ : 3284 , 1738
NMR spectrum δ (CDCl₃) ppm : 1.43-1.51(1H, m), 1.57-1.66(1H, m), 1.73-1.81(1H, m), 1.88(2H, qn, J=7.5Hz), 1.93-2.17(2H, m), 2.29(2H, t, J=7.5Hz), 2.56(2H, t, J=7.5Hz), 3.68(3H, s), 4.27(1H, q, J=7.5Hz), 5.04(1H, d, J=8Hz), 6.87(2H, d, J=8Hz), 6.96(2H, t, J=8.5Hz), 6.97(2H, d, J=8Hz), 7.61(2H, dd, J=8.5, 5Hz)

### Example 137: Methyl 4-[4-[5,5,6,6,6-Pentafluoro-1-(4-nitrophenylsulfonylamino)hexyl]phenyl]butyrate

Appearance pale yellow plates (i-Pr₂O)
mp 69.5∼70.5°C

| Analysis for C₂₃H₂₅F₅N₂O₆S | | | |
|---|---|---|---|
| Calculated % | C, 59.00; | H, 4.56; | N, 5.07 |
| Found % | C, 50.11; | H, 4.66; | N, 4.90 |

### Example 138: Methyl 4-[4-[5,5,6,6,6-Pentafluoro-1-(4-methoxyphenylsulfonylamino)hexyl]phenyl]butyrate

Appearance pale brown viscous oil
IR spectrum ν (liq) cm⁻¹ : 3280 , 1738
NMR spectrum δ (CDCl₃) ppm : 1.38-1.47(1H, m), 1.55-1.62(1H, m), 1.73-1.80(1H, m), 1.84-2.00(5H, m), 2.30(2H, t, J=7.5Hz), 2.56(2H, t, J=7.5Hz), 3.68(3H, s), 3.82(3H, s), 4.22(1H, q, J=7.5Hz), 4.84(1H, d, J=7.5Hz), 6.79(2H, d, J=9Hz), 6.90(2H, d, J=8Hz), 6.99(2H, d, J=8Hz), 7.58(2H, d, J=9Hz)

### Example 139: Ethyl 4-[4-[5,5,6,6,6-Pentafluoro-1-(4-fluorophenylsulfonylamino)hexyl]phenyl]butyrate

Appearance colorless viscous oil
IR spectrum ν (liq) cm⁻¹ : 3284 , 1734
NMR spectrum δ (CDCl₃) ppm : 1.27(3H, t, J=7.5Hz), 1.43-1.51(1H, m), 1.57-1.67(1H, m), 1.73-1.81(1H, m), 1.84-2.02(5H, m), 2.28(2H, t, J=7.5Hz), 2.56(2H, t, J=7.5Hz), 4.14(2H, q, J=7.5Hz), 4.27(1H, q, J=7.5Hz), 5.06(1H, d, J=7.5Hz), 6.87(2H, d, J=8Hz), 6.96(2H, t, J=8.5Hz), 6.97(2H, d, J=8Hz), 7.61(2H, dd, J=8.5, 5Hz)

### Example 140: Methyl 5-[4-[1-(4-Chlorophenylsulfonylamino)-5,5,6,6,6-pentafluorohexyl]phenyl]valerate

Appearance colorless needles (i-Pr₂O)
mp 79∼81°C

| Analysis for C₂₄H₂₇ClF₅NO₄S | | | |
|---|---|---|---|
| Calculated % | C, 51.85; | H, 4.89; | N, 2.52 |
| Found % | C, 51.67; | H, 4.85; | N, 2.55 |

### Example 141: Methyl 5-[4-[5,5,6,6,6-Pentafluoro-1-(4-fluorophenylsulfonylamino)hexyl]phenyl]valerate

Appearance pale yellow viscous oil
IR spectrum ν (liq) cm⁻¹ : 3284 , 1738
NMR spectrum δ (CDCl₃) ppm : 1.42-1.51(1H, m), 1.54-1.66(5H, m), 1.73-1.81(1H, m), 1.85-2.02(3H, m), 2.34(2H, t, J=7.5Hz), 2.54(2H, t, J=7.5Hz), 3.68(3H, s), 4.26(1H, q, J=7.5Hz), 5.00(1H, d, J=8Hz), 6.85(2H, d, J=8Hz), 6.95(2H, t, J=8.5Hz), 6.96(2H, d, J=8Hz), 7.61(2H, dd, J=8.5, 5Hz)

### Example 142: Methyl 4-[4-[1-(4-Chlorophenylsulfonylamino)-6,6,6-trifluorohexyl]phenyl]butyrate

Appearance colorless viscous oil
IR spectrum ν (liq) cm⁻¹ : 3284 , 1738
NMR spectrum δ (CDCl₃) ppm : 1.18-1.27(1H, m), 1.34-1.42(1H, m), 1.51(2H, qn, J=8Hz), 1.66-1.73(1H, m), 1.78-1.84(1H, m), 1.89(2H, qn, J=7.5Hz), 1.94-2.02(2H, m), 2.31(2H, t, J=7.5Hz), 2.56(2H, t, J=8Hz), 3.68(3H, s), 4.26(1H, q, J=7.5Hz), 4.83(1H, d, J=7.5Hz), 6.85(2H, d, J=8Hz), 6.96(2H, d, J=8Hz), 7.24(2H, d, J=8.5Hz), 7.50(2H, d, J=8.5Hz)

### Example 143: Methyl 4-[4-[6,6,6-Trifluoro-1-(4-fluorophenylsulfonylamino)hexyl]phenyl]butyrate

Appearance colorless viscous oil
IR spectrum ν (liq) cm⁻¹ : 3284 , 1738
NMR spectrum δ (CDCl₃) ppm : 1.18-1.27(1H, m), 1.34-1.43(1H, m), 1.50(2H, qn, J=8Hz), 1.66-1.73(1H, m), 1.78-1.84(1H, m), 1.88(2H, qn, J=7.5Hz), 1.94-2.02(2H, m), 2.29(2H, t, J=7.5Hz), 2.56(2H, t, J=7.5Hz), 3.68(3H, s), 4.26(1H, q, J=7.5Hz), 4.84(1H, d, J=7.5Hz), 6.86(2H, d, J=8Hz), 6.95(2H, t, J=9Hz), 6.96(2H, d, J=8Hz), 7.60(2H, dd, J=9, 5Hz)

### Example 144: Methyl 4-[4-[6,6,6-Trifluoro-1-(4-nitrophenylsulfonylamino)hexyl]phenyl]butyrate

Appearance pale yellow plates (i-Pr₂O)
mp 65.5∼66.5°C

| Analysis for C₂₃H₂₇F₃N₂O₆S | | | |
|---|---|---|---|
| Calculated % | C, 53.48; | H, 5.27: | N, 5.42 |
| Found % | C, 53.47; | H, 5.22; | N, 5.48 |

### Example 145: Methyl 4-[4-[1-(4-Chlorophenylsulfonylamino)-6,6,7,7,7-pentafluoroheptyl]phenyl]butyrate

Appearance pale yellow viscous oil
IR spectrum ν (liq) cm⁻¹ : 3288 , 1738
NMR spectrum δ (CDCl₃) ppm : 1.17-1.28(1H, m), 1.38-1.45(1H, m), 1.50-1.57(2H, m), 1.67-1.75(1H, m), 1.80-1.98(5H, m), 2.31(2H, t, J=7.5Hz), 2.56(2H, t, J=7.5Hz), 3.68(3H, s), 4.27(1H, q, J=7.5Hz), 4.98(1H, d, J=8Hz), 6.86(2H, d, J=8Hz), 6.96(2H, d, J=8Hz), 7.24(2H, d, J=8.5Hz), 7.51(2H, d, J=8.5Hz)

### Example 146: Methyl 4-[4-[6,6,7,7,7-Pentafluoro-1-(4-fluorophenylsulfonylamino)heptyl]phenyl]butyrate

Appearance pale yellow viscous oil
IR spectrum ν (liq) cm⁻¹ : 3288 , 1740
NMR spectrum δ (CDCl₃) ppm : 1.18-1.27(1H, m), 1.34-1.44(1H, m), 1.48-1.57(2H, m), 1.67-1.75(1H, m), 1.80-1.98(5H, m), 2.30(2H, t, J=7.5Hz), 2.56(2H, t, J=7.5Hz), 3.68(3H, s), 4.27(1H, q, J=7.5Hz), 4.82-4.90(1H, m), 6.86(2H, d, J=7.5Hz), 6.95(2H, t, J=8.5Hz), 6.96(2H, d, J=7.5Hz), 7.60(2H, dd, J=8.5, 5Hz)

### Example 147: Methyl 4-[4-[1-(4-Chlorophenylsulfonylamino)-4,4,5,5,6,6,6-heptafluorohexyl]phenyl]butyrate

Appearance colorless plates (80% aq MeOH)
mp 100∼102°C

| Analysis for C₂₃H₂₃ClF₇NO₄S | | | |
|---|---|---|---|
| Calculated % | C, 47.80; | H, 4.01; | N, 2.42 |
| Found % | C, 47.66; | H, 3.84; | N, 2.49 |

### Example 148: Methyl 4-[4-[4,4,5,5,6,6,6-Heptafluoro-1-(4-fluorophenylsulfonylamino)hexyl]phenyl]butyrate

Appearance colorless plates (i-Pr₂O-n-Hexane)
mp 59.5∼61°C

| Analysis for C₂₃H₂₃F₈NO₄S | | | |
|---|---|---|---|
| Calculated % | C, 49.20; | H, 4.13; | N, 2.49 |
| Found % | C, 49.16; | H, 3.95; | N, 2.57 |

### Example 149: Methyl 4-[4-[1-(4-Chlorophenylsulfonylamino)-5,5,6,6,7,7,7-heptafluoroheptyl]phenyl]butyrate

Appearance colorless viscous oil
IR spectrum ν (liq) cm⁻¹ : 3284 , 1738
NMR spectrum δ (CDCl₃) ppm : 1.43-1.52(1H, m), 1.59-1.68(1H, m), 1.74-1.82(1H, m), 1.86-1.92(3H, m), 1.95-2.07(2H, m), 2.31(2H, t, J=8Hz), 2.57(2H, t, J=8Hz), 3.68(3H, s), 4.28(1H, q, J=7.5Hz), 4.89(1H, d, J=8Hz), 6.86(2H, d, J=8Hz), 6.97(2H, d, J=8Hz), 7.25(2H, d, J=8.5Hz), 7.52(2H, d, J=8.5Hz)

### Example 150: Methyl 4-[4-[5,5,6,6,7,7,7-Heptafluoro-1-(4-fluorophenylsulfonylamino)heptyl]phenyl]butyrate

Appearance colorless viscous oil
IR spectrum ν (liq) cm⁻¹ : 3288 , 1738
NMR spectrum δ (CDCl₃) ppm : 1.43-1.52(1H, m), 1.54-1.68(1H, m), 1.74-1.82(1H, m), 1.85-1.91(3H, m), 1.93-2.06(2H, m), 2.29(2H, t, J=7.5Hz), 2.56(2H, t, J=7.5Hz), 3.68(3H, s), 4.28(1H, q, J=7.5Hz), 4.96(1H, d, J=8Hz), 6.87(2H, d, J=8Hz), 6.96(2H, t, J=9Hz), 6.97(2H, d, J=8Hz), 7.61(2H, dd, J=9, 5Hz)

### Example 151: Methyl 4-[4-[1-(4-Chlorophenylsulfonylamino)-4,4,5,5,6,6,7,7,7-nonafluoroheptyl]phenyl]butyrate

Appearance pale yellow viscous oil
IR spectrum ν (liq) cm⁻¹ : 3276 , 1738
NMR spectrum δ (CDCl₃) ppm : 1.90(2H, qn, J=7.5Hz), 1.93-2.26(4H, m), 2.31(2H, t, J=7.5Hz), 2.58(2H, t, J=7.5Hz), 3.68(3H, s), 4.30(1H, q, J=7.5Hz), 4.90(1H, d, J=8.5Hz), 6.86(2H, d, J=8Hz), 6.99(2H, d, J=8Hz), 7.26(2H, d, J=8.5Hz), 7.53(2H, d, J=8.5Hz)

### Example 152: Methyl 4-[4-[4,4,5,5,6,6,7,7,7-Nonafluoro-1-(4-fluorophenylsulfonylamino)heptyl]phenyl]butyrate

Appearance pale yellow viscous oil
IR spectrum ν (liq) cm⁻¹ : 3280 , 1738
NMR spectrum δ (CDCl₃) ppm : 1.88(2H, qn, J=7.5Hz), 1.94-2.02(2H, m), 2.04-2.25(2H, m), 2.29(2H, t, J=7.5Hz), 2.56(2H, t, J=7.5Hz), 3.68(3H, s), 4.30(1H, q, J=7.5Hz), 5.16(1H, d, J=8.5Hz), 6.87(2H, d, J=8Hz), 6.96(2H, t, J=9Hz), 6.99(2H, d, J=8Hz), 7.63(2H, dd, J=9, 5Hz)

### Example 153: Methyl 4-[4-[1-(4-Chlorophenylsulfonylamino)-6,6,6-trifluoro-5-methylhexyl]phenyl]butyrate

Appearance colorless viscous oil
IR spectrum ν (liq) cm⁻¹ : 3288 , 1738
NMR spectrum δ (CDCl₃) ppm : 1.01(1.5H, d, J=7.5Hz), 1.02(1.5H, d, J=7.5Hz), 1.08-1.18(0.5H, m), 1.22-1.34(2H, m), 1.37-1.46(0.5H, m), 1.56-1.73(2H, m), 1.75-1.85(1H, m), 1.89(2H, qn, J=7.5Hz), 1.98-2.08(1H, m), 2.31(2H, t, J=7.5Hz), 2.56(2H, t, J=7.5Hz), 3.68(3H, s), 4.24-4.30(1H, m), 4.87(1H, d, J=8Hz), 6.85(1H, d, J=8Hz), 6.86(1H, d, J=8Hz), 6.95(1H, d, J=8Hz), 6.96(1H, d, J=8Hz), , 7.23(1H, d, J=8.5Hz), 7.24(1H, d, J=8.5Hz), 7.50(1H, d, J=8.5Hz), 7.51(1H, d, J=8.5Hz)

### Example 154: Methyl 4-[4-[6,6,6-Trifluoro-1-(4-fluorophenylsulfonylamino)-5-methylhexyl]phenyl]butyrate

Appearance colorless viscous oil
IR spectrum ν (liq) cm⁻¹ : 3288 , 1740
NMR spectrum δ (CDCl₃) ppm : 1.01(1.5H, d, J=7.5Hz), 1.02(1.5H, d, J=7.5Hz), 1.09-1.19(0.5H, m), 1.21-1.35(2H, m), 1.38-1.48(0.5H, m), 1.56-1.85(3H, m), 1.88(2H, qn, J=7.5Hz), 1.98-2.10(1H, m), 2.29(2H, t, J=7.5Hz), 2.56(2H, t, J=7.5Hz), 3.68(3H, s), 4.26-4.30(1H, m), 4.83(1H, d, J=8Hz), 6.86(1H, d, J=8Hz), 6.87(1H, d, J=8Hz), 6.91-7.00(4H, m), 7.55-7.64(2H, m)

### Example 155: Methyl 4-[4-[1-(4-Chlorophenylsulfonylamino)-5,6,6,6-tetrafluoro-5-trifluoromethylhexyl]phenyl]butyrate

Appearance colorless viscous oil
IR spectrum ν (liq) cm⁻¹ : 3284 , 1738
NMR spectrum δ (CDCl₃) ppm : 1.40-1.50(1H, m), 1.55-1.64(1H, m), 1.71-1.78(1H, m), 1.82-1.92(3H, m), 1.97-2.04(2H, m), 2.31(2H, t, J=7.5Hz), 2.57(2H, t, J=7.5Hz), 3.68(3H, s), 4.27(1H, q, J=7.5Hz), 4.98(1H, d, J=8Hz), 6.85(2H, d, J=8Hz), 6.97(2H, d, J=8Hz), 7.24(2H, d, J=9Hz), 7.52(2H, d, J=9Hz)

### Example 156: Methyl 4-[4-[5,6,6,6-Tetrafluoro-5-trifluoromethyl-1-(4-fluorophenylsulfonylamino)hexyl]phenyl]butyrate

Appearance colorless viscous oil
IR spectrum ν (liq) cm⁻¹ : 3288 , 1738
NMR spectrum δ (CDCl₃) ppm : 1.40-1.49(1H, m), 1.54-1.63(1H, m), 1.71-1.78(1H, m), 1.82-1.91(3H, m), 1.97-2.04(2H, m), 2.29(2H, t, J=7.5Hz), 2.56(2H, t, J=7.5Hz), 3.68(3H, s), 4.27(1H, q, J=7.5Hz), 4.94(1H, d, J=8Hz), 6.86(2H, d, J=8Hz), 6.96(2H, t, J=8.5Hz), 6.97(2H, d, J=8Hz), 7.61(2H, dd, J=8.5, 5Hz)

### Example 157: Methyl 4-[4-[1-(4-Chlorophenylsulfonylamino)-4,4,5,5,6,7,7,7-octafluoro-6-trifluoromethylheptyl]phenyl]butyrate

Appearance colorless viscous oil
IR spectrum ν (liq) cm⁻¹ : 3276 , 1738
NMR spectrum δ (CDCl₃) ppm : 1.89(2H, qn, J=7.5Hz), 1.94-2.28(4H, m), 2.31(2H, t, J=7.5Hz), 2.57(2H, t, J=7.5Hz), 3.68(3H, s), 4.29(1H, q, J=7.5Hz), 5.01(1H, d, J=8.5Hz), 6.86(2H, d, J=8Hz), 6.99(2H, d, J=8Hz), 7.26(2H, d, J=8.5Hz), 7.53(2H, d, J=8.5Hz)

### Example 158: Methyl 4-[4-[4,4,5,5,6,7,7,7-Octafluoro-6-trifluoromethyl-1-(4-fluorophenylsulfonylamino)heptyl]phenyl]butyrate

Appearance colorless viscous oil
IR spectrum ν (liq) cm⁻¹ : 3276, 1738
NMR spectrum δ (CDCl₃) ppm : 1.88(2H, qn, J=7.5Hz), 1.95-2.12(3H, m), 2.15-2.27(1H, m), 2.29(2H, t, J=7.5Hz), 2.57(2H, t, J=7.5Hz), 3.68(3H, s), 4.29(1H, q, J=7.5Hz), 4.98(1H, d, J=8.5Hz), 6.87(2H, d, J=8Hz), 6.97(2H, t, J=8.5Hz), 6.99(2H, d, J=8Hz), 7.63(2H, dd, J=8.5, 5Hz)

### Example 159: Methyl 4-[4-[1-(4-Chlorophenylsulfonylamino)-6-fluorohexyl]phenyl]butyrate

Appearance colorless needles (80% aq MeOH)
mp 73.5∼75°C

| Analysis for C₂₃H₂₉ClFNO₄S | | | |
|---|---|---|---|
| Calculated % | C, 58.78; | H, 6.22; | N, 2.98 |
| Found % | C, 58.60; | H, 6.17; | N, 3.00 |

### Example 160: Methyl 4-[4-[6-Fluoro-1-(4-fluorophenylsulfonylamino)hexyl]phenyl]butyrate

Appearance pale yellow viscous oil
IR spectrum ν (liq) cm⁻¹ : 3292 , 1738
NMR spectrum δ (CDCl₃) ppm : 1.14-1.22(1H, m), 1.29-1.39(3H, m), 1.56-1.73(3H, m), 1.76-1.83(1H, m), 1.88(2H, qn, J=7.5Hz), 2.29(2H, t, J=7.5Hz), 2.55(2H, t, J=7.5Hz), 3.68(3H, s), 4.28(1H, q, J=7.5Hz), 4.37(2H, dt, J=47, 6Hz), 4.88(1H, d, J=7.5Hz), 6.87(2H, d, J=8.5Hz), 6.94(2H, t, J=9Hz), 6.95(2H, d, J=8.5Hz), 7.59(2H, dd, J=9, 5Hz)

### Example 161: Methyl 4-[4-[6-Fluoro-1-(4-nitrophenylsulfonylamino)hexyl]phenyl]butyrate

Appearance pale yellow viscous oil
IR spectrum ν (liq) cm⁻¹ : 3288 , 1738
NMR spectrum δ (CDCl₃) ppm : 1.05-1.26(1H, m), 1.32-1.42(3H, m), 1.58-1.74(3H, m), 1.80-1.86(3H, m), 2.28(2H, t, J=7.5Hz), 2.52(2H, t, J=7.5Hz), 3.69(3H, s), 4.36(1H, q, J=7.5Hz), 4.38(2H, dt, J=47, 6Hz), 6.86(2H, d, J=8.5Hz), 6.91(2H, d, J=8.5Hz),7.70(2H, d, J=8.5Hz), 8.06(2H, d, J=8.5Hz)

### Example 162: 4-[5-[1-(4-Chlorophenylsulfonylamino)-5,5,6,6,6-pentafluorohexyl]-2-thienyl]butyric Acid

To a solution of 2.40 g of methyl 4-[5-[1-(4-chlorophenylsulfonylamino)-5,5,6,6,6-pentafluorohexyl]-2-thienyl]butyrate in 8 ml of tetrahydrofuran, 4.38 ml of 2N aqueous sodium hydroxide solution was added and stirring was continued at room temperature for 1.5 hours. After the reaction solvent was removed under reduced pressure, water was added to the residue. The mixture was acidified with dilute hydrochloric acid, and then extracted with methylene chloride. The methylene chloride layer was washed with water, dried, and then the solvent was removed. The resulting residue was crystallized in diisopropyl ether and the crystals were collected by filtration to obtain 1.63 g of colorless crystals. Recrystallization from a mixture of diisopropyl ether and ethyl acetate gave colorless needles, mp 114.5∼115°C.

| Analysis for C₂₀H₂₁ClF₅NO₄S₂ | | | |
|---|---|---|---|
| Calculated % | C, 44.99; | H, 3.96; | N, 2.62 |
| Found % | C, 45.00; | H, 3.90; | N, 2.63 |

The compounds of Example 163 through 213 were obtained in the same manner as described in Example 162.

### Example 163: 4-[5-[5,5,6,6,6-Pentafluoro-1-(4-fluorophenylsulfonylamino)hexyl]-2-thienyl]butyric Acid

Appearance colorless plates (i-Pr₂O-n-Hexane)
mp 84.5∼86°C

| Analysis for C₂₀H₂₁F₆NO₄S₂ | | | |
|---|---|---|---|
| Calculated % | C, 46.42; | H, 4.09; | N, 2.71 |
| Found % | C, 46.27; | H, 3.94; | N, 2.75 |

### Example 164: 4-[5-[5,5,6,6,6-Pentafluoro-1-(4-nitrophenylsulfonylamino)hexyl]-2-thienyl]butyric Acid

Appearance pale yellow needles (80% aq MeOH)
mp 122.5∼123°C

| Analysis for C₂₀H₂₁F₅N₂O₆S₂ | | | |
|---|---|---|---|
| Calculated % | C, 44.12; | H, 3.89; | N, 5.14 |
| Found % | C, 44.02; | H, 3.75; | N, 5.25 |

### Example 165: 5-[5-[1-(4-Chlorophenylsulfonylamino)-5,5,6,6,6-pentafluorohexyl]-2-thienyl]valeric Acid

Appearance colorless needles (i-Pr₂O)
mp 109∼111°C

| Analysis for C₂₁H₂₃ClF₅NO₄S₂ | | | |
|---|---|---|---|
| Calculated % | C, 46.03; | H, 4.23; | N, 2.56 |
| Found % | C, 45.89; | H, 4.16; | N, 2.64 |

### Example 166: 5-[5-[5,5,6,6,6-Pentafluoro-1-(4-fluorophenylsulfonylamino)hexyl]-2-thienyl]valeric Acid

Appearance colorless needles (i-Pr₂O)
mp 95∼96.5°C

| Analysis for C₂₁H₂₃F₆NO₄S₂ | | | |
|---|---|---|---|
| Calculated % | C, 47.45; | H, 4.36; | N, 2.64 |
| Found % | C, 47.25; | H, 4.28; | N, 2.78 |

### Example 167: 4-[5-[1-(4-Chlorophenylsulfonylamino)-5,5,5-trifluoropentyl]-2-thienyl]butyric Acid

Appearance colorless needles (i-Pr₂O-AcOEt)
mp 122.5∼123°C

| Analysis for C₁₉H₂₁ClF₃NO₄S₂ | | | |
|---|---|---|---|
| Calculated % | C, 47.15; | H, 4.37; | N, 2.89 |
| Found % | C, 47.06; | H, 4.25; | N, 2.93 |

### Example 168: 4-[5-[1-(4-Chlorophenylsulfonylamino)-6,6,6-trifluorohexyl]-2-thienyl]butyric Acid

Appearance colorless needles (80% aq MeOH)
mp 115.5∼117°C

| Analysis for C₂₀H₂₃ClF₃NO₄S₂ | | | |
|---|---|---|---|
| Calculated % | C, 48.24; | H, 4.66; | N, 2.81 |
| Found % | C, 48.13; | H, 4.65; | N, 2.82 |

### Example 169: 4-[5-[6,6,6-Trifluoro-1-(4-fluorophenylsulfonylamino)hexyl]-2-thienyl]butyric Acid

Appearance colorless plates (80% aq MeOH)
mp 135∼136°C

| Analysis for C₂₀H₂₃F₄NO₄S₂ | | | |
|---|---|---|---|
| Calculated % | C, 49.89; | H, 4.81; | N, 2.91 |
| Found % | C, 49.82; | H, 4.73; | N, 2.97 |

### Example 170: 4-[5-[6,6,6-Trifluoro-1-(4-nitrophenylsulfonylamino)hexyl]-2-thienyl]butyric Acid

Appearance pale yellow needles (80% aq MeOH)
mp 116∼116.5°C

| Analysis for C₂₀H₂₃F₃N₂O₆S₂ | | | |
|---|---|---|---|
| Calculated % | C, 47.24; | H, 4.56; | N, 5.51 |
| Found % | C, 47.12; | H, 4.53; | N, 5.59 |

### Example 171: 4-[5-[1-(4-Chlorophenylsulfonylamino)-7,7,7-trifluoroheptyl]-2-thienyl]butyric Acid

Appearance colorless plates (i-Pr₂O)
mp 86∼87°C

| Analysis for C₂₁H₂₅ClF₃NO₄S₂ | | | |
|---|---|---|---|
| Calculated % | C, 49.26; | H, 4.92; | N, 2.74 |
| Found % | C, 49.26; | H, 4.91; | N, 2.78 |

### Example 172: 4-[5-[7,7,7-Trifluoro-1-(4-fluorophenylsulfonylamino)heptyl]-2-thienyl]butyric Acid

Appearance colorless needles (i-Pr₂O)
mp 96∼96.5°C

| Analysis for C₂₁H₂₅F₄NO₄S₂ | | | |
|---|---|---|---|
| Calculated % | C, 50.90; | H, 5.08; | N, 2.83 |
| Found % | C, 50.96; | H, 5.08; | N, 2.88 |

### Example 173: 4-[5-[1-(4-Chlorophenylsulfonylamino)-6,6,7,7,7-pentafluoroheptyl]-2-thienyl]butyric Acid

Appearance colorless plates (80% aq MeOH)
mp 111∼113°C

| Analysis for C₂₁H₂₃ClF₅NO₄S₂ | | | |
|---|---|---|---|
| Calculated % | C, 46.03; | H, 4.23; | N, 2.56 |
| Found % | C, 46.10; | H, 4.22; | N, 2.70 |

### Example 174: 4-[5-[6,6,7,7,7-Pentafluoro-1-(4-fluorophenylsulfonylamino)heptyl]-2-thienyl]butyric Acid

Appearance colorless plates (80% aq MeOH)
mp 103.5∼105°C

| Analysis for C₂₁H₂₃F₆NO₄S₂ | | | |
|---|---|---|---|
| Calculated % | C, 47.45; | H, 4.36; | N, 2.64 |
| Found % | C, 47.46; | H, 4.24; | N, 2.81 |

### Example 175: 4-[5-[1-(4-Chlorophenylsulfonylamino)-4,4,5,5,6,6,6-heptafluorohexyl]-2-thienyl]butyric Acid

Appearance colorless plates (80% aq MeOH)
mp 139.5∼141.5°C

| Analysis for C₂₀H₁₉ClF₇NO₄S₂ | | | |
|---|---|---|---|
| Calculated % | C, 42.15; | H, 3.36; | N, 2.46 |
| Found % | C, 42.12; | H, 3.29; | N, 2.50 |

### Example 176: 4-[5-[4,4,5,5,6,6,6-Heptafluoro-1-(4-fluorophenylsulfonylamino)hexyl]-2-thienyl]butyric Acid

Appearance colorless needles (i-Pr₂O-n-Hexane)
mp 86.5∼87.5°C

| Analysis for C₂₀H₁₉F₈NO₄S₂ | | | |
|---|---|---|---|
| Calculated % | C, 43.40; | H, 3.46; | N, 2.53 |
| Found % | C, 43.41; | H, 3.31; | N, 2.60 |

### Example 177: 4-[5-[1-(4-Chlorophenylsulfonylamino)-5,5,6,6,7,7,7-heptafluoroheptyl]-2-thienyl]butyric Acid

Appearance colorless plates (80% aq MeOH)
mp 125∼126°C

| Analysis for C₂₁H₂₁ClF₇NO₄S₂ | | | |
|---|---|---|---|
| Calculated % | C, 43.19; | H, 3.62; | N, 2.40 |
| Found % | C, 43.20; | H, 3.61; | N, 2.43 |

### Example 178: 4-[5-[5,5,6,6,7,7,7-Heptafluoro-1-(4-fluorophenylsulfonylamino)heptyl]-2-thienyl]butyric Acid

Appearance colorless needles (80% aq MeOH)
mp 100∼101°C

| Analysis for C₂₁H₂₁F₈NO₄S₂ | | | |
|---|---|---|---|
| Calculated % | C, 44.44; | H, 3.73; | N, 2.47 |
| Found % | C, 44.54; | H, 3.77; | N, 2.53 |

### Example 179: 4-[5-[4,4,5,5,6,6,7,7,7-Nonafluoro-1-(4-fluorophenylsulfonylamino)heptyl]-2-thienyl]butyric Acid

Appearance colorless plates (i-Pr₂O-n-Hexane)
mp 108∼110°C

| Analysis for C₂₁H₁₉F₁₀NO₄S₂ | | | |
|---|---|---|---|
| Calculated % | C, 41.79; | H, 3.17; | N, 2.32 |
| Found % | C, 41.84; | H, 3.13; | N, 2.43 |

### Example 180: 4-[5-[1-(4-Chlorophenylsulfonylamino)-6,6,6-trifluoro-5-methylhexyl]-2-thienyl]butyric Acid

Appearance colorless needles (80% aq MeOH)
mp 107.5∼108.5°C

| Analysis for C₂₁H₂₅ClF₃NO₄S₂ | | | |
|---|---|---|---|
| Calculated % | C, 49.26; | H, 4.92; | N, 2.74 |
| Found % | C, 49.26; | H, 4.83; | N, 2.83 |

### Example 181: 4-[5-[6,6,6-Trifluoro-1-(4-fluorophenylsulfonylamino)-5-methylhexyl]-2-thienyl]butyric Acid

Appearance colorless needles (80% aq MeOH)
mp 125.5∼126.5°C

| Analysis for C₂₁H₂₅F₄NO₄S₂ | | | |
|---|---|---|---|
| Calculated % | C, 50.90; | H, 5.08; | N, 2.83 |
| Found % | C, 50.82; | H, 4.93; | N, 2.83 |

### Example 182: 4-[5-[1-(4-Chlorophenylsulfonylamino)-5,6,6,6-tetrafluoro-5-trifluoromethylhexyl]-2-thienyl]butyric Acid

Appearance colorless plates (70% aq MeOH)
mp 122.5∼124°C

| Analysis for C₂₁H₂₁ClF₇NO₄S₂ | | | |
|---|---|---|---|
| Calculated % | C, 43.19; | H, 3.62; | N, 2.40 |
| Found % | C, 43.18; | H, 3.64; | N, 2.37 |

### Example 183: 4-[5-[5,6,6.6-Tetrafluoro-5-trifluoromethyl-1-(4-fluorophenylsulfonylamino)hexyl]-2-thienyl]butyric Acid

Appearance colorless plates (i-Pr₂O)
mp 89∼90.5°C

| Analysis for C₂₁H₂₁F₈NO₄S₂ | | | |
|---|---|---|---|
| Calculated % | C, 44.44; | H, 3.73; | N, 2.47 |
| Found % | C, 44.49; | H, 3.75; | N, 2.46 |

### Example 184: 4-[5-[4,4,5,5,6,7,7,7-Octafluoro-6-trifluoromethyl-1-(4-fluorophenylsulfonylamino)heptyl]-2-thienyl]butyric Acid

Appearance pale yellow viscous oil
IR spectrum ν (liq) cm⁻¹ : 3272 , 1712
NMR spectrum δ (CDCl₃) ppm : 1.90(2H, qn, J=7.5Hz), 1.98-2.30(4H, m), 2.36(2H, t, J=7.5Hz), 2.75(2H, t, J=7.5Hz), 4.56(1H, q, J=7.5Hz), 5.00(1H, d, J=8Hz), 6.49(2H, s), 7.09(2H t, J=8.5Hz), 7.75(2H, dd, J=8.5, 5Hz)

### Example 185: 4-[5-[1-(4-Chlorophenylsulfonylamino)-6-fluorohexyl]-2-thienyl]butyric Acid

Appearance colorless needles (i-Pr₂O-AcOEt)
mp 85.5∼87°C

| Analysis for C₂₀H₂₅ClFNO₄S₂ | | | |
|---|---|---|---|
| Calculated % | C, 51.99; | H, 5.45; | N, 3.03 |
| Found % | C, 51.93; | H, 5.32; | N, 3.10 |

### Example 186: 4-[5-[6-Fluoro-1-(4-fluorophenylsulfonylamino)hexyl]-2-thienyl]butyric Acid

Appearance colorless needles (80% aq MeOH)
mp 99∼100.5°C

| Analysis for C₂₀H₂₅F₂NO₄S₂ | | | |
|---|---|---|---|
| Calculated % | C, 53.91; | H, 5.66; | N, 3.14 |
| Found % | C, 53.78; | H, 5.65; | N, 3.18 |

### Example 187: 4-[5-[6-Fluoro-1-(4-nitrophenylsulfonylamino)hexyl]-2-thienyl]butyric Acid

Appearance pale yellow plates (i-Pr₂O-AcOEt)
mp 91∼93°C

| Analysis for C₂₀H₂₅FN₂O₆S₂ | | | |
|---|---|---|---|
| Calculated % | C, 50.83; | H, 5.33; | N, 5.93 |
| Found % | C, 50.65; | H, 5.26; | N, 6.00 |

### Example 188: 4-[4-[1-(4-Chlorophenylsulfonylamino)-5,5,6,6,6-pentafluorohexyl]phenyl]butyric Acid

Appearance colorless plates (i-Pr₂O-AcOEt)
mp 125∼126°C

| Analysis for C₂₂H₂₃ClF₅NO₄S | | | |
|---|---|---|---|
| Calculated % | C, 50.05; | H, 4.39; | N, 2.65 |
| Found % | C, 50.11; | H, 4.14; | N, 2.48 |

### Example 189: 4-[4-[5,5,6,6,6-Pentafluoro-1-(4-fluorophenylsulfonylamino)hexyl]phenyl]butyric Acid

Appearance colorless needles (80% aq MeOH)
mp 142∼143°C

| Analysis for C₂₂H₂₃F₆NO₄S | | | |
|---|---|---|---|
| Calculated % | C, 51.66; | H, 4.53; | N, 2.74 |
| Found % | C, 51.68; | H, 4.31; | N, 2.69 |

### Example 190: 4-[4-[5,5,6,6,6-Pentafluoro-1-(4-nitrophenylsulfonylamino)hexyl]phenyl]butyric Acid

Appearance colorless needles (i-Pr₂O-AcOEt)
mp 112∼113°C

| Analysis for C₂₂H₂₃F₅N₂O₆S | | | |
|---|---|---|---|
| Calculated % | C, 49.07; | H, 4.31; | N, 5.20 |
| Found % | C, 48.95; | H, 4.04; | N, 5.25 |

### Example 191: 4-[4-[5,5,6,6,6-Pentafluoro-1-(4-methoxyphenylsulfonylamino)hexyl]phenyl]butyric Acid

Appearance pale brown plates (70% aq MeOH)
mp 114.5∼116°C

| Analysis for C₂₃H₂₆F₅NO₅S | | | |
|---|---|---|---|
| Calculated % | C, 52.77; | H, 5.01; | N, 2.68 |
| Found % | C, 52.71; | H, 4.99; | N, 2.72 |

### Example 192: 5-[4-[1-(4-Chlorophenylsulfonylamino)-5,5,6,6,6-pentafluorohexyl]phenyl]valeric Acid

Appearance colorless needles (i-Pr₂O)
mp 105∼108°C

| Analysis for C₂₃H₂₅ClF₅NO₄S | | | |
|---|---|---|---|
| Calculated % | C, 50.97; | H, 4.65: | N, 2.58 |
| Found % | C, 50.78; | H, 4.71; | N, 2.61 |

### Example 193: 5-[4-[5,5,6,6,6-Pentafluoro-1-(4-fluorophenylsulfonylamino)hexyl]phenyl]valeric Acid

Appearance colorless needles (i-Pr₂O)
mp 104∼105.5°C

| Analysis for C₂₃H₂₅F₆NO₄S | | | |
|---|---|---|---|
| Calculated % | C, 52.57; | H, 4.80; | N, 2.67 |
| Found % | C, 52.43; | H, 4.81; | N, 2.81 |

### Example 194: 4-[4-[1-(4-Chlorophenylsulfonylamino)-6,6,6-trifluorohexyl]phenyl]butyric Acid

Appearance colorless needles (90% aq MeOH)
mp 155∼156°C

| Analysis for C₂₂H₂₅ClF₃NO₄S | | | |
|---|---|---|---|
| Calculated % | C, 53.71; | H, 5.12; | N, 2.85 |
| Found % | C, 53.69; | H, 5.07; | N, 3.00 |

### Example 195: 4-[4-[6,6,6-Trifluoro-1-(4-fluorophenylsulfonylamino)hexyl]phenyl]butyric Acid

Appearance colorless needles (90% aq MeOH)
mp 161∼163°C

| Analysis for C₂₂H₂₅F₄NO₄S | | | |
|---|---|---|---|
| Calculated % | C, 55.57; | H, 5.30; | N, 2.95 |
| Found % | C, 55.41; | H, 5.33; | N, 3.02 |

### Example 196: 4-[4-[6,6,6-Trifluoro-1-(4-nitrophenylsulfonylamino)hexyl]phenyl]butyric Acid

Appearance pale yellow needles (i-Pr₂O-AcOEt)
mp 122.5∼123°C

| Analysis for C₂₂H₂₅F₃N₂O₆S | | | |
|---|---|---|---|
| Calculated % | C, 52.58; | H, 5.01; | N, 5.57 |
| Found % | C, 52.47; | H, 5.02; | N, 5.68 |

### Example 197: 4-[4-[1-(4-Chlorophenylsulfonylamino)-6,6,7,7,7-pentafluoroheptyl]phenyl]butyric Acid

Appearance colorless plates (80% aq MeOH)
mp 127∼129°C

| Analysis for C₂₃H₂₅ClF₅NO₄S | | | |
|---|---|---|---|
| Calculated % | C, 50.97; | H, 4.65; | N, 2.58 |
| Found % | C, 50.91; | H, 4.49; | N, 2.69 |

### Example 198: 4-[4-[6,6,7,7,7-Pentafluoro-1-(4-fluorophenylsulfonylamino)heptyl]phenyl]butyric Acid

Appearance colorless plates (80% aq MeOH)
mp 146∼147.5°C

| Analysis for C₂₃H₂₅F₆NO₄S | | | |
|---|---|---|---|
| Calculated % | C, 52.57; | H, 4.80; | N, 2.67 |
| Found % | C, 52.55; | H, 4.63; | N, 2.83 |

### Example 199: 4-[4-[1-(4-Chlorophenylsulfonylamino)-4,4,5,5,6,6,6-heptafluorohexyl]phenyl]butyric Acid

Appearance colorless needles (i-Pr₂O)
mp 104∼105.5°C

| Analysis for C₂₂H₂₁ClF₇NO₄S | | | |
|---|---|---|---|
| Calculated % | C, 46.86; | H, 3.75; | N, 2.48 |
| Found % | C, 46.65; | H, 3.68; | N, 2.62 |

### Example 200: 4-[4-[4,4,5,5,6,6,6-Heptafluoro-1-(4-fluorophenylsulfonylamino)hexyl]phenyl]butyric Acid

Appearance colorless plates (i-Pr₂O)
mp 107∼109°C

| Analysis for C₂₂H₂₁F₈NO₄S | | | |
|---|---|---|---|
| Calculated % | C, 48.27; | H, 3.87; | N, 2.56 |
| Found % | C, 47.97; | H, 3.80; | N, 2.63 |

### Example 201: 4-[4-[1-(4-Chlorophenylsulfonylamino)-5,5,6,6,7,7,7-heptafluoroheptyl]phenyl]butyric Acid

Appearance colorless plates (80% aq MeOH)
mp 117.5∼119°C

| Analysis for C₂₃H₂₃ClF₇NO₄S | | | |
|---|---|---|---|
| Calculated % | C, 47.80; | H, 4.01; | N, 2.42 |
| Found % | C, 47.78; | H, 3.91; | N, 2.47 |

### Example 202: 4-[4-[5,5,6,6,7,7,7-Heptafluoro-1-(4-fluorophenylsulfonylamino)heptyl]phenyl]butyric Acid

Appearance colorless plates (80% aq MeOH)
mp 108∼109.5°C

| Analysis for C₂₃H₂₃F₈NO₄S | | | |
|---|---|---|---|
| Calculated % | C, 49.20; | H, 4.13; | N, 2.49 |
| Found % | C, 49.13; | H, 3.99; | N, 2.48 |

### Example 203: 4-[4-[1-(4-Chlorophenylsulfonylamino)-4,4,5,5,6,6,7,7,7-nonafluoroheptyl]phenyl]butyric Acid

Appearance colorless needles (80% aq MeOH)
mp 108∼110°C

| Analysis for C₂₃H₂₁ClF₉NO₄S | | | |
|---|---|---|---|
| Calculated % | C, 45.00; | H, 3.45; | N, 2.28 |
| Found % | C, 44.87; | H, 3.36; | N, 2.34 |

### Example 204: 4-[4-[4,4,5,5,6,6,7,7,7-Nonafluoro-1-(4-fluorophenylsulfonylamino)heptyl]phenyl]butyric Acid

Appearance colorless plates (80% aq MeOH)
mp 90.5∼92.5°C

| Analysis for C₂₃H₂₁F₁₀NO₄S | | | |
|---|---|---|---|
| Calculated % | C, 46.24; | H, 3.54; | N, 2.34 |
| Found % | C, 46.12; | H, 3.34; | N, 2.40. |

### Example 205: 4-[4-[1-(4-Chlorophenylsulfonylamino)-6,6,6-trifluoro-5-methylhexyl]phenyl]butyric Acid

Appearance colorless crystals (80% aq MeOH)
mp 133∼134°C

| Analysis for C₂₃H₂₇ClF₃NO₄S | | | |
|---|---|---|---|
| Calculated % | C, 54.60; | H, 5.38; | N, 2.77 |
| Found % | C, 54.42; | H, 5.22; | N, 2.78 |

### Example 206: 4-[4-[6,6,6-Trifluoro-1-(4-fluorophenylsulfonylamino)-5-methylhexyl]phenyl]butyric Acid

Appearance colorless crystals (80% aq MeOH)
mp 137.5∼138.5°C

| Analysis for C₂₃H₂₇F₄NO₄S | | | |
|---|---|---|---|
| Calculated % | C, 56.43; | H, 5.56; | N, 2.86 |
| Found % | C, 56.16; | H, 5.74; | N, 2.93. |

### Example 207: 4-[4-[1-(4-Chlorophenylsulfonylamino)-5,6,6,6-tetrafluoro-5-trifluoromethylhexyl]phenyl]butyric Acid

Appearance colorless needles (i-Pr₂O)
mp 111∼112°C

| Analysis for C₂₃H₂₃ClF₇NO₄S | | | |
|---|---|---|---|
| Calculated % | C, 47.80; | H, 4.01; | N, 2.42 |
| Found % | C, 47.83; | H, 4.05; | N, 2.52 |

### Example 208: 4-[4-[5,6,6,6-Tetrafluoro-5-trifluoromethyl-1-(4-fluorophenylsulfonylamino)hexyl]phenyl]butyric Acid

Appearance colorless plates (70% aq MeOH)
mp 122.5∼124°C

| Analysis for C₂₃H₂₃F₈NO₄S | | | |
|---|---|---|---|
| Calculated % | C, 49.20; | H, 4.13; | N, 2.49 |
| Found % | C, 49.23; | H, 4.22; | N, 2.57. |

### Example 209: 4-[4-[1-(4-Chlorophenylsulfonylamino)-4,4,5,5,6,7,7,7-octafluoro-6-trifluoromethylheptyl]phenyl]butyric Acid

Appearance colorless scales (80% aq MeOH)
mp 111.5∼112.5°C

| Analysis for C₂₄H₂₁ClF₁₁NO₄S | | | |
|---|---|---|---|
| Calculated % | C, 43.42; | H, 3.19; | N, 2.11 |
| Found % | C, 43.31; | H, 3.30; | N, 2.12 |

### Example 210: 4-[4-[4,4,5,5,6,7,7,7-Octafluoro-6-trifluoromethyl-1-(4-fluorophenylsulfonylamino)heptyl]phenyl]butyric Acid

Appearance colorless columns (i-Pr₂O-n-Hexane)
mp 103.5∼104.5°C

| Analysis for C₂₄H₂₁F₁₂NO₄S | | | |
|---|---|---|---|
| Calculated % | C, 44.52; | H, 3.27; | N, 2.16 |
| Found % | C, 44.56; | H, 3.35; | N, 2.25 |

### Example 211: 4-[4-[1-(4-Chlorophenylsulfonylamino)-6-fluorohexyl]phenyl]butyric Acid

Appearance colorless needles (80% aq MeOH)
mp 130.5∼131.5°C

| Analysis for C₂₂H₂₇ClFNO₄S | | | |
|---|---|---|---|
| Calculated % | C, 57.95; | H, 5.97; | N, 3.07 |
| Found % | C, 57.95; | H, 5.92; | N, 3.14 |

### Example 212: 4-[4-[6-Fluoro-1-(4-fluorophenylsulfonylamino)hexyl]phenyl]butyric Acid

Appearance colorless needles (80% aq MeOH)
mp 140.5∼142°C

| Analysis for C₂₂H₂₇F₂NO₄S | | | |
|---|---|---|---|
| Calculated % | C, 60.12; | H, 6.19; | N, 3.19 |
| Found % | C, 60.23; | H, 6.15; | N, 3.26. |

### Example 213: 4-[4-[6-Fluoro-1-(4-nitrophenylsulfonylamino)hexyl]phenyl]butyric Acid

Appearance colorless plates (AcOEt-i-Pr₂O)
mp 97∼98°C

| Analysis for C₂₂H₂₇FN₂O₆S | | | |
|---|---|---|---|
| Calculated % | C, 56.64; | H, 5.83; | N, 6.00 |
| Found % | C, 56.70; | H, 5.83; | N, 6.01 |

### Example 214: (+)-4-[4-[5,5,6,6,6-Pentafluoro-1-(4-fluorophenylsulfonylamino)hexyl]phenyl]butyric Acid

A solution of 22.0 g of (±)-4-[4-[5,5,6,6,6-pentafluoro-1-(4-fluorophenylsulfonylamino)hexyl]phenyl]butyric acid and 5.47 g of (S)-(+)-1-cyclohexylethylamine in 88 ml of ethyl acetate was allowed to stand at room temperature. The crystals precipitated were collected by filtration to obtain 13.1 g of colorless crystals. Repeated recrystallization from ethyl acetate gave 8.54 g of (S)-(+)-1-cyclohexylethylamine salt of the title compound as colorless needles, mp 124∼127.5°C.

| Analysis for C₃₀H₄₀F₆N₂O₄S | | | |
|---|---|---|---|
| Calculated % | C, 56.41; | H, 6.31; | N, 4.39 |
| Found % | C, 56.30; | H, 6.42; | N, 4.16. |

Specific rotation [α]²⁰_{D} +15.4° (c=1.0, MeOH)

8.20 g of the salt obtained above was added to dilute hydrochloric acid and extraction was carried out using ethyl acetate. The ethyl acetate layer was washed with water, dried, and then the solvent was removed. The resulting residue was recrystallized from diisopropyl ether to obtain 5.90 g of the title compound as colorless prisms, mp 106∼108°C.

| Analysis for C₂₂H₂₃F₆NO₄S | | | |
|---|---|---|---|
| Calculated % | C, 51.66; | H, 4.53; | N, 2.74 |
| Found % | C, 51.61: | H, 4.38; | N, 2.56 |

Specific rotation [α]²⁰_{D} +17.3° (c=1.0, MeOH)

### Example 215: (-)-4-[4-[5,5,6,6,6-Pentafluoro-1-(4-fluorophenylsulfonylamino)hexyl]phenyl]butyric Acid

10.7 g of free acid was recovered in an usual manner from the residue obtained from the filtrate that was recovered after the salt formation between 22.0 g of (±)-4-[4-[5,5,6,6,6-pentafluoro-1-(4-fluorophenylsulfonylamino)hexyl]phenyl]butyric acid and 5.47 g of (S)-(+)-1-cyclohexylethylamine in ethyl acetate. A solution of the crystals obtained and 2.66 g of (R)-(-)-1-cyclohexylethylamine in 40 ml of ethyl acetate was allowed to stand at room temperature. The crystals precipitated were collected by filtration to obtain 10.5 g of colorless crystals. Repeated recrystallization from ethyl acetate gave 7.51 g of (R)-(-)-1-cyclohexylethylamine salt of the title compound as colorless needles, mp 124∼127.5°C.

| Analysis for C₃₀H₄₀F₆N₂O₄S | | | |
|---|---|---|---|
| Calculated % | C, 56.41; | H, 6.31; | N, 4.39 |
| Found % | C, 56.19; | H, 6.30; | N, 4.20. |

Specific rotation [α]²⁰_{D} -15.3° (c=1.0, MeOH)

7.17g of the salt obtained above was added to dilute hydrochloric acid and extraction was carried out using ethyl acetate. The ethyl acetate layer was washed with water, dried, and then the solvent was removed. The resulting residue was recrystallized from diisopropyl ether to obtain 4.96 g of the title compound as colorless prisms, mp 103∼108°C.

| Analysis for C₂₂H₂₃F₆NO₄S | | | |
|---|---|---|---|
| Calculated % | C, 51.66; | H, 4.53; | N, 2.74 |
| Found % | C, 51.58; | H, 4.28; | N, 2.63 |

Specific rotation [α]²⁰_{D} -16.3° (c=1.0, MeOH)

### Example 216: Tablet

A tablet is prepared according to the following formulation:

| | |
|---|---|
| Compound of Example 189 | 50mg |
| Lactose | Sufficient Quantity |
| Corn starch | 34mg |
| Magnesium stearate | 2mg |
| Hydroxypropylmethylcellulose | 8mg |
| Polyethyleneglycol 6000 | 0.5mg |
| Titanium oxide | 0.5mg |
| | 160mg |

### Example 217: Capsule

A capsule is prepared according to the following formulation:

| | |
|---|---|
| Compound of Example 189 | 50mg |
| Lactose | Sufficient Quantity |
| Calcium carboxymethylcellulose | 15mg |
| Hydroxypropylcellulose | 2mg |
| Magnesium stearate | 2mg |
| | 140mg |

The above ingredients are mixed in an ordinary manner and filled into a hard capsule.

### Example 218: Powder

Powder is prepared according to the following formulation:

| | |
|---|---|
| Compound of Example 189 | 50mg |
| Lactose | Sufficient Quantity |
| D-Mannitol | 500mg |
| Hydroxypropylcellulose | 5mg |
| Talc | 2mg |
| | 1000mg |

### Example 219: Injection

Injection is prepared according to the following formulation:

| | |
|---|---|
| Compound of Example 189 | 10mg |
| Glucose | 100mg |
| Sodium hydroxide | Sufficient Quantity |
| Distilled water for injection | Sufficient Quantity. |
| | 2ml |

Results of experiments in thromboxane A₂ antagonistic effect, leukotriene D₄ antagonistic effect, inhibitory effect on bronchoconstriction induced by U-46619, inhibitory effect on bronchoconstriction induced by leukotriene D₄, and in vitro metabolism using the compounds of the present invention are set out below. 4-[4-[1-(4-Chlorophenylsulfonylamino)hexyl]phenyl]butyric acid (Reference compound 1) and 4-[4-[1-(4-chlorophenylsulfonylamino)-5-methylhexyl]phenyl]butyric acid (Reference compound 2) disclosed in the Japanese Patent Unexamined Publication (Kokai) No. (Hei) 7-53505 were used as reference drugs.

### Experiment 1: Thromboxane A₂ antagonistic effect

According to an ordinary method, tracheas were isolated from male Hartley guinea-pigs, and spiral specimens of approximately 2 cm length were prepared. These specimens were suspended in organ baths that contained Krebs-Henseleit solution at 37°C under a resting tension of 1.5 g, and the changes in isometric tension were recorded. The first concentration-response curves were obtained by cumulative applications of 1 × 10⁻⁹ to 3 × 10⁻⁷M of U-46619 (Cayman, thromboxane A₂ / prostaglandin H₂ receptor agonist) in the organ baths. After treatments with test compounds at adequate concentrations for 5 minutes, the second concentration-response curves were obtained in the same manner as the first applications. pK_{B} values were calculated using these two concentration-response curves according to the method of Schild [Pharmacological Review, Vol. 9, 242 (1957)]. Indomethacin (3 × 10⁻⁶M, Sigma) was added beforehand to the organ bath to eliminate possible affections by thromboxane A₂ and prostaglandin produced endogenously [Japan J. Pharmacol., Vol. 60, 227 (1992)]. The results are shown in Table 1.

**Table 1**

| Thromboxane A₂ antagonistic effect | | | |
|---|---|---|---|
| Test compound | pK_{B} | Test compound | pK_{B} |
| Example 162 | 9.4 | Example 183 | 8.7 |
| Example 163 | 9.1 | Example 188 | 9.1 |
| Example 172 | 9.4 | Example 189 | 8.6 |
| Example 176 | 8.5 | Example 200 | 8.0 |
| Example 177 | 8.2 | Example 202 | 7.9 |
| Example 178 | 8.5 | Reference compound 1 | 9.0 |
| Example 179 | 8.1 | Reference compound 2 | 8.9 |

### Experiment 2: Inhibitory effect on bronchoconstriction induced by U-46619

Airway resistance was evaluated according to the method of Konzett-Roessler [Naunyn-Schmiedberg's Arch. Exp. Path. Pharmak., Vol. 195, 71 (1940)]. Male Hartley guinea-pigs (about 400g wt.) were anesthetized with urethane (1.5g/kg, i.p.) and ventilated by an artificial respirator (Model 683; Harvard). At the same time, overflowed air volume above the pressure of about 12cm H₂O was measured by using a sensor (Model 7020; Ugo Basile) and the value was used as an index of bronchoconstriction. 0.3 mg/kg (5 ml/kg) of test compounds suspended in 5% gum Arabic was administered orally to the guinea-pigs fasted beforehand for 24 hours. After 2 hours, U-46619 (4 µg/kg; Cayman) was administered through the cervical vein, and then maximal response was measured. Inhibitory rates compared to the control group were calculated based on the response rate normalized by the complete closure as 100%. The results are shown in Table 2. As clearly shown in Tables 1 and 2, the compounds of the present invention exhibited excellent anti-thromboxane A₂ activities comparable to the reference compounds.

**Table 2**

| Inhibitory effect on bronchoconstriction induced by U-46619 | | | |
|---|---|---|---|
| Test compound | Inhibition rate (%) | Test compound | Inhibition rate (%) |
| Example 162 | 85 | Example 183 | 89 |
| Example 163 | 92 | Example 188 | 88 |
| Example 172 | 73 | Example 189 | 71 |
| Example 176 | 78 | Example 200 | 82 |
| Example 177 | 93 | Example 202 | 85 |
| Example 178 | 89 | Reference compound 1 | 85 |
| Example 179 | 93 | Reference compound 2 | 81 |

### Experiment 3: Leukotriene D₄ antagonistic effect

According to an ordinary method, tracheas were isolated from male Hartley guinea-pigs, and spiral specimens of approximately 2 cm length were prepared. These specimens were suspended isotonically in organ baths that contained Krebs-Henseleit solution at 37°C under a resting tension of 0.5g. After contractions with acetylcholine (1 × 10⁻⁵M; Ovisot for injection, Daiichi Seiyaku), and the first concentration-response curves were obtained by cumulative applications of 10⁻¹⁰ to 10⁻⁸M of leukotriene D₄ (ULTRA FINE) in the organ baths. After treatments with test compounds at adequate concentrations for 30 minutes, the second concentration-response curves were obtained in the same manner as the first applications. pK_{B} values were calculated using these two concentration-response curves according to the method of Schild [Pharmacological Review, Vol. 9, 242 (1957)]. Indomethacin (1 × 10⁻⁶M, Sigma) was added beforehand to the organ bath to eliminate possible affections by thromboxane A₂ and prostaglandin produced by stimulus of leukotriene D₄ [Japan J. Pharmacol., Vol. 60, 227 (1992)]. The results are shown in Table 3. As clearly shown in Table 3, the compounds of the present invention exhibited excellent leukotriene D₄ antagonistic effect comparable to the reference compounds.

**Table 3**

| Leukotriene D₄ antagonistic effect | | | |
|---|---|---|---|
| Test compound | pK_{B} | Test compound | pK_{B} |
| Example 162 | 6.6 | Example 183 | 6.4 |
| Example 163 | 6.4 | Example 188 | 6.7 |
| Example 172 | 6.6 | Example 189 | 6.6 |
| Example 176 | 6.9 | Example 200 | 6.6 |
| Example 177 | 6.4 | Example 202 | 6.1 |
| Example 178 | 6.4 | Reference compound 1 | 6.7 |
| Example 179 | 7.0 | Reference compound 2 | 6.6 |

### Experiment 4: Inhibitory effect on bronchoconstriction induced by leukotriene D₄

Airway resistance was evaluated according to the method of Konzett-Roessler [Naunyn-Schmiedberg's Arch. Exp. Path. Pharmak., Vol. 195, 71 (1940)]. Male Hartley guinea-pigs fasted beforehand for 24 hours were anesthetized with urethane, and then overflowed air volume was measured under artificial ventilation as an index of bronchoconstriction. Various doses of test compounds suspended in 5% gum Arabic were administered orally, and after 2 hours, leukotriene D₄ (1 µg/kg; ULTRA FINE) was administered through the cervical vein. Then, values at 2 minutes were measured when maximum responses were obtained. Inhibitory rates compared to the control group were calculated based on the response rate normalized by the overflowed volume at complete closure as 100% bronchoconstriction rate, and the 50% inhibitory doses (ED₅₀ values) were calculated using dose-inhibitory rate curves. Indomethacin (2mg/kg, i.v.) and propranolol (1mg/kg, i.v.) were administered at 10 minutes and 5 minutes before the administration of leukotriene D₄, respectively. The results are shown in Table 4. As clearly shown in Table 4, the compounds of the present invention exhibited more excellent anti-leukotriene D₄ effect than the reference compounds by oral administration.

**Table 4**

| Inhibitory effect on bronchoconstriction induced by leukotriene D₄ | | | |
|---|---|---|---|
| Test compound | ED₅₀ (mg/kg) | Test compound | ED₅₀ (mg/kg) |
| Example 162 | 1.3 | Example 183 | 1.1 |
| Example 163 | 0.78 | Example 188 | 1.6 |
| Example 172 | 2.4 | Example 189 | 0.92 |
| Example 176 | 0.14 | Example 200 | 0.21 |
| Example 177 | 0.69 | Example 202 | 0.97 |
| Example 178 | 0.67 | Reference compound 1 | 8.1 |
| Example 179 | 0.17 | Reference compound 2 | 9.2 |

### Experiment 5: In vitro metabolism test

0.05 mM EDTA-0.1M phosphate buffer (pH 7.4), distilled water, 0.4mg protein/ml human liver microsomes and 0.02 mM test compound were mixed in a test tube under ice-cooling. After the mixture was shaken at 37°C for 2 minutes beforehand, the mixture was added with a ready-mix of NADPH-generating system and then shaken at 37°C for 30 minutes. After the incubation, the reaction was stopped by cooling with ice. The mixture was added with acetonitrile and well mixted, and then centrifuged at 3000 rpm for 10 minutes. The resulting supernatant was subjected to HPLC measurement, and the residual rate of test compound was calculated from the ratio of a peak area of an unchanged compound in the reaction sample in comparison with a non-reaction mixture. Preparations of reagents and reaction mixtures were carried out by referring to Shin Seikagaku Koza, Vol. 5: Biological oxidation and drug metabolism edited by The Japanese Biochemical Society. The results are shown in Table 5. It is clear from Table 5 that the compounds of the present invention exhibited higher metabolic stability than the reference compounds.

**Table 5**

| In vitro metabolism test | | | |
|---|---|---|---|
| Test compound | Residual rate (%) | Test compound | Residual rate (%) |
| Example 162 | 72 | Example 183 | 78 |
| Example 163 | 87 | Example 188 | 74 |
| Example 172 | 84 | Example 189 | 88 |
| Example 176 | 86 | Example 200 | 83 |
| Example 177 | 87 | Example 202 | 93 |
| Example 178 | 84 | Reference compound 1 | 53 |
| Example 179 | 78 | Reference compound 2 | 50 |

### Industrial Applicability

The benzenesulfonamide derivatives of the present invention have both of excellent thromboxane A₂ and leukotriene D₄ antagonistic activities, and have excellent effect when orally administered. Therefore, they are extremely useful as active ingredients of medicaments such as platelet aggregation inhibitors, antithrombotic agents, antiasthmatic agents, and antiallergic agents.

## Claims

1. A compound represented by the following general formula (I) or a salt thereof: 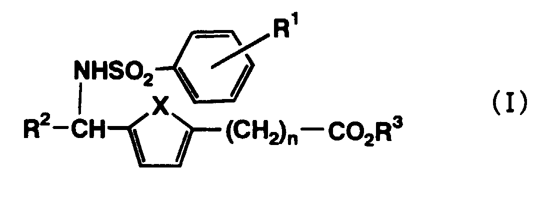 wherein R¹ represents hydrogen atom, a halogen atom, a lower alkyl group, a lower alkoxyl group, or nitro group; R² represents a C₄-C₈ alkyl group which is substituted with one or more fluorine atoms; R³ represents hydrogen atom or a lower alkyl group; X represents sulfur atom or a group represented by CH=CH; and n represents an integer of from 2 to 4.

2. The compound or the salt thereof according to claim 1 wherein X is sulfur atom.

3. The compound or the salt thereof according to claim 1 wherein X is a group represented by CH=CH.

4. The compound or the salt thereof according to any one of claims 1 to 3 wherein R¹ is a halogen atom which substitutes in the para-position, R² is a C₅-C₇ straight or branched chain alkyl group which is substituted with three or more fluorine atoms, and n is 3 or 4.

5. A medicament which comprises as an active ingredient a substance selected from the group consisting of the compound represented by the general formula (I) and the physiologically acceptable salt thereof according to claim 1, and a hydrate thereof and a solvate thereof.

6. The medicament according to claim 5 which is used for preventive and/or therapeutic treatment of thrombosis, asthma, or in allergic disease.

7. The medicament according to claim 5 which is used as a platelet aggregation inhibitor, an antithrombotic agent, an antiasthmatic agent, or an antiallergic agent.

8. A use of a substance selected from the group consisting of the compound represented by the general formula (I) and the physiologically acceptable salt thereof according to claim 1, and a hydrate thereof and a solvate thereof for the manufacture of the medicament according to any one of claims 5 to 7.

9. A method for therapeutic and/or preventive treatment of thrombosis, asthma, or an allergic disease which comprises the step of administering to a mammal including a human an effective amount of a substance selected from the group consisting of the compound represented by the general formula (I) and the physiologically acceptable salt according to claim 1, and a hydrate thereof and a solvate thereof.
